(19) Europäisches Patentamt
European Patent Office
Office européen des brevets

(11) EP 2 233 153 B1

(12) **EUROPEAN PATENT SPECIFICATION**

(45) Date of publication and mention of the grant of the patent:
**21.01.2015 Bulletin 2015/04**

(51) Int Cl.:
***A61K 39/15*** *(2006.01)* ***A61P 1/00*** *(2006.01)*

(21) Application number: **10159949.6**

(22) Date of filing: **15.08.2006**

(54) **Rotavirus vaccine inducing heterotypic cross protection**

Heterotypischen Kreuzschutz auslösende Rotavirus-Vakzine

Vaccin anti-rotavirus inducteur de protection croisée hétérotipique

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **17.08.2005 GB 0516944**
**18.10.2005 GB 0521164**
**05.05.2006 GB 0608962**

(43) Date of publication of application:
**29.09.2010 Bulletin 2010/39**

(62) Document number(s) of the earlier application(s) in accordance with Art. 76 EPC:
**06776899.4 / 1 915 173**

(73) Proprietor: **GlaxoSmithKline Biologicals S.A.**
**1330 Rixensart (BE)**

(72) Inventors:
- **Colau, Brigitte Desiree Alberte**
  **B-1330, Rixensart (BE)**
- **De Vos, Beatrice Arsene Virginie**
  **B-1330, Rixensart (BE)**

(74) Representative: **Crawley, Karen Anne et al**
**GlaxoSmithKline**
**Global Patents (CN925.1)**
**980 Great West Road**
**Brentford, Middlesex TW8 9GS (GB)**

(56) References cited:
**WO-A-01/12797 WO-A-2005/021033**

- **RUIZ-PALACIOS GUILLERMO M ET AL: "Safety and efficacy of an attenuated vaccine against severe rotavirus gastroenteritis." THE NEW ENGLAND JOURNAL OF MEDICINE 5 JAN 2006, vol. 354, no. 1, 5 January 2006 (2006-01-05), pages 11-22, XP002426337 ISSN: 1533-4406**
- **BARNES G L ET AL: "Early phase II trial of human rotavirus vaccine candidate RV3" VACCINE, BUTTERWORTH SCIENTIFIC. GUILDFORD, GB, vol. 20, no. 23-24, 26 July 2002 (2002-07-26), pages 2950-2956, XP004371823 ISSN: 0264-410X**
- **CLARK H F ET AL: "Vaccines for rotavirus gastroenteritis universally needed for infants" PEDIATRIC ANNALS 2004 UNITED STATES, vol. 33, no. 8, 2004, pages 536-543, XP009081064 ISSN: 0090-4481**
- **HOSHINO Y ET AL: "Rotavirus serotypes: classification and importance in epidemiology, immunity, and vaccine development" JOURNAL OF HEALTH, POPULATION, AND NUTRITION, BANGLADESH, vol. 18, no. 1, 1 June 2000 (2000-06-01), pages 5-14, XP009125513 ISSN: 1606-0997**
- **EGLEE PEREZ M ET AL: "Rhesus rotavirus-based quadrivalent vaccine is efficacious despite age, socioeconomic conditions and seasonality in Venezuela" VACCINE, ELSEVIER LTD, GB LNKD- DOI:10.1016/S0264-410X(00)00211-5, vol. 19, no. 7-8, 22 November 2000 (2000-11-22), pages 976-981, XP004225420 ISSN: 0264-410X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 233 153 B1

## Description

### TECHNICAL FIELD

This invention relates to rotavirus vaccine formulations. The invention relates to the use of an attenuated rotavirus population from one rotavirus type in the prevention of disease associated with rotavirus infection from another rotavirus type.

### TECHNICAL BACKGROUND

Acute, infectious diarrhoea is a leading cause of disease and death in many areas of the world. In developing countries, the impact of diarrhoeal disease is staggering. For Asia, Africa and Latin America, it has been estimated that there are between 3-4 billion cases of diarrhoea each year and of those cases about 5-10 million result in death (Walsh, J.A. et al.: N. Engl. J. Med., 301:967-974 (1979)).

Rotaviruses have been recognised as one of the most important causes of severe diarrhoea in infants and young children (Estes, M.K. Rotaviruses and Their Replication in Fields Virology, Third Edition, edited by Fields et al., Raven Publishers, Philadelphia, 1996). It is estimated that rotavirus disease is responsible for over one million deaths annually. Rotavirus-induced illness most commonly affects children between 6 and 24 months of age, and the peak prevalence of the disease generally occurs during the cooler months in temperate climates, and year-round in tropical areas. Rotaviruses are typically transmitted from person to person by the faecal-oral route with an incubation period of from about 1 to about 3 days. Unlike infection in the 6-month to 24-month age group, neonates are generally asymptomatic or have only mild disease. In contrast to the severe disease normally encountered in young children, most adults are protected as a result of previous rotavirus infection so most adult infections are mild or asymptomatic (Offit, P.A. et al. Comp. Ther., 8(8):21-26, 1982).

Rotaviruses are generally spherical, and their name is derived from their distinctive outer and inner or double-shelled capsid structure. Typically, the double-shelled capsid structure of a rotavirus surrounds an inner protein shell or core that contains the genome. The genome of a rotavirus is composed of 11 segments of double-stranded RNA which encode at least 11 distinct viral proteins. Two of these viral proteins designated as VP4 and VP7 are arranged on the exterior of the double-shelled capsid structure. The inner capsid of the rotavirus presents one protein, which is the rotavirus protein designated VP6. The relative importance of these three particular rotaviral proteins in eliciting the immune response that follows rotavirus infection is not yet clear. Nevertheless, the VP6 protein determines the group and subgroup antigen, and VP4 and VP7 proteins are the determinants of serotype (types determined by neutralisation assay) and genotype (types determined by a non-serological assay) specificity. The designations for G serotypes and G genotypes are identical. In contrast, the numbers assigned for P serotypes and genotypes are different (Santos N. et Hoshino Y., 2005, Reviews in Medical Virology, 15, 29-56). Therefore the P serotype is designated as P followed by assigned number, and the P genotype is designated by a P followed by assigned number in brackets.

To date, at least 14 rotavirus G serotypes and 14 rotavirus P serotypes have been identified (Santos N. et Hoshino Y., 2005, Reviews in Medical Virology, 15, 29-56). Among these, 10 G (G1-6, G8-10 and G12) serotypes and 9 P (P1, P2A, P3, P4, P5A, P7, P8, P11 and P12) serotypes have been identified among the human rotavirus. Twenty-three P genotypes have been described ten of which have been recovered from humans (P[3]-[6], P[8]-[11], P[14] and P[19].

VP7 protein is a 38,000 MW glycoprotein (34,000 MW when non-glycosylated) which is the translational product of genomic segment 7, 8 or 9, depending on the strain. This protein stimulates formation of the neutralising antibody following rotavirus infection. VP4 protein is a non-glycosylated protein of approximately 88,000 MW which is the translational product of genomic segment 4. This protein also stimulates neutralising antibody following rotavirus infection.

Since VP4 and VP7 proteins are the viral proteins against which neutralising antibodies are directed, they are believed to be prime candidates for development of rotavirus vaccines, affording protection against rotavirus illness.

Natural rotavirus infection during early childhood is known to elicit protective immunity.

A live attenuated rotavirus vaccine is thus highly desirable. Suitably this should be an oral vaccine, as this is the natural route of infection of the virus.

Early vaccine development for preventing rotavirus infections began in the 1970s after the discovery of the virus. Initially, attenuated strains from animals and humans were studied and had mixed or disappointing results. More recent efforts have focused on human-animal reassortants that have been more successful.

A rotavirus strain known as 89-12 has been described by Ward; see US Patent Number 5,474,773 and Bernstein, D.L. et al, Vaccine, 16 (4), 381-387, 1998. The 89-12 strain was isolated from a stool specimen collected from a 14 month-old child with natural rotavirus illness in 1988. According to US Patent Number 5,474,773 the HRV 89-12 human rotavirus was then culture-adapted by 2 passages in primary African Green Monkey Kidney (AGMK) cells and 4 passages in MA-104 cells as described by Ward in J. Clin. Microbiol., 19, 748-753, 1984. It was then plaque purified 3 times in

MA-104 cells (to passage 9) and grown after 2 additional passages in these cells. One additional passage was made (passage 12) for deposition with the ATCC under the accession number ATCC VR 2272. The deposited strain is known as 89-12C2.

The 1998 paper in Vaccine by Bernstein et al is referred to below as the Vaccine (1998) paper. The paper describes the safety and immunogenicity of an orally administered live human rotavirus vaccine candidate. This vaccine was obtained from strain 89-12, attenuated by passaging without plaque purification 26 times in primary AGMK cells and then another 7 times in an established AGMK cell line (33 passages in total).

Hereinafter the aforesaid material which has been serially passaged 26 times will be referred to as P26 and the material which has been serially passaged 33 times will be referred to as P33. In general, rotavirus derived by passaging 89-12 n times will be referred to as Pn.

In the examples which follow the P33 material was passaged a further 5 times on Vero cells. This is referred to as P38.

The P26 and P33 isolates described in the Vaccine (1998) paper were not deposited in a culture collection, nor were they analysed to establish their genetic characterisation.

It has now been found that the P26 population described in the literature comprises a mixture of variants. This has been established by genetic characterisation as described hereinbelow (see examples). P26 is therefore not a reliably consistent population for further passages, in particular for the production of vaccine lots. Similarly, P33 comprises a mixture of variants and is not reliably consistent for the production of vaccine lots.

It has been found that the P26 material is a mixture of at least three VP4 gene variants. P33 and P38 are similarly a mixture of two variants. These variants appear to be antigenically different, in terms of neutralising epitopes, to the 89-12C2 strain deposited at the ATCC when evaluating the neutralizing antibody titers of sera from infants vaccinated with P33 against these variants.

Furthermore it has been found that when the P33 material is administered to infants, two identified variants are replicated and excreted. Of 100 vaccinated infants, only 2 showed signs of gastro-enteritis due to rotavirus infection, while 20% of a placebo group were infected. These findings suggest that the identified variants are associated with protection from rotavirus disease.

WO 01/12797 discloses a method of separating rotavirus variants and an improved live attenuated rotavirus vaccine derived from a cloned (homogeneous) human rotavirus strain. Also disclosed is an attenuated rotavirus population (isolate), characterised in that it comprises a single variant or substantially a single variant, said variant defined by the nucleotide sequence encoding at least one of the major viral proteins designated as VP4 and VP7. Protective efficacy of such an oral attenuated human rotavirus vaccine against G9 heterologous strain has been reported in Latin American infants (Perez et al. 42nd Interscience Conference on Antimicrobial Agents and Chemotherapy (ICAAC 2002) 27-30 September 2002, San Diego). WO 05/021033 discloses that one rotavirus serotype may be used to protect against disease caused by another serotype. In particular WO 05/021033 discloses the use of a G1 rotavirus population, [for example as deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 OJG, United Kingdom on 13 August 1999 under the deposition number 99081301, under the terms of the Budapest Treaty, also named P43 or RIX4414], to prevent disease caused by both G1 and at least one non-G1 rotavirus serotype, such as but not limited to the G2, G3, G4 and G9 rotavirus serotypes.

The whole content of WO 01/12797 and WO 05/021033 is herein incorporated by reference.

## BRIEF DESCRIPTION OF THE FIGURES

Figure 1A (SEQ ID NO:1) is the nucleotide sequence of P43 (RIX4414) VP4 gene including the sequence encoding the VP4 protein of P43.

Figure 1B (SEQ ID NO:2) has additional nucleotides from both ends of the gene and a nucleotide substitution (in bold - a G instead of a C at position 18 with resulting in TCG instead of TCA with however no impact on the resulting encoded protein) due to the sequencing technique. The non-coding sequence appears in small case. Figure 1 B shows the correct sequence for the P43 deposit.

Figure 2A (SEQ ID NO:3) is the nucleotide sequence of P43 (RIX4414) VP7 gene including the sequence encoding the VP7 protein of P43.

Figure 2B (SEQ ID NO:4) has additional nucleotides from both ends of the gene and a nucleotide substitution (in bold - a A instead of a C at position 58, resulting in a ATT coding for leucine instead of CTT coding for isoleucine) due to the sequencing technique. The non-coding sequence appears in small case. Figure 2B shows the correct sequence for the P43 deposit.

Figure 3 (SEQ ID NO:5) is the polypeptide sequence of RIX4414 VP4.

Figure 4 (SEQ ID NO:6) is the polypeptide sequence of RIX4414 VP7.

Figure 5 (SEQ ID NO:7) shows the polypeptide sequence of NSP4 protein of RIX4414.

Figure 6 (SEQ ID NO:8) shows the nucleotide sequence encoding NSP4 protein of RIX4414. The non-coding sequence appears in small case.

Figure 7 (SEQ ID NO:9) shows the polypeptide sequence of VP6 protein of RIX4414.

Figure 8 (SEQ ID NO:10) shows the nucleotide sequence encoding VP6 protein of RIX4414. The non-coding sequence appears in small case.

## DETAILED DESCRIPTION OF THE INVENTION

In the present disclosure we have determined that an attenuated rotavirus population, for example one such as characterised in WO 01/12797, can be used as a vaccine to provide cross protection against disease caused by rotavirus infection of a different type (serotype and/or genotype) to that used in the vaccine. The VP7 protein specifies the G type (serotype), and the VP4 protein specifies the P type of strain (serotype or genotype).

In particular the present disclosure relates to the use of an attenuated rotavirus population from one P type in the prevention of disease associated with rotavirus infection from a different P type, and specifically to the use of an attenuated rotavirus population or strain from a GxPy type in the induction of an immune response and/or in the prevention of disease associated with rotavirus infection caused by a rotavirus strain which is neither a Gx nor a Py type.

Immunity may be measured by neutralising antibody responses to the vaccine or by serum rotavirus IgA antibody response, such as seroconversion factor (i.e. $\geq$3-fold increase in serum antibody IgA levels following vaccination, as described in Ward et al., 1990, J. Infect. Disease, 161, 440-445).

In the context of this disclosure, and consistent with the common understanding in the art (Santos N. et Hoshino Y., 2005, Reviews in Medical Virology, 15, 29-56), Gx will refer to a specific G type, i.e. G genotype or G serotype (both terminologies being identical), whilst Py terminology will generically refer to a specific P type, either P serotype (e.g. P8, P4) or P genotype (e.g. P[4], P[8]). When referred to a specific P genotype, the P followed by assigned number in brackets will be used; otherwise P type will mean either serotype or genotype.

In one aspect of the invention, there is provided a use of an attenuated rotavirus strain from a G1 P[8] type in the manufacture of a composition for conferring protection against rotavirus infection caused by a rotavirus strain of a G2P[4] type.

Throughout this specification, wording such as the use of a vaccine composition according to the invention in the manufacture of a vaccine composition for the prevention of rotavirus diseases, or such as methods of therapy comprising the use of said vaccine composition will be interchangeably used.

We have now determined that a G1P[8] rotavirus population [for example G1 P[8] as deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty], can be used to prevent disease caused by both a G1P[8] and at least one rotavirus strain which is neither a G1 nor a P8 type. We have determined that a G1P[8] rotavirus population can be used to prevent disease caused by both one G1 P[8] and at least one non- G1P[8] genotypes, such as G2P[4] rotavirus genotype.

Accordingly the present disclosure relates to use of an attenuated rotavirus population from one rotavirus type in the prevention of disease associated with rotavirus infection from another rotavirus type, wherein the type is suitably defined by reference to the sequence of the rotavirus VP4 protein (P type).

The disclosure also relates to the use of an attenuated rotavirus population from one rotavirus strain (defined by both a specific G and P type) in the prevention of disease associated with rotavirus infection from another rotavirus strain, wherein the strain is suitably defined by reference to the sequence of both the rotavirus VP4 protein (P type) and VP7 protein (G type).

In all aspects of the claimed invention said immune response is a protective immune response. Suitably the rotavirus population may comprise VP4 and/or VP7 viral proteins from ECACC deposit 99081301 suitable to provide a cross protective effect.

Throughout the document, it will be referred to cross-protection as being the protection afforded by a rotavirus type against infection caused by a rotavirus of a different type. Cross-protection can be homotypic or heterotypic. Homotypic cross-protection is a protection afforded by a rotavirus strain against a strain of either a G or a P type, such as for example a G1P[8] strain affording cross-protection against a non-G1, P[8] strain (e.g. G2P[8]) via the P[8] type. Another example of a homotypic cross-protection is that afforded by a G1P[8] strain against a G1 non-P[8] strain (e.g. G1 P[4]) via the G1 type. Heterotypic cross-protection is a protection afforded by a rotavirus strain against a rotavirus strain of different P and G types such as for example the protection afforded by a G1P[8] against a non G1- non P[8]-strain (e.g. G2P[4]) (heterotypic protection afforded via both G and P types).

In particular, a G1 attenuated rotavirus population from a G1P[8] type, [for example as deposited at the European

Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty], can be used to prevent disease caused by G1P[8] and at least one, non-G1 rotavirus serotypes selected from the group consisting of: G3, G4, and G9.

The vaccine composition for use according to the invention comprises a G1 P[8] rotavirus strain and is capable of inducing an immune response to a G2P[4] rotavirus strain.

In a particular aspect, the disclosure relates to a method of inducing an immune response against rotavirus strain, the method comprising administering to a subject a composition comprising an attenuated rotavirus strain of a GxPy type, said composition generating an immune response against a rotavirus strain which is neither a Gx nor a Py type.

The rotavirus population within the vaccine composition is of G1P1A (i.e. G1P[8] according to the current nomenclature) strain specificity. Suitably the rotavirus population comprises VP4 and/or VP7 viral proteins from ECACC deposit 99081301 suitable to elicit an immune response and, typically, provide a cross protective eflect. The invention relates to G1P[8] rotavirus strains in methods or uses as described above. In one embodiment, the rotavirus vaccine used is the ECACC deposit 99081301, or is derived from that deposit.

In a specific aspect the vaccine induces a cross-protective immune response or cross-protection against gastro-enteritis in a vaccinated individual compared to the unvaccinated individual (from the placebo group). Suitably the vaccine provides cross-protection against rotavirus infection symptoms such as diarrhea or gastro-enteritis. For example gastro-enteritis may be defined as diarrhea characterised by three or more, watery or looser than normal, stools within a day, or forceful vomiting along with the detection of rotavirus in the examined stool specimen.

As will be understood by the skilled artisan, disease severity and efficacy of the vaccination to induce a protective immune response in a vaccinated individual or a vaccinated population may be assessed by several means. By protective immune response is meant an immune response which leads to a reduction of the severity of clinical symptoms associated with rotavirus infection or that leads to reduced susceptibility to rotavirus infection. Disease severity in an unvaccinated or a vaccinated individual may be graded according to published scoring systems such as the 20-point Vesikari scale or a slightly amended version of said method (Ruuska T et al. Scand. J. Infect. Dis. 1990, 22, 259-267), or according to any other suitable system reporting and grading specific symptoms of the rotavirus infection (such as the methodoly reported in Clark HF, Borian EF, Bell LM. Protective effect of WC3 vaccine against rotavirus diarrhea in infants during a predominantly serotype 1 rotavirus season. J Infect Dis. 1988:570-86). According to the Vesikari method, severe RVGE is usually defined as a score $\geq$11.

Protection may be assessed at the level of a population or a group by vaccine efficacy (VE). Vaccine efficacy is calculated using the following formula:

$$\mathbf{VE\ (\%) = 1 - RR = 1 - (ARV/ARU),}$$

with

RR = relative risk = ARV/ARU
ARU = disease attack rate in unvaccinated population (estimated from the placebo group) = number of subjects reporting at least one RV GE episode / total number of subjects in the control group.
ARV = disease attack rate in vaccinated group = number of subjects reporting at least one RV GE episode / total number of subjects in the HRV vaccine group.

In one aspect, the disclosure describes a use as detailed above wherein the composition comprising an attenuated rotavirus strain of a GxPy type induces a cross-protective immune response and/or protection against rotavirus-induced gastro-enteritis, suitably against severe rotavirus-induced gastroenteritis, caused by infection of a rotavirus strain which is neither a Gx nor a Py type. In a specific aspect, said protective immune response is capable of reducing the severity of the disease or eliminate rotavirus induced disease as measured according to any suitable scoring system.

In still another aspect, there is described a use of the composition according to the invention, to reduce the severity of the disease, e.g. gastroenteritis, or to eliminate rotavirus induced disease, said disease severity or disease being recorded according to any suitable scoring system as taught above.

In a specific aspect, said composition is up to 60% protective, suitably up to 81% protective, in a population of vaccinated individuals, against diarrhoea caused by infection of a rotavirus of a different type to that of the attenuated rotavirus present in the composition. In a specific embodiment, said composition is at least 40% protective, suitably at least 50% protective, in a population of vaccinated individuals, against diarrhoea caused by a rotavirus strain which is of a G2P[4] type. In a further embodiment said composition is between 40% and 80% protective, suitably between 50%

and 70% protective against diarrhoea caused by a rotavirus strain which is of a G2P[4] type. In a specific aspect of the disclosure, said composition comprises a G1P[8] rotavirus strain which affords the level of protection as mentioned above against gastro-enteritis caused by infection of rotavirus strains of G2P[4] type.

Suitably the protection rate against diarrhoea and/or gastro-enteritis and/or severe gastro-enteritis achieved in a population of vaccinated individuals infected by a rotavirus strain which is neither a Gx nor a Py type, may be between 10 to 90%, suitably between 20 to 80%, suitably between 40% and 80%, suitably between 45% and 75% protective. Typically the level of protection against severe gastro-enteritis is at least 40%, suitably at least 50%.

In a specific embodiment said composition comprises a G1P[8] rotavirus strain which is between 45% and 75% protective, in a population of vaccinated individuals against severe gastro-enteritis, as measured according to the Vesikari score, caused by infection of rotaviruses of with a G2P[4] serotype.

Suitably the vaccine is used in a 2 dose or a 3 dose regime. In one embodiment, the composition for use according to the invention is administered in a 2 dose regime.

The rotavirus vaccine used to give cross protection has the following suitable features.

In one embodiment, the G1P[8] rotavirus of the composition for use according to the invention has a VP4 gene comprising a nucleotide sequence comprising at least one of the following: an adenine base (A) at position 788, an adenine base (A) at position 802 and a thymine base (T) at position 501 from the start codon.

In a further embodiment, the G1P[8] rotavirus of the composition for use according to the invention has a VP7 gene comprising a nucleotide sequenece comprising at least one of the following: a thymine (T) at position 605, an adenine (A) at position 897, or a guanine (G) at position 897 from the start codon. Suitably at position 897 there is an adenine (A).

In a specific embodiment, the G1P[8] rotavirus of the composition for use according to the invention has an adenine (A) at positions 788 and 802 and a thymine (T) at position 501 from the start codon in the VP4 gene sequence.

In a particular embodiment, the G1P[8] rotavirus of the composition for use according to the invention has an adenine (A) at positions 788 and 802 and a thymine (T) at position 501 from the start codon in the VP4 gene sequence, and a thymine (T) at position 605 and an adenine at position 897 from the start codon in the VP7 sequence.

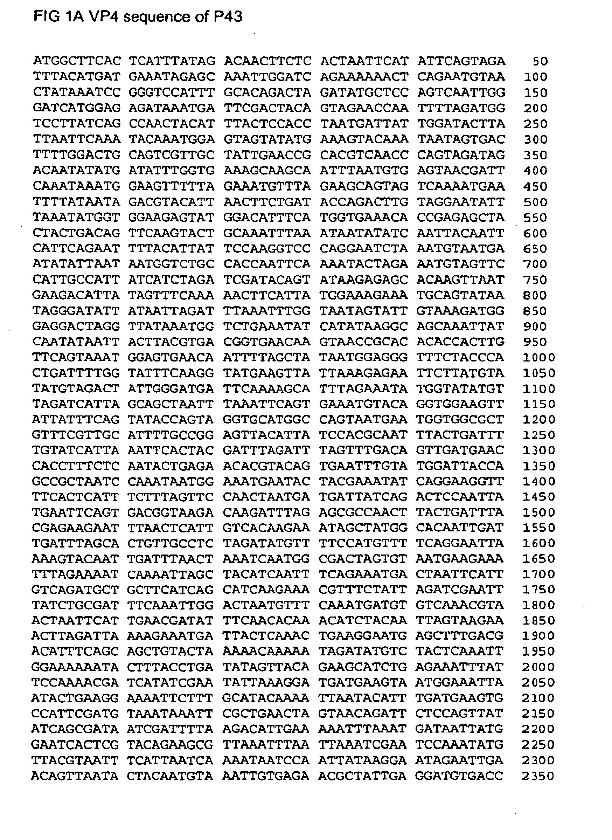

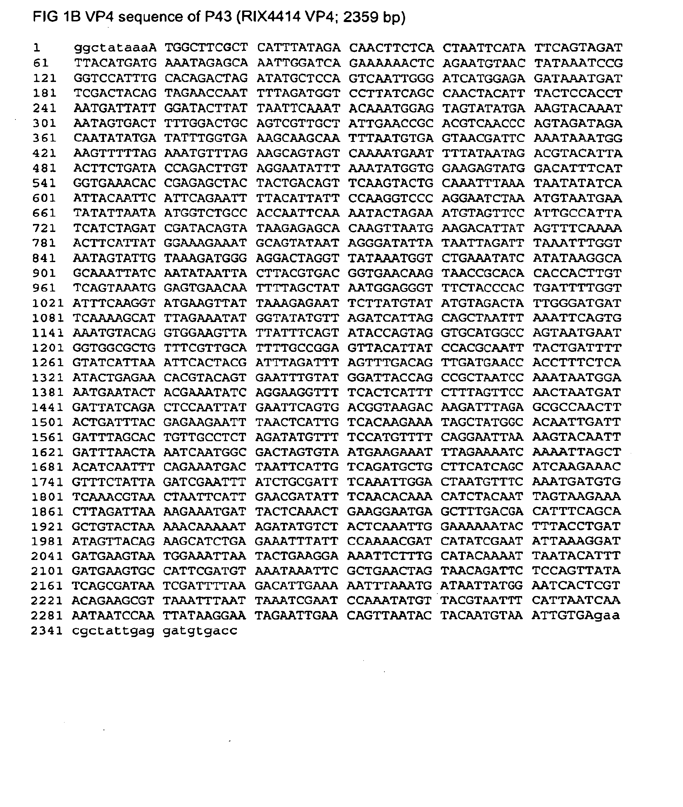

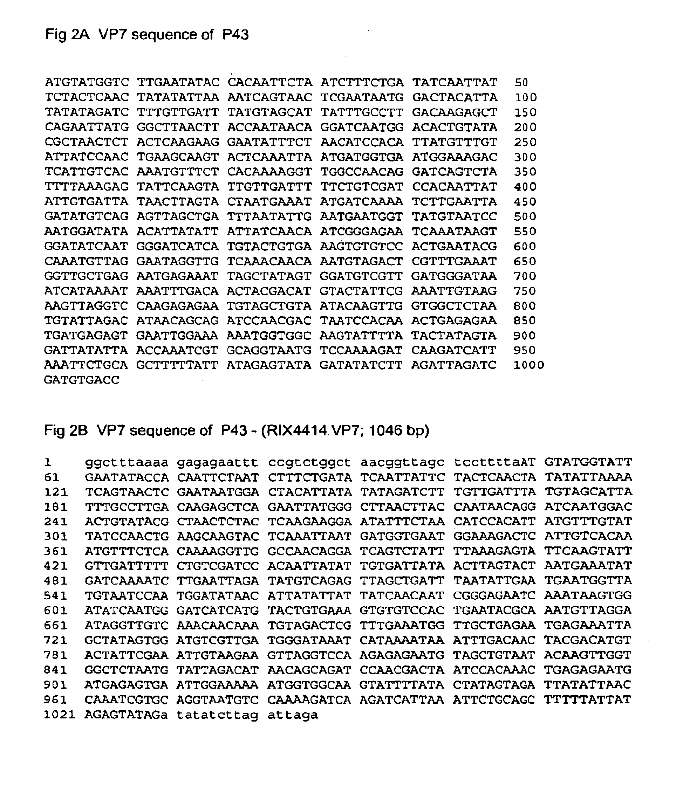

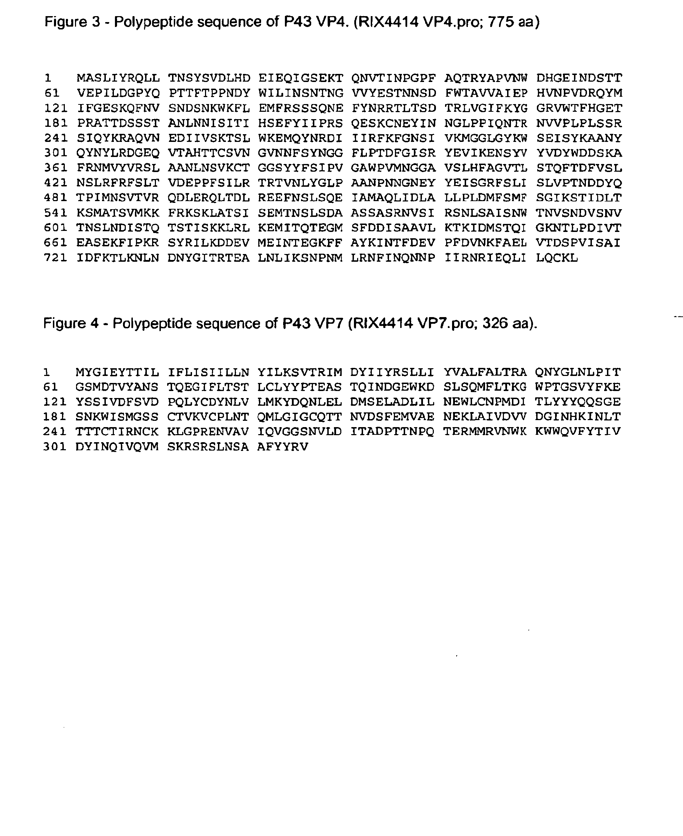

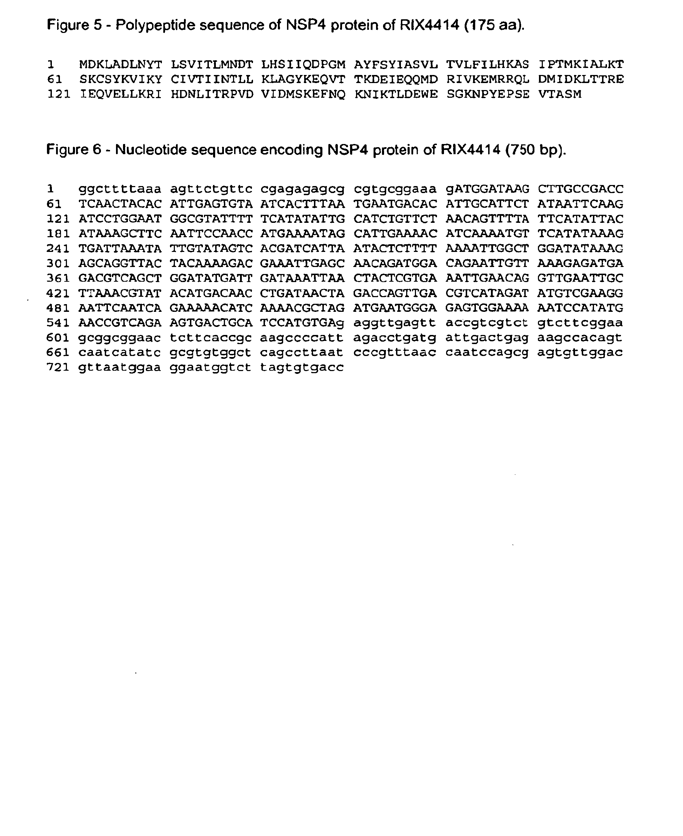

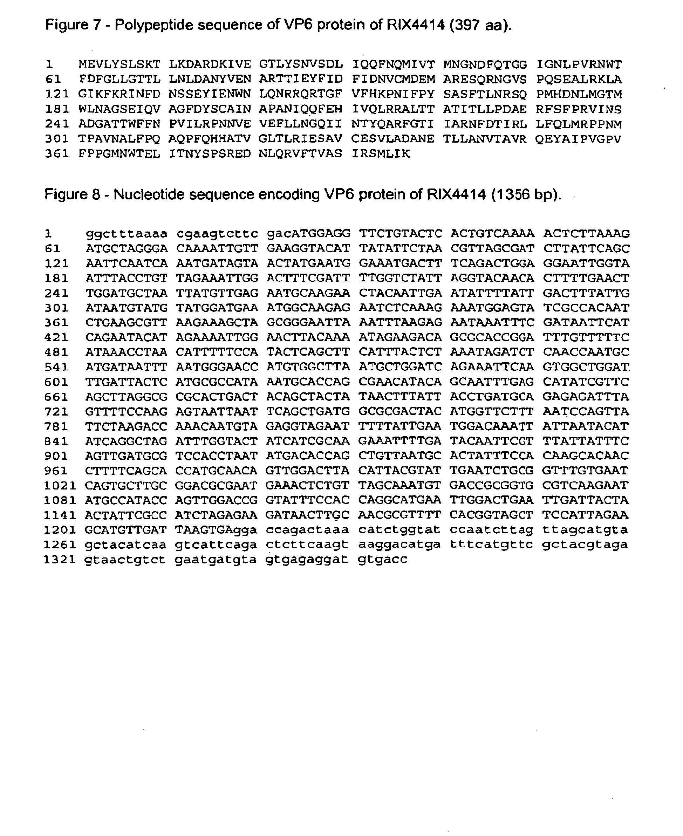

In another aspect of the disclosure, the rotavirus of the composition for use according to the invention comprises a nucleotide sequence encoding a VP4 protein wherein the nucleotide sequence is as shown in Figure 1A (SEQ ID NO:1) or Figure 1B (SEQ ID NO:2), and/or a nucleotide sequence encoding a VP7 protein wherein the nucleotide sequence is as shown in Figure 2A (SEQ ID NO:3) or Figure 2B (SEQ ID NO:4). In a further aspect the rotavirus of the composition for use according to the invention comprises a VP4 protein as set forth is Figure 3 (SEQ ID NO:5), and/or a VP7 protein as set forth is Figure 4 (SEQ ID NO:6). In a further aspect, said rotavirus population for use according to the invention additionally comprises an NSP4 protein as set forth in Figure 5 (SEQ ID NO:7), or encoded by the nucleotide sequence as set forth in Figure 6 (SEQ ID NO:8), and/or a VP6 protein as set forth in Figure 7 (SEQ ID NO:9), or encoded by the nucleotide sequence as set forth in Figure 8 (SEQ ID NO:10).

Suitable rotavirus populations for use in the present invention may be obtained by a method comprising:

passaging a rotavirus preparation on a suitable cell type;
optionally selecting homogeneous culture using the steps of either:

a) limit dilution; or
b) Individual plaque isolation; and

checking for the presence of a substantially single variant by carrying out a sequence determination of an appropriate region of the VP4 and/or VP7 gene sequence.

Suitably the rotavirus population is derived from the P43 (RIX4414), P33 or P26 strains as described above.

The sequence determination may suitably be carried out by a quantitative or semiquantitative hybridisation technique such as slot blot hybridisation or plaque hybridisation.

The resulting cloned virus population resulting from the method according to the invention may be amplified by further passaging on a suitable cell line.

Suitable cell types for passaging the rotavirus population in the above method include African green monkey kidney (AGMK) cells, which may be established cell lines or primary AGMK cells. Suitable AGMK cell lines include for example Vero (ATCC CCL-81), DBS-FRhL-2 (ATCC CL-160), BSC-1 (ECACC 85011422) and CV-1 (ATCC CCL-70). Also suitable are MA-104 (rhesus monkey) and MRC-5 (human -ATCC CCL-171) cell lines. Vero cells are particularly Suitable for amplification purposes. Passaging on Vero cells gives a high virus yield.

Techniques for checking whether there is a single variant in a virus population resulting from the method, and for determining the nature of that single variant involve standard sequencing or hybridisation procedures known in the art and are described hereinbelow.,

In a specific aspect, the method is carried out using an appropriate rotavirus, particularly rotavirus having the characteristics of the 89-12 strain or of a passaged derivative thereof.

A particularly suitable single variant population is P43, which was obtained from P33 (an isolated human rotavirus passages 33 times in culture on appropriate cell types) by a series of end dilution cloning steps followed by passaging the cloned material on Vero cells for amplification.

A P43 population was deposited at the European Collection of Animal Cell Cultures (ECACC), Vaccine Research and Production Laboratory, Public Health Laboratory Service, Centre for Applied Microbiology and Research, Porton Down, Salisbury, Wiltshire, SP4 0JG, United Kingdom on 13 August 1999 under the deposition number 99081301, under the terms of the Budapest Treaty, and is disclosed in WO 01/12797.

Although this indicated public availability is the simplest method of obtaining the human rotavirus P43, similar and functionally substantially identical rotaviruses may be produced by these or other methods in view of the teachings of this invention. Such functionally substantially identical rotaviruses are considered to be biologically equivalent to the human rotavirus P43 of this invention and therefore are within the general scope of the present invention. It will therefore be understood that the invention encompasses rotavirus populations having the characteristics of the P43 variant as described herein.

It will also be understood that the disclosure encompasses materials derived from the deposited P43 ECACC 99081301 by subjecting it to further processing such as by propagating it by further passaging, cloning, or other procedures using the live virus or by modifying P43 in any way including by genetic engineering techniques or reassortant techniques. Such steps and techniques are well known in the art.

Materials derived from the deposited P43 which are covered by the disclosure include protein and genetic material. Of particular interest are reassortant rotaviruses which comprise at least one antigen or at least one segment of P43, for example reassortants which comprise a virulent strain of rotavirus in which one or part of one of the 11 genome segments has been replaced by the genome segment or part thereof of P43. Specifically, a rotavirus reassortant in which the segment or partial segment coding for NSP4 is a P43 segment or partial segment, may have useful properties. Reassortant rotaviruses and techniques for preparing them are well known (Foster, R. H. and Wagstaff, A. J. Tetravalent Rotavirus Vaccine, a review. ADIS drug evaluation, BioDrugs, Gev, 9 (2), 155-178, 1998).

Materials of particular interest are progeny of P43 and immunologically active derivatives of P43. Immunologically active derivatives means materials obtained from or with the P43 virus, particularly antigens of the virus, which are capable of eliciting an immune response that is reactive against Rotavirus when injected into a host animal.

In adapting the rotavirus to an appropriate cell line, for example Vero cells, it may be necessary to treat the virus so as to get rid of any potential contaminant such as any adventitious agents that may be present and which would otherwise cause contamination. In the case of ether-sensitive adventitious viruses, this may be done by ether treatment as described hereinbelow. The present disclosure also relates to inclusion of such ether treatment as an optional step in the overall procedure for obtaining an attenuated live rotavirus or vaccine formulated therewith.

The cross protective rotavirus strain for use according to the present invention may be combined with other rotavirus strains to provide additional protection or cross-protection against rotavirus infection or disease.

The present disclosure also relates to a live attenuated rotavirus vaccine capable of providing cross protection, as defined herein above, admixed with a suitable adjuvant or a pharmaceutical carrier.

In one aspect, the rotavirus vaccine for use according to the invention is a monovalent rotavirus vaccine containing a single rotavirus strain such as the G1P[8] strain.

The present invention is particularly advantageous in providing a live rotavirus vaccine in which the live attenuated rotavirus is a human rotavirus and does not cause intussusception.

Suitable pharmaceutical carriers for use with the attenuated rotavirus strain according to the invention include those known in the art as being suitable for oral administration, especially to infants. Such carriers include and are not limited to carbohydrates, polyalcohols, amino acids, aluminium hydroxide, magnesium hydroxide, hydroxyapatite, talc, titanium oxide, iron hydroxide, magnesium stearate, carboxymethylcellulose, hydroxypropylmethylcellulose, microcrystalline cellulose, gelatin, vegetal peptone, xanthane, caraghenane, arabic gum, ß-cyclodextrin. The disclosure also describes a process for preparing a rotavirus vaccine, for example by freeze drying the virus in the presence of suitable stabilisers or admixing the virus according to the invention with a suitable adjuvant or pharmaceutical carrier.

It may also be advantageous to formulate the virus of the invention in lipid-based vehicles such as virosomes or liposomes, in oil in water emulsions or with carrier particles. Alternatively or in addition immunostimulants such as those known in the art for oral vaccines may be included in the formulation. Such immunostimulants include bacterial toxins, particularly cholera toxin (CT) in the form of the holotoxin (entire molecule) or the B chain only (CTB) and the heat labile enterotoxin of E. coli (LT). Mutated LTs (mLTs) which are less likely to convert to their active form than the native LT are described in WO 96/06627, WO 93/13202 and US 5,182,109.

Further immunostimulants which may advantageously be included are saponin derivatives such as QS21 and monophosphoryl lipid A, in particular 3-de-O-acylated monophosphoryl lipid A (3D-MPL). Purified saponins as oral adjuvants are described in WO 98/56415. Saponins and monophosphoryl lipid A may be employed separately or in

combination (e.g. WO 94/00153) and may be formulated in adjuvant systems together with other agents. 3D-MPL is a well-known adjuvant manufactured by Ribi Immunochem, Montana and its manufacture is described in GB 2122204.

A general discussion of vehicles and adjuvants for oral immunisation can be found in Vaccine Design, The Subunit and Adjuvant Approach, edited by Powell and Newman, Plenum Press, New York, 1995.

The disclosure also relates to a method for vaccinating human subjects, especially infants, by administering to a subject in need thereof an effective amount of a vaccine composition according to the invention. Suitably the live attenuated vaccine is administered by oral administration.

In a specific aspect the attenuated rotavirus strain for use according to the invention is formulated with an antacid to minimise inactivation of the vaccine by acid in the stomach. Suitable antacid components include inorganic antacids for example aluminium hydroxide $Al(OH)_3$ and magnesium hydroxide $Mg(OH)_2$. Commercially available antacids which are suitable for use in the invention include Mylanta (trade mark) which contains aluminium hydroxide and magnesium hydroxide. These are insoluble in water and are given in suspension.

Aluminium hydroxide is a particularly suitable component of a vaccine composition for use according to the invention as it can provide not only an antacid effect but also an adjuvantation effect.

Also suitable for use as antacids in the vaccine of the invention are organic antacids such as organic acid carboxylate salts. A suitable antacid in the vaccine composition for use according to of the invention contains an organic acid carboxylate salt, specifically a salt of citric acid such as sodium citrate or potassium citrate.

A particularly suitable antacid that may be used in the vaccine composition for use according to the present invention is the insoluble inorganic salt, calcium carbonate ($CaCO_3$). The calcium carbonate is able to associate with the rotavirus and the rotavirus activity is maintained during the association with the calcium carbonate.

To prevent sedimentation of calcium carbonate during the filling step, viscous agents are suitably present in the formulation.

Possible viscous agents that may be used include pseudoplastic excipients. A pseudoplastic solution is defined as a solution having higher viscosity on standing compared to its viscosity under agitation. Excipients of this type are natural polymers such as arabic gum, adragante gum, agar-agar, alginates, pectines or semi-synthetic polymers for example: carboxymethylcellulose (Tyloses C®), methylcellulose (Methocels A®, Viscotrans MC®, Tylose MH® and MB®), hydroxypropylcellulose (Klucels®), and hydroxypropylmethylcellulose (Methocels E® and K®, Viscontrans MPHC®). In general those pseudoplastic excipients are used together with thixotropic agents. Alternative viscous agents that may be used are pseudoplastic excipients with low flowing capacity. Those polymers, at a sufficient concentration, give rise to a structural fluid arrangement resulting in a high viscosity solution having low flowing capacity on standing. A certain quantity of energy needs to be given to the system to allow flowing and transfer.

External energies (agitation) are needed to destroy temporarily the structural fluid arrangement in order to obtain a fluid solution.

Examples of such polymers are Carbopols® and xanthane gum.

Thixotropic excipents become a gel structure on standing whilst under agitation they form a fluid solution. Examples of thixotropic excipients are: Veegum ®(Magnesium-aluminium silicate) and Avicel RC® (about 89% microcrystalline cellulose and 11% Carboxymethylcellulose Na).

The vaccine composition for use according to the present invention suitably comprises a viscous agent selected from xanthane gum or starch.

Thus the vaccine composition for use according to the present invention is typically formulated with a combination of calcium carbonate and xanthane gum.

Other components of a composition used in the invention suitably include sugars for example sucrose and/or lactose.

The vaccine composition for use according to the invention may contain additional components including for example flavourings (particularly for an oral vaccine) and bacteriostatic agents.

Different presentations of the vaccine composition for use according to the invention are envisaged.

In one aspect, the disclosure relates to a vaccine administered as a liquid formulation. Suitably the liquid formulation is reconstituted prior to administration from at least the following two components:

i) virus component
ii) liquid component.

In this aspect, the virus component and the liquid component are normally present in separate containers, which may conveniently be separate compartments of a single vessel, or separate vessels which can be connected in such a way that the final vaccine composition is reconstituted without exposing it to the air.

Prior to reconstitution, the virus may be in a dry form or a liquid form. Suitably the virus component is lyophilised. Lyophilised virus is more stable than virus in an aqueous solution. The lyophilised virus may be suitably reconstituted using a liquid antacid composition to produce a liquid vaccine formulation. Alternatively the lyophilised virus may be

reconstituted with water or aqueous solution, in which case the lyophilised virus composition suitably contains an antacid component.

Suitably, the vaccine formulation comprises a virus component formulated with calcium carbonate and xanthane gum in one compartment or vessel and this is reconstituted with water or aqueous solution present in the second compartment or vessel.

In another aspect, the vaccine composition as disclosed therein is a solid formulation, suitably a lyophilised cake which is suitable for immediate dissolution when placed in the mouth. Lyophilised formulations may conveniently be provided in the form of tablets in a pharmaceutical blister pack.

In another aspect, the disclosure describes a rotavirus vaccine in the form of a quick dissolving tablet for oral administration.

In another aspect, the disclosure describes a composition comprising a live attenuated rotavirus strain, in particular a human rotavirus strain, wherein the composition is a lyophilised solid capable of immediate dissolution when placed in the mouth.

Suitably the quick dissolving tablet according to the invention dissolves in the mouth of the subject sufficiently quickly to prevent swallowing of the undissolved tablet. This approach is particularly advantageous for paediatric rotavirus vaccines.

Suitably the virus is a live attenuated human rotavirus which is formulated with an inorganic antacid such as calcium carbonate and a viscous agent such as xanthane gum. A further aspect of the present disclosure is the description of a lyophilised formulation wherein the virus component is any rotavirus strain which is formulated with calcium carbonate and xanthane gum.

Vaccines for use according to the invention may be formulated and administered by known techniques, using a suitable amount of live virus to provide effective protection against rotavirus infection without significant adverse side effects in typical vaccinees. A suitable amount of live virus will normally be between $10^4$ and $10^7$ focus forming units (ffu) per dose. A typical dose of vaccine may comprise $10^5$ - $10^6$ffu per dose and may be given in several doses over a period of time, for example in two doses given with a two-month interval. Benefits may however be obtained by having more than 2 doses, for example a 3 or 4 dose regimen, particularly in developing countries. The interval between doses may be more or less than two months long. An optimal amount of live virus for a single dose or for a multiple dose regimen, and optimal timing for the doses, can be ascertained by standard studies involving observation of antibody titres and other responses in subjects,

The vaccine for use according to the invention may also comprise other suitable live viruses for protection against other diseases, for example poliovirus. Alternatively other suitable live virus vaccines for oral administration may be given in a separate dose but on the same occasion as the rotavirus vaccine composition according to the invention.

Sera from twelve 4 to 6 month old infants vaccinated with the P33 material as described in the Vaccine (1998) paper were tested for neutralization of P33, P38, P43 and 89-12C2.

The range of neutralization titers of all the tested sera is similar for P33, P38 and P43. The statistical analysis shows no significant difference in the overall neutralization titers against all three viruses. This suggests that the conformational and non-conformational neutralization epitopes of P33, P38 and P43 are equally well recognized by the anti-P33 sera of P33 vaccinated infants. This observation indirectly suggests that the neutralization epitopes revealed in this in vitro assay were not altered between P33, P38 and P43.

The range of neutralization titers of P89-12C2 however significantly differs from P33, P38 and P43. This observation suggests that the conformational and non-conformational neutralization epitopes of P33, P38 and P43 are not equally well recognized by the anti-P33 sera of P33 vaccinated infants. This observation indirectly suggests that the neutralization epitopes revealed in this in vitro assay were altered between 89-12 C2 and P33, P38 and P43.

Immunogenic fragments may be defined in the context of this invention as fragments that when administered at an effective dose (either alone or as a hapten bound to a carrier) elicit a protective immune response against rotavirus infection.

The following, non-limiting, examples illustrate the invention.

## EXAMPLES

### EXAMPLE 1: Demonstration that strain 89-12 at passage 26 (P26) is a mixture of variants

#### *Sequencing of VP4 and VP7 genes from different passage lots*

Sequencing of VP4 and VP7 genes from passage P26 (primary AGMK cells), passage P33 (established (as opposed to primary) AGMK cell line), passage P41 and passage P43 was performed. Total RNA extraction was reverse transcribed and amplified through PCR in one tube/one step.

Primers Rota 5bis and Rota 29bis amplified the entire VP4 gene and primers Rota 1 and Rota 2bis amplified

the entire VP7 gene. The PCR material has been sequenced using different primers (see Table 1).

The passage P26 sequence differed from the passage P33 sequence by 3 bases (at positions 501, 788 and 802 bp from the start codon) in VP4 and by three bases in VP7 (108, 605 and 897 bp from the start codon).

The passage P26 sequence scans of VP4 and VP7 show at mutated positions the presence of the passage P33 sequence as a background. Thus it can be seen that passage P26 is a mixture of at least 2 variants.

The passage P33 sequence scans seem homogenous in VP4 and heterogeneous for VP7 (see Table 2).

Passage P38 (derived from passage 33) was passaged 5 times on Vero cells and displayed the same set of VP4 and VP7 sequences as passage P33 (AGMK cell line). Thus there was no major change in populations between P33 and P38.

TABLE 1: Oligonucleotides used for RT-PCR and sequencing

| | name | sequence | position |
|---|---|---|---|
| VP7 | Rota 1 | GGC TTT AAA AGA GAG AAT TTC CGT CTG G (SEQ ID NO:11) | -49 to -22 |
| | Rota 1bis | GGT TAG CTC CTT TTA ATG TAT GGT A (SEQ ID NO:12) | -16 to 10 |
| | Rota 2bis | GGT CAC ATC GAA CAA TTC TAA TCT AAG (SEQ ID NO:13) | 1014-988 |
| | Rota 7 | CAA GTA CTC AAA TCA ATG ATG G (SEQ ID NO:14) | 266-287 |
| | Rota 12 | TGT TGA TTT TTC TGT CGA TCC AC (SEQ ID NO:15) | 372-394 |
| | Rota 46 | GGT TGC TGA GAA TGA GAA ATT AGC TAT AGT GG (SEQ ID NO:16) | 651-682 |
| | Rota 18 | CCA CTA TAG CTA ATT TCT CAT TCT CAG CAA CC (SEQ ID NO:17) | 682-651 |
| VP4 | Rota 5 | TGG CTT CGC CAT TTT ATA GAC A (SEQ ID NO:18) | 2-23 |
| | Rota 6 | ATT TCG GAC CAT TTA TAA CC (SEQ ID NO:19) | 878-859 |
| | Rota 5bis | TGG CTT CAC TCA TTT ATA GAC A (SEQ ID NO:20) | 2-23 |
| | Rota 6bis | ATT TCA GAC CAT TTA TAA CCT AG (SEQ ID NO:21) | 878-856 |
| | Rota 25 | GGA GTA GTA TAT GAA AGT ACA AAT AAT AG (SEQ ID NO:22) | 268-296 |
| | Rota 26 | CTA TTA TTT GTA CTT TCA TAT ACT ACT CC (SEQ ID NO:23) | 296-268 |
| | Rota 27bis | TCG ATA CAG TAT AAG AGA GCA CAA G (SEQ ID NO:24) | 721-745 |
| | Rota 28 | TTC ATT AAC TTG TGC TCT CTT ATA CTG (SEQ ID NO:25 | |
| | Rota 31 | GTA TAT GTA GAC TAT TGG GAT G (SEQ ID NO:26) | 753-727 |
| | Rota 32 | CAT CCC AAT AGT CTA CAT ATA C (SEQ ID NO:27) | 1048-1070 |
| | Rota 45 | TGT AAC TCC GGC AAA ATG CAA CG (SEQ ID NO:28) | 1070-1048 |
| | Rota 53 | CGT TGC ATT TTG CCG GAG TTA CA (SEQ ID NO:29) | 1205-1227 |
| | Rota 54 | GTA AGA CAA GAT TTA GAG CGC CA (SEQ ID NO:30) | 1227-1205 |
| | Rota 55 | TGG CGC TCT AAA TCT TGT CTT AC (SEQ ID NO:31) | 1465-1487 |
| | Rota 40 | CTT GAT GCT GAT GAA GCA GCA TCT G (SEQ ID NO:32) | 1487-1465 |
| | Rota 39 | CAG ATG CTG CTT CAT CAG CAT CAA G (SEQ ID NO:33) | 1703-1727 |
| | Rota 33 | CGA TCA TAT CGA ATA TTA AAG GAT G (SEQ ID NO:34) | 1727-1703 |
| | Rota 34 | CAT CCT TTA ATA TTC GAT ATG ATC G (SEQ ID NO:35) | 2008-2032 |
| | Rota 29bis | AGC GTT CAC ACA ATT TAC ATT GTA G (SEQ ID NO:36) | 2032-2008 |
| | | | 2335-2311 |

TABLE 2: oligonucleotides used in hybridization

| | name | sequence | position |
|---|---|---|---|
| VP7 | Rota 41 | AGT ATT TTA TAC TAT AGT AGA TTA TAT TAA TC (SEQ ID NO:37) | 882-913 |
| | Rota 42 | AGT ATT TTA TAC TAT GGT AGA TTA TAT TAA TC (SEQ ID NO:38) | 882-913 |
| VP4 | Rota 15 | ATC CCC ATT ATA CTG CAT TCC TTT C (SEQ ID NO:39) | 807-783 |
| | Rota 16 | ATC CCT ATT ATA CTG CAT TTC TTT C (SEQ ID NO:40) | 807-783 |
| | Rota 35 | ATC CCC ATT ATA CTG CAT TTC TTT C (SEQ ID NO:41) | 807-783 |
| | Rota 36 | ATC CCT ATT ATA CTG CAT TCC TTT C (SEQ ID NO:42) | 807-783 |

The bases shown in bold type in Table 2 are the sites of specific sequence variation in VP4 and VP7.

TABLE 3: sequence variation of VP4 and VP7 genes

Table 3.1

| | VP4 | | | VP7 | | |
|---|---|---|---|---|---|---|
| | 501 bp 167 aa | 788 bp 263 aa | 802 bp 268 aa | 108 bp 36 aa | 605 bp 202 aa | 897 bp 299 aa |
| P26 (AGMK) | A | G/A | G/A | A | C/T | A |
| P33 (AGMK) | T | A | A | G/A | T/C | A/G |
| P38 (VERO) | T | A | A | A/G | T | G/A |
| P43 (VERO) | T | A | A | A | T | A |

N.B. In a second clone from the 3 clones which were developed to the level of production lot, the VP7 897 bp position nucleotide is G, rather than A as in the P43 selected clone. This results in a methionine in place of an isoleucine in the amino acid sequence. Variants corresponding to both the selected P43 clone and the clone in which there is a G in VP7 at 897 bp from the start codon, were excreted in the stools of infants who had been vaccinated with the P33 material.

In Table 3.1, where there are two alternative bases at a particular position, the first of the two represents the base which appears in a major population and the second is the base which appears in a minor population. Major and minor variant populations are judged by the strength of the signal in sequencing.

Table 3.2

| | VP4 | | | VP7 | | |
|---|---|---|---|---|---|---|
| | 501 bp 167 aa | 788 bp 263 aa | 802 bp 268 aa | 108 bp 36 aa | 605 bp 202 aa | 897 bp 299 aa |
| P26 (AGMK) | Leu | Gly/Glu | Gly/Arg | Arg | Thr/Met | Ile |
| P33 (AGMK) | Phe | Glu | Arg | Arg/Arg | Met/Thr | Ile/Met |
| P38 (VERO) | Phe | Glu | Arg | Arg/Arg | Met | Met/Ile |
| P43 (VERO) | Phe | Glu | Arg | Arg | Met | Ile |

Table 3.2 shows the amino acid changes resulting from the nucleotide differences between the variants.

TABLE 4

| | VP4 ( 788 -802 positions ) | | | | VP7 ( 897 position ) | |
|---|---|---|---|---|---|---|
| | G-G | A-A | A-G | G-A | A | G |
| Probes | Rota 15 | Rota 16 | Rota 35 | Rota 36 | Rota 41 | Rota 42 |
| Passages | | | | | | |
| P26 | - | + | + | + | nd | nd |
| P33 | - | + | - | - | ++ | + |
| P38 | - | + | - | - | + | ++ |
| P43 | - | + | - | - | + | - |

***Slot blot hybridization***

The change in populations between passages P26 to P33 on AGMK cells has been further confirmed by slot blot hybridization. The VP4 and the VP7 gene fragments generated by RT/PCR were hybridized with oligonucleotide probes specific for each variant (see Table 3.1 and 3.2). In contrast to P26 which hybridized with Rota 16, Rota 35 and Rota 36 and not with Rota 15, the VP4 PCR fragment of the P33 material, at positions 788 and 802 hybridized only with Rota 16 and not with either Rota 15 or Rota 35 or Rota 36. These results established the presence of at least 3 variants

in P26 (see Table 4).

For the VP7 PCR fragment of the P33 material, position 897 hybridized with Rota 41 and Rota 42. These results established the presence of at least two variants in the P33 material.

**EXAMPLE 2: Isolation and characterization of the P43 clone**

To isolate P33 components as a homogeneous virus population, three end-point dilutions of P33/AGMK on Vero cells were performed and the resulting virus was used to infect Vero cells.

Positive wells were selected using two criteria: growth demonstrated by the largest number of foci detected in the wells and the most isolated positive wells on the plates, as is done classically. After 3 end dilution passages in 96 well microtiter plates, 10 positive wells were amplified successively on Vero cells and evaluated for their yield.

Based on yield, three clones were developed to passage level of production lot. Immunorecognition by polyclonal antibodies was shown to be similar both between the three clones and between the clones and P33. Homogeneity of the clones was assessed by slot blot hybridization. The final selection of a single clone was based on yield and sequence.

The selected clone was amplified by successive passages on Vero cells to generate a Master seed, a Working seed and finally production lots.

The selected clone was genetically characterized at different passage levels by sequencing of VP4 and VP7 (identity) and by specific slot blot hybridization of the VP4 and VP7 (homogeneity) of the PCR amplified materials. The sequence of the VP4 and VP7 genes of the P43 material are given in Figures 1 and 2 respectively and are identical to P41.

Homogeneity of the selected clone was assessed by a selective hybridization using oligonucleotide probes discriminating nucleotide changes in VP4 and/or VP7 regions for each variant identified during sequencing of P26/primary AGMK (see Table 4).

The VP4 fragment hybridized with Rota 16 and not with Rota 15, Rota 35 or Rota 36. The VP7 fragment hybridized with Rota 41 and not with Rota 42.

These results confirmed that P43 is a homogeneous population.

**EXAMPLE 3: Removal of potential adventitious virus**

Ether was added to P33 (AGMK grown) to a final concentration of 20% for 1 hr. Ether was then bubbled out with $N_2$ for 35 min. No impact on the titre of P33 seed was observed.

**EXAMPLE 4: Formulation of a live attenuated vaccine**

The production lots described above are formulated for oral adminstration to infants by the following method.

1. <u>Lyophilised virus</u>

Standard techniques are used for preparing virus doses. Frozen purified viral bulk is thawed and diluted with appropriate medium composition, in this case Dulbecco's modified eagle Medium, up to a desired standard viral concentration, in this case $10^{6.2}$ ffu/ml. The diluted virus is then further diluted with lyophilisation stabiliser (sucrose 4%, dextran 8%, sorbitol 6%, amino-acid 4%) up to the target viral titre, in this case $10^{5.6}$ ffu/dose. 0.5 ml aliquots of stabilised virus composition are aseptically transferred to 3 ml vials. Each vial is then partially closed with a rubber stopper, the sample is freeze dried under a vacuum, the vial is then fully closed and an aluminium cap is crimped in place around the vial to keep the stopper in place.

For use, the virus is reconstituted using one of the following antacid reconstituents:

<u>(a) Citrate reconstituent</u>

Sodium citrate is dissolved in water, sterilized by filtration and aseptically transferred into reconstituent containers in 1.5 ml amounts at a concentration of 544 mg $Na_3Citrate.2H_2O$ per 1.5ml dose. The reconstituent containers may be for example 3 ml vials, or 4 ml vials, or 2 ml syringes, or soft plastic squeezable capsules for oral administration. As an alternative to maintaining sterile components under sterile conditions, the final container can be autoclaved.

<u>(b) $Al(OH)_3$ reconstituent</u>

An aseptic aluminium hydroxide suspension (Mylanta - trademark) is aseptically diluted in sterile water, aseptically transferred to reconstituent containers (for example 2 ml syringes, or soft plastic squeezable capsules) in 2 ml amounts each containing 48 mg $Al(OH)_3$. An alternative to using sterile components under sterile conditions is to y

irradiate the aluminium hydroxide suspension (preferably at a diluted stage).

Standard ingredients are included to prevent the suspension from settling. Such standard ingredients include for example magnesium stearate, carboxymethylcellulose, hydroxypropylmethylcellulose, microcrystalline cellulose, and silicone polymers. Bacteriostatic agents for example butylparaben, propylparaben or other standard bacteriostatic agents used in food, and flavourings, may also be included.

2. Lyophilised virus with $Al(OH)_3$ in liquid formulation

Standard techniques are used for preparing virus doses. Frozen purified viral bulk is thawed and diluted with appropriate medium composition, in this case Dulbecco's modified eagle Medium, up to a desired standard viral concentration, in this case $10^{6.2}$ ffu/ml. Aluminium hydroxide suspension is added to reach a final quantity of 48 mg/dose and the virus composition is diluted with lyophilisation stabiliser (sucrose 4%, dextran 8%, sorbitol 6%, amino-acid 4%) up to the target viral titre, in this case $10^{5.6}$ ffu/dose. 0.5 ml aliquots of stabilised virus composition are aseptically transferred to 3 ml vials. Lyophilisation and closing of the vials is then carried out as described in part 1.

3. Lyophilized virus with $Al(OH)_3$ for blister presentation

Standard techniques are used for preparing virus doses. Frozen purified viral bulk is thawed and diluted with appropriate medium composition, in this case Dulbecco's modified eagle Medium, up to a desired standard viral concentration, in this case $10^{6.2}$ ffu/ml. Aluminium hydroxide suspension is added to reach a final quantity of 48 mg/dose and the virus composition is diluted with lyophilisation stabiliser which may be sucrose, dextran or amino-acid 4%, or gelatin, or vegetal peptone, or xanthane up to the target viral titre of $10^{5.6}$ ffu/dose. An aseptic filling operation is employed to transfer doses of 0.5 ml or preferably less to blister cavities. The composition is lyophilised, and the blister cavities are sealed by thermic sealing.

Optionally standard ingredients are included to prevent the aluminium hydroxide suspension from settling. Such standard ingredients include for example magnesium stearate, carboxymethylcellulose, hydroxypropylmethylcellulose, microcrystalline cellulose, and silicone polymers. Flavourings may also be included.

Example 5: Rotavirus viral titration for various formulations

**5.1:Comparison between lactose and sucrose based formulations:**

Table 5

| Batch n° | Formulation composition | Viral titer before lyophilisation | Viral titer after lyophilisation and 1 week at 37°C |
|---|---|---|---|
| 98G06/01 | Lactose:2%;<br>Dextran:4%;<br>Sorbitol:3%;<br>AminoAcids:2% | $10^{5.22}$ | $10^{4.67}$ |
| 98G06/03 | Sucrose:2%;<br>Dextran:4%;<br>Sorbitol:3%;<br>AminoAcids:2% | $10^{5.28}$ | $10^{4.92}$ |

P43 rotavirus was formulated either with sucrose or with lactose as shown in the table above.

Viral titration before lyophilisation is the viral titre in the completed formulated liquid (containing sucrose dextran sorbitol aminoacids) and without the lyophilisation step.

Good results are those in which a $<0.5$ log decrease at the lyophilisation step and $<0.5$ log decrease during the "1 week at 37°C" (accelerated stability test) are achieved.

The precision of the viral titration is around + or - 0.2 log.

The results indicate that sucrose may be used instead of lactose.

**5.2: Effect of arginine and replacement of sorbitol by maltitol:**

Table 6

| Batch n° | Fomulation composition | Viral titer at time = zero after lyophilisation | Viral titer after lyopjhilisation and 1 week at 37°C |
|---|---|---|---|
| 98L16/01 | Lactose:2%; Dextran:4%; Sorbitol:3%; AminoAcids:2% | $10^{4.8}$ | $10^{4.8}$ |
| 98L16/02 | Lactose:2%; Dextran:4%; Sorbitol:3%; AminoAcids:2% Arginine: 3% | $10^{4.8}$ | $10^{4.9}$ |
| 98L16/04 | Lactose:2%; Dextran:4%; Maltitol:3%; AminoAcids:2% Arqinine: 3% | $10^{4.7}$ | $10^{5}$ |

The results demonstrate that the addition of arginine (which is known to improve the stability of the virus during lyophilisation and also provides a basic medium in order to compensate for the stomach acidity) maintains the viral titer.

Sorbitol tends to decrease the glass transition temperature of the lyophilised cake by too great a degree. This can be overcome by using maltitol instead of sorbitol as shown above and the viral titer is still maintained.

**5.3: Various formulation compositions**

This experiment demonstrates that a number of formulations are possible.

Table 7

| Batch n° | Fomulation composition | Viral titer before lyophilisation | Viral titer after lyophilisation and 1 week at 37° |
|---|---|---|---|
| 99C11/01 | Sucrose:2%; Dextran:4%; Sorbitol:3%; AminoAcids:2% | $10^{5.24}$ | $10^{5.07}$ |
| 99C11102 | Sucrose:2%; Dextran:4%; Maltitol:3%; AminoAcids:2% | $10^{5.09}$ | $10^{4.92}$ |
| 99C11/04 | Dextran:4%; Maltitol:3%; AminoAcids:2% | $10^{4.89}$ | $10^{5.06}$ |
| | | | |
| Batch n° | Fomulation composition | Viral titer at time = zero after lyophilisation | Viral titer after lyophilisation and 1 week at 37° |
| 99C17/01 | Sucrose:2%; Dextran:4%; | $10^{5.40}$ | $10^{5.41}$ |

(continued)

| Batch n° | Fomulation composition | Viral titer before lyophilisation | Viral titer after lyophilisation and 1 week at 37° |
|---|---|---|---|
| | Sorbitol:3%; AminoAcids:2% | | |
| 99C17/02 | Sucrose:2%; Dextran:4%; Sorbitol:1.5%; AminoAcids:2% | $10^{5.30}$ | $10^{4.93}$ |
| 99C17/03 | Sucrose:2%; Dextran:4%; AminoAcids:2% | $10^{5.31}$ | $10^{5.24}$ |
| 99C17/04 | Sucrose:2%; Dextran:4%; Maltitol:3%; AminoAcids:2% | $10^{4.42}$ | $10^{4.45}$ |
| 99C17/05 | Sucrose:2%; Dextran:4%; Maltitol:1.5%; AminoAcids:2% | $10^{4.39}$ | $10^{4.40}$ |
| 99C17/06 | Sucrose:2%; Dextran:4%; Sorbitol:3%; | $10^{5.44}$ | $10^{4.97}$ |
| 99C17/07 | Sucrose:2%; Dextran:4%; Sorbitol:1.5%; | $10^{5.11}$ | $10^{4.89}$ |

**5.4: Association between Rotavirus and $Al(OH)_3$ antacid:**

Table 8

| Rotavirus | $Al(OH)_3$ | $H_2O$ | Contact time at room temperature | Centrifugation | Supernatant viral titer in ffu/ml | Pellets viral titer in ffu/ml |
|---|---|---|---|---|---|---|
| $10^{5.6}$ ffu/ml | 48 mg in 0.240ml | 0.76 ml | 30 min | 8000rpm, 10 min | $10^{3.66}$ | |
| $10^{5.6}$ ffu/ml | 0.48 mg in 0.240ml | 0.76 ml | 30 min | 8000rpm, 10 min | $10^{4.41}$ | |
| $10^{5.6}$ ffu/ml | | 1 ml | 30 min | 8000rpm, 10 min | $10^{5.68}$ | |
| Rotavirus in Lyophilised Cake | 12 mg in 0.120ml | 1.38 0ml | 30 min | 8000rpm, 10 min | Below detection | $10^{4.7}$ |

$Al(OH)_3$ is used as an antacid. This shows that Rotavirus is associated with the insoluble inorganic salt ($Al(OH)_3$) since it centrifuged together with the $Al(OH)_3$ (decrease of viral activity in the supernatant).

**5.5: Dissolution of $Al(OH)_3$ antacid by SodiumCitrate before viral titration**

Table 9

| Viral samples | Dissolution | Conditions | Viral titers ffu/ml |
|---|---|---|---|
| 99B10/06 liquid formulation before lyophilisation; $10^{5.43}$ | 1.5ml $Na_3$Citrate | 24h at room temperature | $10^{5.11}$ |
| 99B10/06:lyophilized $10^{5.43}$ | 1.5ml $Na_3$Citrate | 24h at room temperature | $10^{4.53}$ |

When Rotavirus is associated with the $Al(OH)_3$, it is possible to lyophilise everything (including the $Al(OH)_3$). After lyophilisation, it is possible to recover the Rotavirus by dissolving $Al(OH)_3$ in SodiumCitrate. This step does not damage the Rotavirus and retains its activity after this dissolution step.

**5.6: Infectivity of Rotavirus after liberation of the $Al(OH)_3$-Rotavirus association:**

The mechanism of virus liberation (by dissolution of the carrier) may very well occur *in vivo.* Indeed below pH 6, aluminium hydroxide becomes completely soluble, and thus, Rotavirus will be liberated in the stomach.

$$Al(OH)_3 + 3\ H^+ \dashrightarrow Al^{+++} \text{ (water soluble)} + 3\ H_2O$$

In the stomach, $Al^{+++}$ ions are not absorbed (J.J. Powell, R.Jugdaohsingh and R.P.H. Thompson, The regulation of mineral adsorption in the gastrointestinal track, Proceedings of the Nutrition Society (1999), 58, 147-153).

In the intestine, due to the increase of pH, insoluble forms of aluminium are precipitated ($Al(OH)_3$ or $AlPO_4$), and eliminated by the natural way.

It is unknown whether the newly formed $Al(OH)_3$ (or $AlPO_4$) precipitate will be able to reassociate with free Rotavirus. This raises the question of the infectivity of the $Al(OH)_3$-Rotavirus association itself.

Liberation of Rotavirus from the $Al(OH)_3$-Rotavirus association by other mechanisms is also possible. Lysine, for example, interferes with the viral adsorption on $Al(OH)_3$.

Other anions like borate, sulfate, carbonate and phosphate are known to be specifically adsorbed on aluminium hydroxide, thus, theoretically, it should be possible to displace (by competition for the adsorption site) Rotavirus from the $Al(OH)_3$-Rotavirus association.

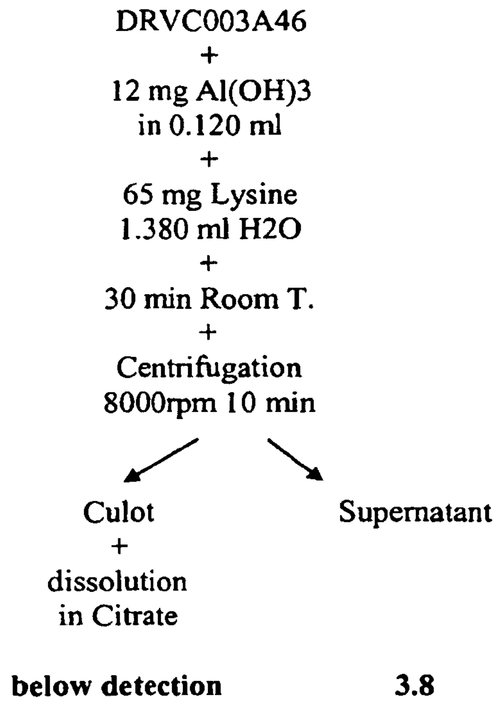


Thus, Rotavirus may be liberated from the Rotavirus - $Al(OH)_3$ association and the liberated Rotavirus remains active.

This liberation can be done either by dissolving $Al(OH)_3$ (by HCl in the stomach, or by $Na_3$Citrate in vitro) or by displacing Rotavirus by a basic amino acid (lysine).

**5.7: Infectivity of the $Al(OH)_3$-Rotavirus association**

A single dose of lyophilised Rotavirus was reconstituted with water and divided into two parts. The first part, considered as the reference, received an additional volume of water. The second part received 24mg of $Al(OH)_3$ suspended in 0.240 ml of water (Preclinical viral titrations).

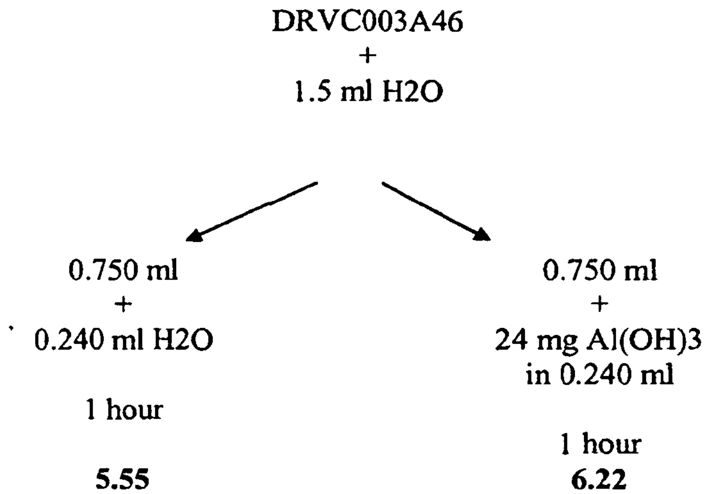


When $Al(OH)_3$ is present, Rotavirus is active and the viral titration value is higher compared to the reference sample.

This experiment was repeated without dividing the lyophilised dose, and by adding 12 mg $Al(OH)_3$ or 24 mg $Al(OH)_3$.

Here the reference sample was the one reconstituted with a Citrate-Bicarbonate buffer. Thus, the viral titer is again higher in the presence of $Al(OH)_3$.

| DRVC003A46 + 1.5 ml WL buffer | DRVC003A46 + 12 mg Al(OH)3 in 0.120ml + 1.380ml H2O | DRVC003A46 + 24 mg Al(OH)3 in 0.240ml + 1.260ml H2O |
|---|---|---|
| **5.34** | **6.24** | **6.05** |
| **5.32** | **5.95** | **6.26** |

As in the example above, Rotavirus associates with the $Al(OH)_3$ particles, since the virus can be discarded by centrifugation. DRVC003A46 is a lyophilised formulated Rotavirus (Sucrose:2%; Dextran: 4%,Sorbitol:3%; Amino-acids:2%).

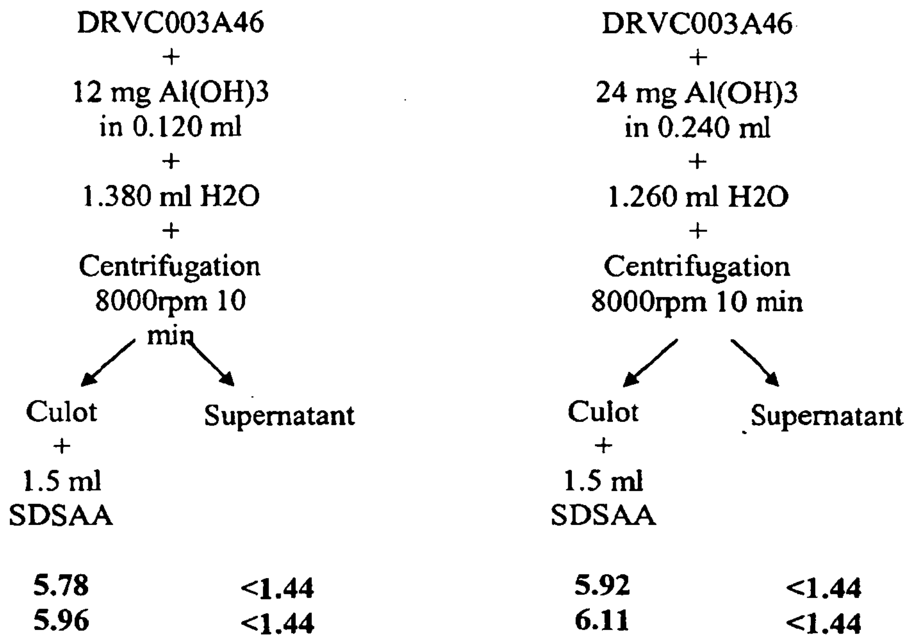


SDSAA= Sucrose 2%, Dextran 4%, Sorbitol 3%, Amino-Acid 2%.

According to the viral titration carried out on the supernatant, the quantity of $Al(OH)_3$ needed to adsorb Rotavirus seems to be low (starting with one lyophilised dose 5.7 log) scaling Up viral titration):

Table 10

| **Al(OH)3** | **Adsorption time** | **Titer in supernatant** |
|---|---|---|
| | | |
| 12 mg | 1 hour RT | 2.7 |
| 24 | 1 hour RT | 3.4 |
| 48 mg | 1 hour RT | 3.4 |
| 72 mg | 1 hour RT | 2.0 |
| 96 mg | 1 hour RT | Below detection |
| | | |
| 12 mg | Overnight | 2.7 |
| 24 mg | Overnight | Below detection |
| 48 mg | Overnight | 2.5 |
| | | |
| 12 mg | Immediate | Below detection |
| 24 mg | Immediate | 2.0 |

(continued)

| Al(OH)3 | Adsorption time | Titer in supernatant |
|---|---|---|
| 48 mg | Immediate | Below detection |

Time needed to adsorb Rotavirus on $Al(OH)_3$ seems to be short:
One dose of lyophilised Rotavirus was reconstituted in presence of 24 mg $Al(OH)_3$, and centrifuged after 0, 15, 60 min and 24 hours. The "culot" were resuspended in SDSAA before viral titration:

Table 11

| Time | Culot | Supernatant |
|---|---|---|
| 0 min | 5.26 | 3.17 |
| 15 min | 5.34 | <1.44 |
| 60 min | 5.96 | <1.44 |
| 24 hours | 6.13 | <1.44 |

### 5.8: Using $CaCO_3$ as antacid

In order to avoid aluminium in the vaccine, the antacid $Al(OH)_3$ was replaced by another insoluble inorganic salt: $CaCO_3$ (calcium carbonate).

The phenomena observed with $CaCO_3$ are parallel to those described for $Al(OH)_3$:

- Association of Rotavirus with the inorganic salt;
- Maintainance of Rotavirus activity when associated with the inorganic salt;
- Possibility of liberation of Rotavirus from the association by dissolution of the inorganic base by an acid;
- Possibility of co-lyophilisation of the antacid and the Rotavirus.

### $CaCO_3$ and Rotavirus association

In a first trial, lyophilised Rotavirus (viral titer 5.7) was reconstituted with a suspension of $CaCO_3$ in water (50mg in 1.5ml); and then centrifuged, and the viral titer of the supernatant compared to the pellet.

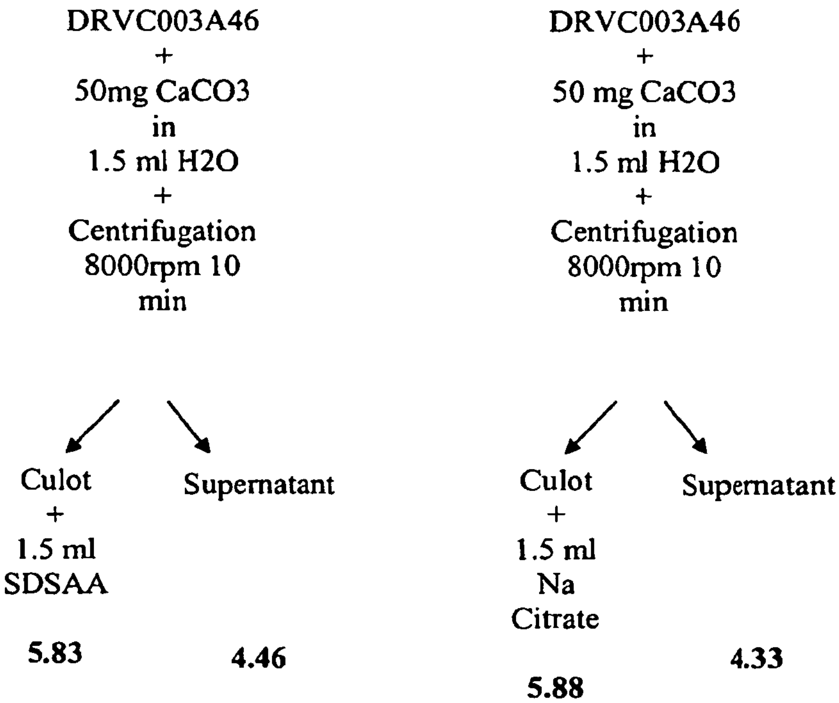


This indicates that more that 90% of the Rotavirus is associated with $CaCO_3$.

Also, when the virus was associated, it was possible to realise the titration and to recover the original viral quantities.

Also, viral titers are slightly higher that those obtained without $CaCO_3$.

**Quantity of $CaCO_3$ and Rotavirus association**

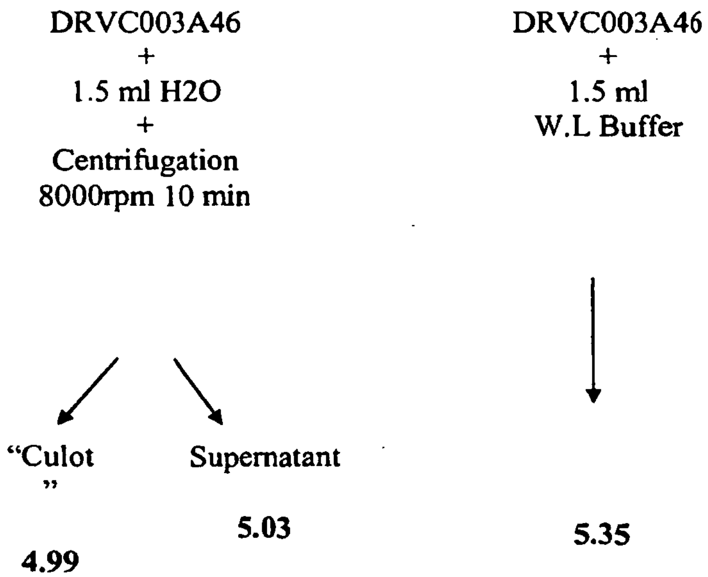


Lyophilised Rotavirus was reconstituted with a $CaCO_3$ suspension in water (1.5ml):

10 mg
50 mg
100 mg

and then centrifuged, and the viral titer of the supernatant compared to the culot.

Table 12

| CaCO3 | Extempo + Centri. | | 1 Hour + Centri | |
|---|---|---|---|---|
| | Culots | Surpernatant | Culots | Surpernatant |
| 100mg | 4.57 | 3.01 | 4.79 | 3.09 |
| 50mg | 4.17 | 4.15 | 4.22 | 3.86 |
| 10mg | 3.17 | 4.77 | 3.87 | 4.87 |

Thus, clearly, more $CaCO_3$ and more virus is associated, and less is found in the supernatant.

However, the full dose is not completely recovered (expected a total of 5.3 at least or even 5.8 as obtained earlier - see above).

**$CaCO_3$ Protection of Rotavirus during Baby Rossett-Rice antacid titration**

Using 10 doses of lyophilised Rotavirus (DRVC003A46) and 50mg of $CaCO_3$, two types of baby Rossett-Rice titration were carried out:

In a classic Rossett-Rice titration, the antacid is mixed with Rotavirus and HCl is poured into this medium.

In the "inverse" baby Rossett-Rice, the situation is the reverse: antacid is dropped into the HCl pool (as it occurs in vivo).

Table 13

| Classical baby Rossett-Rice titration | | | |
|---|---|---|---|
| Lyophi. Rota stored at: | Buffer | Theoretical Viral Titer | Measured Viral Titer |
| 4°C | 60 mg CaCO3 | 5.3 | 4.6 |
| -80°C | 60 mg CaCO3 | 5.3 | 4.6 |
| 4°C | 24 mg Al(OH)3 | 5.4 | <2.9 |
| -80°C | 24 mg Al(OH)3 | 5.4 | <2.9 |
| | | | |
| **Inverse baby Rossett-Rice titration** | | | |
| Lyophi. Rota stored at: | Buffer | Theoretical Viral Titer | Measured Viral Titer |
| 4°C | 60 mg CaCO3 | 5.3 | 4.6 |
| -80°C | 60 mg CaCO3 | 5.3 | 4.6 |
| 4°C | 24 mg Al(OH)3 | 5.4 | <2.9 |
| -80°C | 24 mq Al(OH)3 | 5.4 | <2.9 |

Thus, in this *in vitro* experiment, calcium carbonate is able to protect about 20% of Rotavirus from the presence of HCl, while aluminium hydroxide is not able to.

**5.9: Lyophilisation of Rotavirus in presence of $CaCO_3$ antacid:**

Table 14

| Batch n° | Composition | Viral titer at time = zero after lyophilisation | Viral titer after lyopjhilisation and 1 week at 37°C |
|---|---|---|---|
| 99K08/01 | Sucrose: 2%<br>Dextran: 4%<br>Sorbitol: 3%<br>Am. Acids: 2%<br>$CaCO_3$: 50 mg | $10^{5.28}$ | $10^{5.10}$ |
| 99K08/02 | Sucrose: 2%<br>Dextran: 4%<br>Sorbitol: 3%<br>Am. Acids: 2%<br>$CaCO_3$: 60 mg | $10^{5.16}$ | $10^{5.15}$ |
| 00C24/01 | Sucrose: 2%<br>Dextran: 4%<br>Sorbitol: 3%<br>Am. Acids: 2%<br>$CaCO_3$: 60 mg<br>Xanthane 0.3% | $10^{5.07}$ | $10^{4.69}$ |
| 00C24/03 | Sucrose: 2%<br>Dextran: 4%<br>Sorbitol: 3%<br>Am. Acids: 2%<br>$CaCO_3$: 60 mg<br>Xanthane 0.3% | $10^{5.07}$ | $10^{4.85}$ |
| 00E09/25 | Sucrose: 2%<br>Dextran: 4% | $10^{5.03}$ | $10^{4.91}$ |
| | Sorbitol: 3%<br>Am. Acids: 2%<br>$CaCO_3$: 60 mg<br>Xanthane 0.25% | | |
| 00E09/30 | Sucrose: 2%<br>Dextran: 4%<br>Sorbitol: 3%<br>Am. Acids: 2%<br>$CaCO_3$: 60 mg<br>Xanthane 0.30% | $10^{5.01}$ | $10^{4.87}$ |
| 00F26/06 | Sucrose: 2%<br>Dextran: 4%<br>Sorbitol: 3%<br>Am. Acids: 2%<br>$CaCO_3$: 60 mg<br>Starch: 2% | $10^{4.50}$ | $10^{4.70}$ |

This is the "all in one" - lyophilisation of Rotavirus and antacid (CaCO3) together in the same vial. To prevent sedimentation of $CaCO_3$ during the filling step, viscous agents are needed. Examples of such viscous agents include Xanthane gum and Starch. The Rotavirus activity is maintained even in the presence of Xanthane gum and Starch.

**5.10 Lyophilised tablets for quick disintegration when placed in the mouth:**

The following formulations demonstrate the "lyoc" concept. That is, quick dissolution of the lyophilised cake in the mouth.

Table 15

| Batch n° | Formulation composition | Viral titer before lyophilisation | Viral titer after lyopjhilisation and 1 week at 37° |
|---|---|---|---|
| 99B10/06 | Sucrose 4%<br>Sodium glutamate 3.7%<br>Al(OH)3 48mg | $10^{5.11}$ | $10^{4.53}$ |
| 99C11/12 | Maltitol 3%<br>Al(OH) 48mg<br>Hydroxypropylmethylcellulose: 1% | $10^{4.16}$ | $10^{3.79}$ |
| | | | |
| Batch n° | Fomulation composition | Viral titer at time = zero after lyophilisation | Viral titer after lyopjhilisation and 1 week at 37° |
| 00C24/05 | Sucrose: 2%<br>Dextran: 4%<br>Sorbitol: 3%<br>Am. Acids: 2%<br>$CaCO_3$: 60 mg<br>Xanthane 0.3% | $10^{5.02}$ | $10^{4.54}$ |
| 00C24/06 | Sucrose: 2%<br>Dextran: 4%<br>Sorbitol: 3%<br>Am. Acids: 2%<br>$CaCO_3$: 60 mg<br>Xanthane 0.3% | $10^{4.86}$ | $10^{4.56}$ |
| 00F26/11 | Sucrose: 1%<br>Dextran: 2%<br>Sorbitol: 1.5%<br>Am. Acids: 1%<br>$CaCO_3$: 60 mg<br>Starch: 2% | $10^{4.70}$ | $10^{4.40}$ |

In the "lyoc concept" both Xanthane and Starch can be used (maintaining the quick dissolution properties of the lyophilised cake).

**EXAMPLE 6: Use of Calcium Carbonate as the antacid for the Rotavirus vaccine composition**

When a suspension of $CaCO_3$ in water is used as the antacid for Rotavirus there is a problem that the calcium carbonate particles sediment rapidly when placed in water since the powder density value approaches 2.6 and the average particle size is 30$\mu$m. This sedimentation can be slowed by:

1 increasing the density of the surrounding medium
2 increasing the viscosity of the surrounding medium
3 reducing the particles size
4 keeping particles away from each other

6.1: Increasing density of the surrounding medium:

When the $CaCO_3$-Water suspension (when placed in the syringe) is placed on the lyophilised cake (containing sucrose 2%, dextran 4%; sorbitol 3%; amino-acids 2%) the density of the surrounding medium is increased, but the speed of $CaCO_3$ sedimentation is not very much different from the $CaCO_3$-Water suspension.

6.2 increasing the viscosity of the surrounding medium:

**Pseudoplastic excipents**

A pseudoplastic solution is defined as a solution having higher viscosity on standing compared to its viscosity under agitation.
Usual excipients of this type are:

natural Polymers for example:

arabic gum
adragante gum
agar-agar
alginates
pectines

semi-synthetic polymers for example:

carboxymethylcellulose (Tyloses C®)
methylcellulose (Methocels A®, Viscotrans MC®, Tylose MH® and MB®)
hydroxypropylcellulose (Klucels®)
hydroxypropylmethylcellulose (Methocels E® and K®, Viscontrans MPHC®)

In general those pseudoplastic excipients are used together with thixotropic agents.

**Pseudoplastic excipients with low flowing capacity**

Those polymers, at a sufficient concentration, give rise to a structural fluid arrangement resulting in a high viscosity solution having low flowing capacity on standing. A certain quantity of energy needs to be given to the system to allow flowing and transfer. External energies (agitation) are needed to destroy temporarily the structural fluid arrangement in order to obtain a fluid solution.
Examples of such polymers are Carbopols® and Xanthane gum.

**Thixotropic excipents**

With these excipents, on standing, a gel structure is obtained; while under agitation a fluid solution is obtained.
Examples of thixotropic excipients are: Veegum ®(Magnesium-aluminium silicate) and Avicel RC® (about 89% microcrystalline cellulose and 11 % Caboxymethylcellulose Na).

6.3 Educing the particles size

A reduction in the $CaCO_3$ particle size resulted in a decrease in the antacid capacity of the compound.

6.4 Kneeping particles away from each other

This is the case in Veegum® and Avicel® for which insoluble particles smaller (about 1 μm) than the $CaCO_3$ particles, are placed between $CaCO_3$ particles in order to prevent aggregation.

**EXAMPLE 7: Product design**

The following schemes demonstrate examples of possible product designs.

7. 1 $CaCO_3$ in the springe

Having already clinical batches of Rotavirus in lyophilised vials, the antacid can be placed in the reconstituent liquid contained in the syringe.

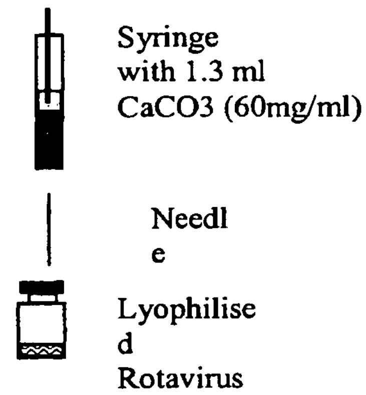


In this product presentation, sedimentation of $CaCO_3$ must be under control not only during the filling steps, but also during the complete shelf-live of the product (at least 2 years).

7. 2 $CaCO_3$ in the lyophilised vial

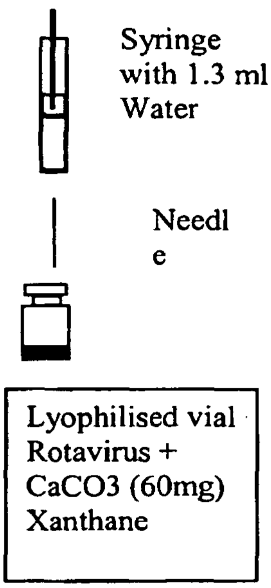


7.3. Lyophilisation in a blister

In this case Rotavirus, $CaCO_3$ and Xanthane gum are lyophilised together directly in the blister.

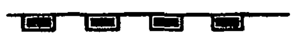

**EXAMPLE 8: Lyophilisation of different strain of Rotavirus**

Table 16

| Batch n° | Rotavirus strain | Formulation composition | Viral titer at t = zero after lyophilisation | Viral titer after lyopjhilisation and 1 week at 37° |
|---|---|---|---|---|
| 00F26/01 | G1<br>SB purif n°61 | Sucrose: 2%<br>Dextran: 4% | $10^{4.6}$ | $10^{4.7}$ |
| | PRO/0232 | Sorbitol: 3%<br>Am. Acids: 2% | | |
| 00F26/02 | G2 (DS-1) | Sucrose: 2%<br>Dextran: 4%<br>Sorbitol: 3%<br>Am. Acids: 2% | $10^{4.4}$ | $10^{4.4}$ |
| 00F26/03 | G3(P) | Sucrose: 2%<br>Dextran: 4%<br>Sorbitol: 3%<br>Am. Acids: 2% | $10^{4.6}$ | $10^{4.5}$ |
| 00F26/04 | G4 (VA-70) | Sucrose: 2%<br>Dextran: 4%<br>Sorbitol: 3%<br>Am. Acids: 2% | $10^{4.8}$ | $10^{4.8}$ |
| 00F26/05 | G9 (W161) | Sucrose: 2%<br>Dextran: 4%<br>Sorbitol: 3%<br>Am. Acids: 2% | $10^{4.6}$ | $10^{4.5}$ |

The strains DS-1, P and VA70 are described as Human rotavirus reference strains for serotype G2, G3 and G4 respectively at page 1361 of "Fields" Raven press 1990, second edition.

In this experiment different Rotavirus strains have been lyophilised.

For all, both the viral titer have been maintained during lyophilisation and accelarated stability (one week at 37°C) has been shown.

**EXAMPLE 9: Phase I safety study in adults of one oral administration of the Rotavirus vaccine.**

A Phase I study was carried out to assess the safety and reactogenicity of a single oral dose of $10^{6.0}$ ffu of the P43 vaccine in healthy adults aged 18 to 45 years.

The clinical trial was double blind and randomized. It was placebo-controlled and self-contained. The study was performed in one single centre in Belgium.

**9.1. Study Population**

A total of 33 subjects, 11 in the placebo group and 22 in the vaccine group, were enrolled and all completed the study. All volunteers were Caucasians. Their mean age at the time of vaccination was 35.3 years, with a range of 18 to 44 years. The trial began in January and ran for just over one month.

**9.2. Material**

<u>Vaccine</u>

Clinical lots of P43 vaccine were produced, purified, formulated and lyophilized according to Good Manufacturing Practices. The lots were released by Quality Control and Quality Assurance. Each vial of vaccine contained the following components:

Active ingredient:

| | |
|---|---|
| P43 strain | Min. $10^{5.8}$ ffu |

| Excipients, stabilizers: | |
|---|---|
| Sucrose | 9 mg |
| Dextran | 18 mg |
| Sorbitol | 13.5 mg |
| Amino acids | 9 mg |

Placebo

Vials of placebo were prepared and released. Each vial of placebo contained the following components:

| Excipients, stabilizers: | |
|---|---|
| Sucrose | 9 mg |
| Dextran | 18 mg |
| Sorbitol | 13.5 mg |
| Amino acids | 9 mg |

Diluent

Water for injection was used as diluent to reconstitute vaccine and placebo.

**9.3. Administration**

Approximately 10 to 15 minutes before administration of the vaccine or the placebo, subjects of both groups were given 10 ml of Mylanta® orally. Mylanta® is a registered antacid. The antacid increases the pH of the stomach and prevents inactivation of the rotavirus during its passage through the stomach.

To prepare the vaccine, two vials of lyophilized P43 containing $10^{5.8}$ ffu per vial were reconstituted with 1.5 ml of diluent water for injection. This achieved a calculated viral titer of $10^{6.1}$ ffu per dose. The reconstituted vaccine was administered promptly as a single oral dose.

To prepare the placebo, two vials of lyophilized placebo were reconstituted with 1.5 ml water for injection and administered orally as a single dose.

**9.4. Safety and Reactogenicity**

The following criteria of safety and reactogenicity applied:

Solicited general symptoms were fever, diarrhea, vomiting, nausea, abdominal pain and loss of appetite. They were recorded during eight days post administration.

Unsolicited symptoms were recorded during 30 days post administration.

Serious adverse events were recorded during the entire study period.

Diarrhea samples were to be collected during eight days post administration.

The results were:

No solicited symptoms, no unsolicited and no serious adverse events were reported during the respective observation periods.

No cases of diarrhea were reported.

### 9.5. Conclusions

SB Biologicals P43 vaccine was safe relative to the placebo when administered orally in a double-blind fashion as a single dose at the dose of $10^{6.1}$ ffu to healthy adult volunteers aged 18 to 44.

## EXAMPLE 10 - Efficacy of two doses of a human monovalent Rotavirus vaccine, containing RIX 4414 in preventing Gastro-enteritis due to G1 and non-G1 (G9) Rotavirus

### 10.1. Methods

A randomised, double-blind, placebo-controlled phase II trial was conducted in Latin America to evaluate the protective efficacy of a vaccine (RIX4414 human rotavirus strain) derived from the G1P[8] human strain 89-12 for infant immunisation. RIX4414 vaccine comprises as rotavirus component the attenuated G1P[8] human strain deposited as ECACC deposit 99081301 (WO 01/12797).

Vaccine *composition* (Table 17)

The HRV vaccine or placebo was prepared for administration by injecting the entire content of one pre-filled syringe containing the calcium carbonate buffer into the vial of the lyophilized product (vaccine or placebo). The vial was shaken to resuspend the vaccine/placebo. The entire volume of the resuspended product was withdrawn into the same syringe, the needle discarded and the resuspended product administered promptly as a single oral dose (approximately 1.0 ml).

**Table 17 - RIX4414 rotavirus vaccine composition**

| **Ingredients** | | **Quantity (per nominal dose: 1 ml)** |
|---|---|---|
| ***Active ingredient*** | | |
| RIX4414 | | $10^{5.8}$ ffu/dose |
| ***Excipients*** | | |
| Lyophilized vaccine in glass vial | Sucrose | 9 mg |
| | Dextran | 18 mg |
| | Sorbitol | 13.5 mg |
| | Amino acids | 9 mg |
| | Dulbecco's Modified Eagle Medium (DMEM) | 2.25 mg |
| **Liquid diluent ($CaCO_3$-based) in pre-filled syringe** | | |
| | Calcium carbonate | 60 mg |
| | Xanthan | 2.5 mg |
| | Water for Injections | q. s. ad 1 ml |

*Vaccine administration*

Healthy infants (493) received two doses of the RIX4414-rotavirus vaccine at a viral concentration of $10^{5.8}$ ffu per dose, or placebo (504) at age 2 and 4 months, concomitantly with DTPw-HBV and Hib vaccines. Three doses of OPV (oral polio virus vaccine) were given 2 weeks apart from study vaccine, i.e. were not to be administered during the period starting 2 weeks before each dose of study vaccine and ending 2 weeks after. Two other groups received 2 doses of the RIX4414-rotavirus vaccine at different viral concentrations: $10^{4.7}$ ffu and $10^{5.2}$ ffu. Diarrhoeal samples were tested for the presence of rotavirus (ELISA) and the serotypes determined in positive samples (RT-PCR). Diarrhoeal episodes reported from two weeks after the second dose were considered for the efficacy analysis. Severity was determined using a 20-point scale (Ruuska and Vesikari, 1990). The 20-point scoring system used to assess the severity of each diarrhoea episode in this study is shown below in Table 18. A score ≥ 11 defined severe disease.

**Table 18**

| Adverse Experience | Points |
|---|---|
| **Duration of looser than normal stools (days)** | |
| 1-4 | 1 |
| 5 | 2 |
| ≥ 6 | 3 |
| **Maximum number of looser than normal stools/24 hours** | |
| 1-3 | 1 |
| 4-5 | 2 |
| ≥ 6 | 3 |
| **Duration of vomiting (days)** | |
| 1 | 1 |
| 2 | 2 |
| ≥ 3 | 3 |
| **Maximum number of episodes of vomiting/24 hours** | |
| 1 | 1 |
| **2-4** | 2 |
| ≥ 5 | 3 |
| **Fever (measured rectally/axillary)*** | |
| 37.1-38.4°C/36.6-37.9°C | 1 |
| 38.5-38.9°C/38.0-38.4°C | 2 |
| ≥ 39°C/≥ 38.5°C | 3 |
| **Treatment** | |
| Rehydration | 1 |
| Hospitalization | 2 |
| **Dehydration** | |
| 1-5 % | 2 |
| ≥ 6% | 3 |
| * The highest temperature recorded during the episode was scored. | |

**10.2. Results**

An interim analysis of efficacy was performed on the above mentioned group and the isolated serotypes were mainly G1 and G9, almost evenly distributed. The overall attack rate in the placebo group varied from 4.8% for G1 to 3.6% for G9 during the 6 months observation period. Two doses of RIX4414 rotavirus vaccine at $10^{5.8}$ ffu protected against all types of diarrhoea caused by G1 with 83% efficacy [95% CI: 50.4-95.7] and 92.1 % efficacy [95% CI: 47.6-99.8] against severe gastro-enteritis. If the diarrhoea was caused by G9, the protection against all types of diarrhoea was 60.2 % [95% CI: 0.2-86.0] and 80.8% [95% CI: 33.0-96.4] against severe gastro-enteritis. For each of these efficacy endpoints (any and severe for G1 and G9), there was a statistically significant decrease in diarrhoea episodes in the HRV group as compared to the placebo group ($p < 0.05$, two-sided Fisher's exact test).

The results obtained in the other 2 vaccine groups (different rotavirus concentration) are consistent with those reported in the Example, and are presented in the final analysis (Example 11). Efficacy data for G2, G3 and G4 were also analysed. No conclusion from this study was drawn about G2, G3 and G4 cross-protection as too few cases were reported. However data of efficacy against G2, G3 and G4 are presented in the final analysis on a more important sample size (Example 11).

### 10.3. Conclusion.

These results are highly supportive of the efficacy of 2 doses of a monovalent HRV vaccine, RIX4414 rotavirus vaccine in protecting young infants against G1 strain and cross-protect against the G9 strain.

## EXAMPLE 11 - Efficacy of two doses of a human monovalent Rotavirus vaccine, containing RIX4414 strain, administered at three different virus concentrations in preventing Gastro-enteritis due to G1 and non-G1 (G2, G3, G4, G9) Rotavirus

### 11.1. Methods

A randomised, double-blind, placebo-controlled phase II trial was conducted in Latin America to evaluate the protective efficacy and efficacy against hospitalization of a vaccine derived from the G1P[8] human strain 89-12 for infant immunisation. Specifically the vaccine used was named RIX4414 rotavirus vaccine, and comprises as the rotavirus component the attenuated G1 human strain deposited as ECACC deposit 99081301.

Healthy infants received two doses of RIX4414 rotavirus vaccine at three different virus concentrations. The cohort for efficacy analysis consisted of 1846 subjects (468 subjects in the $10^{4.7}$ ffu HRV vaccine group, 460 subjects in the $10^{5.2}$ ffu HRV vaccine group, 464 subjects in the $10^{5.8}$ ffu HRV vaccine group and 454 subjects in the placebo group at age 2 and 4 months, concomitantly with DTPw-HBV and Hib vaccines. Three doses of OPV were given 2 weeks apart from study vaccine, i.e. were not to be administered during the period starting 2 weeks before each dose of study vaccine and ending 2 weeks after. Diarrhoeal samples were tested for the presence of rotavirus (ELISA) and the serotypes determined in positive samples (RT-PCR). Diarrhoeal episodes reported from two weeks after the second dose until subjects were one year of age were considered for the efficacy analysis. Severity was determined using a 20-point scale (Ruuska and Vesikari, 1990). A score ≥ 11 defined severe disease (see Example 10 for the description of the 20-point scoring system).

### 11.2. Results

Results which are the final analysis of the data mentioned in Example 10 are illustrated in the tables below. Infants in the vaccine groups had significantly fewer rotavirus gastroenteritis episodes than children in the placebo group ($p < 0.001$, two-sided Fisher's exact test) (Table 19). Depending on the dosage, protective efficacy against severe rotavirus gastroenteritis reached 85.6% (95%Cl: 63.0%-95.6%), and 70% (95%Cl, 45.7%-84.4%) against any rotavirus gastroenteritis (Table 20). For each of these efficacy endpoints, there was a statistically significant decrease in diarrhoea episodes in the HRV group as compared to the placebo group ($p < 0.001$, two-sided Fisher's exact test). Multiple rotavirus serotypes (G1, G2, G3, G4 and G9) were identified from gastroenteritis stools (ELISA and RT-PCR) allowing to also calculate vaccine efficacy against non-G1 serotypes. As can be seen from Table 21 in particular, for non-G1 serotypes (G2, G3, G4 and G9), and depending on the dosage, efficacy against severe rotavirus gastroenteritis reached 82.7% (95%Cl: 40.3%-96.8%), providing proof of concept that the monovalent G1-based G1P1A P[8] human rotavirus vaccine elicits cross-protection against heterotypic (i.e. non-G1 and non-P[8]) strains.

**Table 19: Features of rotavirus gastro-enteritis episodes reported during the study**

| | | **RIX4414 $10^{4.7}$ ffu** | **RIX4414 $10^{5.2}$ ffu** | **RIX4414 $10^{5.8}$ ffu** | **Placebo** |
|---|---|---|---|---|---|
| **Any rotavirus gastroenteritis** | | 21 | 22 | 15 | 51 |
| **no. of episodes (percent) with specific feature among all rotavirus gastroenteritis episodes reported** | | | | | |
| **Severity scores** | <7 | 4 (19.0) | 8 (36.4) | 2 (13.3) | 5 (9.8) |
| | 7-10 | 5 (23.8) | 4 (18.2) | 8 (53.3) | 12 (23.5) |
| | ≥ 11 | 12 (57.1) | 10 (45.5) | 5 (33.3) | 34 (66.7) |
| **Identified rotavirus serotypes** | | | | | |
| | wild G1 | 12 (57.1) | 6 (27.3) | 7 (46.7) | 30 (58.8) |
| | G2 | 0 | 0 | 1 (6.7) | 3 (5.9) |
| | G3 | 1 (4.8) | 0 | 0 | 2 (3.9) |
| | G4 | 0 | 0 | 1 (6.7) | 0 |

(continued)

| | RIX4414 $10^{4.7}$ ffu | RIX4414 $10^{5.2}$ ffu | RIX4414 $10^{5.8}$ ffu | Placebo |
|---|---|---|---|---|
| **Any rotavirus gastroenteritis** | 21 | 22 | 15 | 51 |
| **no. of episodes (percent) with specific feature among all rotavirus gastroenteritis episodes reported** | | | | |
| G9 | 8 (38.1) | 14 (63.6) | 7 (46.7) | 15 (29.4) |
| Canine | 0 | 0 | 0 | 1 (2.0) |
| Unknown | 0 | 2 (9.1) | 0 | 0 |

**Table 20: Protective efficacy of two doses of RIX4414 human rotavirus vaccine against rotavirus gastroenteritis**

| | | Any rotavirus gastroenteritis | | Severe rotavirus gastroenteritis | | Hospitalization for rotavirus gastroenteritis | |
|---|---|---|---|---|---|---|---|
| | **N** | **n (%)** | **Efficacy (95% CI)** | **n (%)** | **Efficacy (95% CI)** | **n (%)** | **Efficacy (95% CI)** |
| Pooled vaccine groups | 1392 | 58 (4.2)* | 61.4 (42.3;4.1) | 27 (1.9)* | 74.1 (55.8;85.0) | 9 (0.6) * | 79.0 (48.0;92.0) |
| RIX4414 $10^{5.8}$ ffu | 464 | 15 (3.2)* | 70.0 (45.7;84.4) | 5 (1.1)* | 85.6 (63.0;95.6) | 3 (0.6)‡ | 79.0 (24.9;96.1) |
| RIX4414 $10^{5.2}$ ffu | 460 | 22 (4.8)* | 55.7 (25.3;74.5) | 10 (2.2)* | 71.0 (39.9;87.2) | 1 (0.2)* | 93.0 (53.7;99.8) |
| RIX4414 $10^{4.7}$ ffu | 468 | 21 (4.5)* | 58.4 (29.4;76.3) | 12 (2.6)* | 65.8 (32.2;83.9) | 5 (1.1)t | 65.4 (-1.8;90.2) |
| Placebo | 454 | 49 (10.8) | - | 34 (7.5) | | 14 (3.1) | - |

*p<0.001 for each comparison between the vaccine and placebo groups by two-sided Fisher's exact test (significant level of $\alpha$=0.05)
tp=0.037 for the comparison between the vaccine and placebo groups by two-sided Fisher's exact test (significant level of $\alpha$=0.05)
‡p=0.007 for the comparison between the vaccine and placebo groups by two-sided Fisher's exact test (significant level of $\alpha$=0.05)
N = number of subjects
n/% = number/percentage of subjects reporting at least one specified rotavirus gastroenteritis episode
Exact 95% confidence intervals are shown

**Table 21: Protective efficacy of two doses of RIX4414 human rotavirus vaccine against serotype specific severe rotavirus gastroenteritis**

| | | Severe rotavirus gastroenteritis | | |
|---|---|---|---|---|
| | **N** | **n(%)** | **Efficacy (95% CI)** | **p-value*** |
| **G1 wild type rotavirus** | | | | |
| Pooled vaccine groups | 1392 | 13 (0.9) | 73.5 (41.2;88.3) | <0.001 |
| RIX4414 $10^{5.8}$ ffu | 464 | 2 (0.4) | 87.8 (48.0;98.6) | <0.001 |
| RIX4414 $10^{5.2}$ ffu | 460 | 4 (0.9) | 75.3 (23.5;94.0) | 0.006 |
| RIX4414 $10^{4.7}$ ffu | 468 | 7 (1.5) | 57.6 (-9.0;85.2) | 0.057 |

(continued)

| | Severe rotavirus gastroenteritis | | | |
|---|---|---|---|---|
| | **N** | **n(%)** | **Efficacy (95% CI)** | **p-value*** |
| **G1 wild type rotavirus** | | | | |
| Placebo | 454 | 16 (3.5) | - | - |
| **Non-G1 rotavirus (mainly G9 with G2, G3 and G4 types)** | | | | |
| Pooled vaccine groups | 1392 | 14 (1.0) | 73.1 (42.1;87.7) | <0.001 |
| RIX4414 $10^{5.8}$ ffu | 464 | 3 (0.6) | 82.7 (40.3;96.8) | 0.001 |
| RIX4414 $10^{5.2}$ ffu | 460 | 6 (1.3) | 65.2 (7.4;88.8) | 0.020 |
| RIX4414 $10^{4.7}$ ffu | 468 | 5 (1.1) | 71.5 (19.4;91.8) | 0.009 |
| Placebo | 454 | 17 (3.7) | - | - |

*Two-sided Fisher's exact test (significant level of $\alpha$=0.05) used for each comparison between the vaccine and placebo groups.
N = number of subjects
n/% = number/percentage of subjects reporting at least one specified severe rotavirus gastroenteritis episode
Exact 95% confidence intervals are shown

### 11.3. Conclusion

These results are highly supportive of the efficacy of 2 doses of a monovalent HRV vaccine containing RIX4414, in protecting young infants against any and severe rotavirus gastroenteritis caused by the G1 strain and broad cross-protection against other RV G types, namely G2, G3, G4 and G9.

### EXAMPLE 12 -Two doses of the Human Attenuated Rotavirus vaccine RIX4414 vaccine show heterotypic protection in Latin America and Europe

The Efficacy of a 2 dose oral, live attenuated G1P[8] human rotavirus (RV) vaccine containing RIX 4414 strain was analysed in a Phase II/III clinical trials in Finnish and Latin American infants. RIX 4414 rotavirus vaccine comprises as the rotavirus component the attenuated G1 human strain deposited as ECACC deposit 99081301.

### 12.1. Methods

Part of the results of Example 12 is already presented in Examples 10 and 11. Data were pooled from Phase II studies, one in Finland and one in Latin America (Brazil, Mexico and Venezuela) (Examples 10 and 11) and from one Phase III study in 11 Latin American countries (Example 13) using the same methodology and efficacy criteria. In total, 20081 healthy infants (cohort for efficacy) vaccinated with 2 doses of RIX 4414 vaccine or placebo at 2 and 4 months of age were followed until one year of age for severe gastroenteritis (GE) with a score on the Vesikari (Ruuska T et al. Scand. J. Infect. Dis. 1990, 22, 259-267) severity scale ≥ 11. GE samples were tested for rotavirus (by ELISA) and typed by RT-PCR.

A meta analysis was conducted on the three mentioned studies. Pooled efficacy for severe RV GE (defined as Vesikari severity score ≥ 11) was calculated from 2 weeks post-dose 2 to 1 year of age (adjustment for study effect using the Mantel-Haenszel approximation).

### 12.2. Results

In the cohort for efficacy 5 severe rotavirus GE episodes of G2P[4] type with a Vesikari score ≥ 11 were detected in the vaccine group and 1.3 episodes in the placebo group. Vaccine efficacy against the G2P[4] type was 67.2% (95% CI: 14.8; 87.1), which shows that in addition to protecting against homotypic strains (G1 P[8], G3P[8] and G4P[8]), RIX4414 vaccine protects against severe rotavirus GE caused by the heterotypic non-P[8] non-G1 G2P[4] strain.

Type-specific efficacy across the different studies is given below (Table 22).

**Table 22**

| Strain | Number of severe RV cases N Vaccinees (N = 10646) | N Placebo (N = 9435) | % Vaccine Efficacy* (VE) (95% CI) |
|---|---|---|---|
| G1 | 17 | 52 | 83.7 (70.0; 91.2) |
| G2P[4] | 5 | 13 | 67.2 (14.8; 87.1) |
| G3 | 2 | 8 | 82.7 (5.9; 96.8) |
| G9 | 15 | 34 | 79.5 (59.2; 89.7) |
| *VE adjusted for study effect using Mantel-Haenszel approximation | | | |

Only 3 G4 cases occurred in all 3 trials, 1 in vaccine and 2 in placebo recipients.

**12.3. Conclusion**

This analysis shows that, in addition to giving a high level of protection against homologous G1 rotavirus strains (which have two outer capsid proteins (VP4 and VP7) and one inner capsid protein (VP6) antigenically similar to the vaccine), RIX 4414 vaccine is also highly protective against other strains which have either a different G type (eg G3, G9), a different P type (eg P[4]), or both different G type and P type, as illustrated by the efficacy against G2P[4].

**EXAMPLE 13 - Meta analysis showing that two doses of the Human Attenuated Rotavirus vaccine RIX 4414 show heterotypic protection**

As more data became available from Singapore and from a European study (Example 15), an additional meta analysis was carried out to include these studies in addition to studies mentioned in Example 12.

**13.1. Methods**

Three phase II (Finland and Latin America and Singapore) and two phase III studies (Latin America and Europe) were included in the meta-analysis. Two oral doses were administered according to 0,1 to 2 month schedule to healthy infants who were 6-14 weeks of age at Dose 1. In all studies, severe RVGE was defined as a score ≥11 on the 20-point Vesikari scale. Diarrhoeal samples were analyzed for the presence of RV by ELISA and typed by RT-PCR based method. Efficacy against any RV GE was evaluated in the three phase II studies and the phase III Europe study only as in the phase III Latin America study, only severe RV GE were recorded.

VE and its 95%CI was estimated as 1-rate of RVGE relative to placebo using exact Poisson rate ratio stratified by study (Proc StatXact4 for SAS Users, 1999, cytel software corporation, exact Confidence Interval for common relative risk, p298)

**13.2. Results**

In a total of 8221 infants vaccinated with two doses of RIX4414 or placebo, 4 episodes of any G2P[4] RVGE were detected in the RIX4414 (N=5783) and 9 episodes in the placebo (N=2438) group, indicating a VE of 81.0% (95% CI:31.6;95.8) against RVGE of any severity due to G2P[4] strain.

In a total of 26088 healthy infants vaccinated with two doses of RIX4414 or placebo, 6 episodes of severe RVGE due to G2P[4] type were detected in the RIX4414 (N=14792) and 15 episodes in the placebo (N=11296) group, indicating a VE of 71.4% (95% CI:20.1;91.1) against severe RVGE due to G2P[4] strain. Results are reported in Table 23.

**Table 23 - Number of subjects reporting any or severe RV GE episodes caused by G2P[4] RV type and percentage of vaccine efficacy during the first efficacy period - (meta analysis), cohort for efficacy**

**Table 23**

| Groups | | Any RV GE* (Latin America excluded) | | | | Severe RV GE (score ≥11 on Vesikari scale) (5 studies) | | | | |
|---|---|---|---|---|---|---|---|---|---|---|
| | N | n | % | Vaccine efficacy | | N | n | % | Vaccine efficacy | |
| ***RV Strain*** | | | | % VE | 95% CI | | | | % VE | 95% CI |
| **G2P[4]** | | | | | | | | | | |
| HRV vaccine | 5783 | 4 | 0.07 | **81.0** | 31.6-95.8 | 14792 | 6 | 0.04 | **71.4** | 20.1-91.1 |
| Placebo | 2438 | 9 | 0.37 | | | 11296 | 15 | 0.13 | | |

N = number of subjects included in each group;
n/% = number/percentage of subjects reporting at least one specified RV G2P[4] GE episode in each group;
% VE = observed vaccine efficacy,
95%CI = 95%Confidence Intervals
* Two out of 13 G2 were not P typed

**13.3. Conclusion:**

This meta analysis on vaccine efficacy against G2P[4] RV type shows a vaccine efficacy of 81.0% (95% CI: 31.6%; 95.8%) against any RV GE due to G2P[4] type and a vaccine efficacy of 71.4% (95% CI: 20.1 %; 91.1 %) against severe RV GE due to G2P[4] type.

**EXAMPLE 14 - Efficacy of Human Attenuated Rotavirus vaccine Rotarix™ in a multi-country phase III trial**

**14.1. Methods**

20169 healthy infants from 11 Latin American countries were to receive two oral doses of HRV vaccine (10159) or placebo (10010) at approximately 2 and 4 months of age. Stool specimens were tested for rotavirus (RV) by ELISA and typed by RT-PCR using suitable primers and type-specific probes. The clinical case definition for capture of severe gastroenteritis episode was an episode of diarrhea (passage of three or more looser than normal or watery stools within 24 hours) with or without vomiting that required overnight hospitalization and/or rehydration therapy equivalent to WHO plan B (oral rehydration therapy) or WHO plan C (intravenous rehydration therapy) in a medical facility such as hospital, clinic or supervised rural health care center (hftr)://www.who.int/child-adolescent-health/New_Publications/CHILDHEALTH/textrev4.htm). Disease severity was graded using the 20-point Vesikari scale; severe RVGE was defined as a score ≥11. Vesikari's score was modified: Since the dehydration was not recorded in the eCRF, the following rule was applied: a subject that had a severe GE episode was considered as being dehydrated between 1 to 5% if this subject received oral re-hydration. A subject was considered as being dehydrated ≥ 6 % if the subject was hospitalized and/or received intravenous (IV) re-hydration.

**14.2. Vaccine efficacy**

*Vaccine efficacy against severe rotavirus gastroenteritis* (Table 24)

The cohort for efficacy consisted of 9009 subjects vaccinated with HRV vaccine and 8858 subjects receiving a placebo recipient. There were 12 children with severe rotavirus gastroenteritis according to the clinical definition in the vaccine and 77 in the placebo group (2.0 *vs.* 13.3 children with ≥1 episode per 1,000 child-years, respectively; p<0.001, two-sided Fisher's exact test), resulting in a vaccine efficacy of 84.7% against severe rotavirus gastroenteritis from 15 days post-dose 2 until one year of age (shown in Table 24). Similar results were obtained with the total vaccinated cohort (vaccine efficacy of 81.1%; 95 % C.I. 68.5-89.3; p< 0.001, two-sided Fisher's exact test) from dose 1 until one year of age. Hospitalization for at least one night was required in 9 children in the vaccine and 59 in the placebo group (1.5 vs. 10.2 hospitalizations per 1,000 child-years, respectively), for a vaccine efficacy against hospitalization for severe rotavirus gastroenteritis of 85 % (p<0.001, two-sided Fisher's exact test) (Table 24).

**Table 24 - Vaccine efficacy against rotavirus severe gastroenteritis, specific rotavirus G types severe gastroenteritis and all-cause severe gastroenteritis, during the period from two weeks after dose 2 until one year of age**

| | Vaccine group (N = 9,009) | | Placebo group (N = 8,858) | | RR | Vaccine efficacy (95% CI) and p_values |
|---|---|---|---|---|---|---|
| | n | 1,000 infants-year ratio | n | 1,000 infants-year ratio | | |
| **Severe rotavirus gastroenteritis according to the clinical case definition* Rotavirus gastroenteritis** | | | | | | |
| Severe | 12 | 2.0 | 77 | 13.3 | 0.153 | 84.7 (71.7; 92.4) <0.001 |
| Hospitalization | 9 | 1.5 | 59 | 10.2 | 0.150 | 85.0 (69.6; 93.5) <0.001 |
| **All-cause gastroenteritis** | | | | | | |
| Severe | 183 | 30.9 | 300 | 51.7 | 0.600 | 40.0 (27.7; 50.4) <0.001 |
| Hospitalization | 145 | 24.5 | 246 | 42.4 | 0.580 | 42.0 (28.6; 53.1) <0.001 |
| **Type specific gastroenteritis** | | | | | | |
| G1P[8][#] | 3[a] | 0.5 | 36[b] | 6.2 | 0.082 | 91.8 (74.1; 98.4) <0.001 |
| G3P[8], G4P[8], G9P[8] | 4[c] | 0.66 | 31[d] | 5.3 | 0.126 | 87.3 (64.1 ;96.7) <0.001 |
| G2P[4] | 6 | 1.0 | 10[e] | 1.7 | 0.590 | 41.0 (-79.2; 82.4) 0.328 |
| **Severe rotavirus gastroenteritis with a score ≥ 11 on the Vesikari scale** | | | | | | |
| | **Vaccine group (N = 9,009)** | | **Placebo group (N = 8,858)** | | **RR** | **Vaccine efficacy (95% CI) and p_values** |
| | **n** | **1,000 infants-year ratio** | **n** | **1,000 infants-year ratio** | | |
| **Type specific gastroenteritis** | | | | | | |
| G1P[8][#] | 3 | 0.5 | 32 | 5.5 | 0.092 | 90.8 (70.5;98.2) <0.001 |
| G3P[8], G4P[8], G9P[8] | 4 | 0.66 | 30 | 5.2 | 0.130 | 86.9 (62.8;96.6) <0.001 |

(continued)

| | Vaccine group (N = 9,009) | | Placebo group (N = 8,858) | | RR | Vaccine efficacy (95% CI) and p_values |
|---|---|---|---|---|---|---|
| | n | 1,000 infants-year ratio | n | 1,000 infants-year ratio | | |
| G2P[4] | 5 | 0.8 | 9 | 1.5 | 0.546 | 45.4 (-81.5;85.6) 0.298 |

Legend to Table 24:
Participants with episodes with more than one isolated G type were counted in each of the detected rotavirus type category.
n = number of infants reporting at least one specified episode RR = Relative Risk = ratio of the incidence rate of subjects reporting at least one episode in the vaccine group over the incidence rate of subjects reporting at least one episode in the placebo group.
CI = confidence interval
The 1000-infant year ratio is the number of infants presenting with >= 1 specified episode per infant-year
*Case definition according to the study protocol: an episode of diarrhea (passage of three or more looser than normal or watery stools within a day) with or without vomiting that required overnight hospitalization and/or rehydration therapy equivalent to WHO plan B (oral rehydration therapy) or WHO plan C (intravenous rehydration therapy) in a medical facility such as hospital, clinic or supervised rural health care center.
# All G1 types isolated were wild-type rotavirus; G1P[8] and G9P[8] were isolated from one infant
[a] G1P[8] type alone was isolated from 2 infants; G1P[8] and G9P[8] were isolated from one infant
[b] ; G1P[8] type alone was isolated from 34 infants; G1P[8] and G9P[8] were isolated from one infant; G1, G2, G9 types were isolated from one infant
[c] G3P[8] type alone was isolated from one infant, G4P[8] type alone from 1 infant; and G9P[8] alone from one infant; G1P[8] and G9P[8] were isolated from one infant
[d] G3P[8] type alone was isolated from 8 infants, G4P[8] type alone from 2 infants; and G9P[8] alone from 19 infants; G1P[8] and G9P[8] were isolated from 1 infant; and G1P[8] and G2P[4 and G9P[8] from 1 infant
[e] G2P[4] alone was isolated from 9 infants and G1P[8], G2P[4] and G9P[8] were isolated from 1 infant
p_values =two-sided Fisher's exact test (significant level of $\alpha$=0.05)

*Vaccine efficacy, according to Vesikari score*

Eleven of 12 children with severe rotavirus episodes in the vaccine group and 71 of 77 in the placebo group had Vesikari score ≥11, resulting in a vaccine efficacy of 84.7%t (P<0.001, two-sided Fisher's exact test). For increasing disease severity with scores between 11 and 20, vaccine efficacy was increasingly higher, reaching 100% against more severe rotavirus gastroenteritis. A total of 16 severe rotavirus gastroenteritis episodes with Vesikari score ≥11 were reported from dose 1 until dose 2, six in the vaccine group and 10 in the placebo group.

*Vaccine efficacy according to Vesikari* score *per rotavirus type*

Type specific vaccine efficacy against wild-type strains is shown in Table 24. Vaccine efficacy against severe rotavirus episodes with Vesikari score ≥11 caused by G1P[8] type strains, homologous to the vaccine strain, was 91.8% (P<0.001, two-sided Fisher's exact test). Vaccine efficacy against strains sharing the P[8] antigen (G3P[8], G4P[8] and G9P[8]) was 86.9% (P<0.001, two-sided Fisher's exact test). G2P[4] rotavirus type, which is not sharing either the G nor the P antigen with the vaccine strain was detected in five episodes in the vaccine and nine in the placebo group, resulting in an efficacy of 45 percent (P=0.298, two-sided Fisher's exact test). Because of the small number of G2 episodes observed in this study, a meta-analysis of 5 studies (Example 13) was performed and the trend observed in this study has become a significant value when the results of the 5 studies were pooled. (Example 13)

*Vaccine efficacy on the burden of diarrhea illness*

Children with gastroenteritis of any cause requiring hospitalization and/or rehydration according to WHO plan B/C had an incidence rate of 30.9/1,000 child-years in the vaccine compared to 51.7 in the placebo group, for an overall 40 % (P<0.001, two-sided Fisher's exact test) reduction in severe diarrhea episodes of all cause among vaccine recipients. Likewise, hospitalization for diarrhea of any etiology was significantly reduced by 42 % (P<0.001, two-sided Fisher's

exact test) (Table 24, all causes GE)).

### 14.3. Summary of results

Vaccine efficacy against severe rotavirus gastroenteritis (RV GE) and against rotavirus associated-hospitalization was 85% (P<0.001, two-sided Fisher's exact test), reaching 100% in a population having RV GE with a Vesikari's score >=19. Efficacy against G1P[8] and strains sharing only the P[8] epitope with HRV was 92% (95%C.I. 74,98) and 87% (95%C.I. 64,97) respectively (P<0.001, two-sided Fisher's exact test). Hospitalization for diarrhea of all cause was reduced by 42% (95%C.I. 29,53; P<0.001, two-sided Fisher's exact test).

## Example 15 - Efficacy of Human Attenuated Rotavirus vaccine Rotarix™ in six European countries

### 15.1. Methods

3,994 children in six European countries were randomized to receive $10^{6.5}$ $CCID_{50}$ HRV (human rotavirus) vaccine Rotarix™ (see composition) or placebo when co-administered with routine childhood vaccinations. The first efficacy follow-up period started from two weeks after Dose 2 and ended June - July 2005. A total of 3874 subjects were part of the 1st year efficacy cohort.

<u>*Vaccine composition:*</u> (Table 25)

| Vaccine | Formulation |
|---|---|
| GSK Biologicals' HRV vaccine | RIX4414 HRV strain derived from the 89-12 HRV vaccine strain<br>106.5 median Cell Culture Infective Dose (CCID50)<br>Dulbecco's Modified Eagle Medium (DMEM) 3.7 mg<br>Sucrose 9 mg<br>Dextran 18 mg<br>Sorbitol 13.5 mg<br>Amino acids 9 mg |
| GSK Biologicals' diluent | Calcium carbonate 80 mg<br>Xanthane 3.25 mg<br>Water for injection q.s. ad 1.3 ml |

### 15.2. Vaccine efficacy

The HRV vaccine was highly effective in protecting against RV GE during the first efficacy period. Vaccine efficacy was **87.1%** (95% CI: 79.6%; 92.1%) against any episodes of RV GE and **95.8%** (95% CI: 89.6%; 98.7%) against severe RV GE episodes. For increasing disease severity (Vesikari scores between 11 and 20), vaccine efficacy was increasingly higher, reaching 100% in a population having RV GE with a Vesikari score ≥ 17 points. Vaccine efficacy against hospitalization for RV GE was 100% (95% CI: 81.8%; 100%) and against RV GE episodes requiring medical attention was 91.8% (95% CI: 84.0%; 96.3%) (Tables 26 and 27).

**Table 26 - Percentage of subjects reporting any and severe RV GE, percentage of subjects hospitalized due to RV GE episodes, and vaccine efficacy during first efficacy period - cohort for efficacy**

| Groups | | Any RV GE | | | | | Severe RV GE (score ≥11 on Vesikari scale) | | | | |
|---|---|---|---|---|---|---|---|---|---|---|---|
| | | Subjects | | | Vaccine efficacy | | Subjects | | | Vaccine efficacy | |
| | N | n | % | P-value | % VE | 95%CI | n | % | P-value | % VE | 95%CI |
| $10^{6.5}$ $CCID_{50}$ | 2572 | 24 | 0.9 | <0.001 | **87.1** | 79.6-92.1 | 5 | 0.2 | <0.001 | **95.8** | 89.6-98.7 |
| Placebo | 1302 | 94 | 7.2 | - | - | - | 60 | 4.6 | - | - | - |

(continued)

| | N | Hospitalized RV GE Subjects n | % | P-value | Vaccine efficacy % VE | 95%CI |
|---|---|---|---|---|---|---|
| **Study** | | | | | | |
| $10^{6.5}$ $CCID_{50}$ | 2572 | 0 | 0.0 | <0.001 | **100** | 81.8-100 |
| Placebo | 1302 | 12 | 0.9 | - | - | - |

N = number of subjects included in each group
N% = number/percentage of subjects reporting at least one specified RV GE episode in each group
P-value = two-sided Fisher's exact test (significant level of $\alpha$=0.05)
% VE = observed vaccine efficacy
95%CI = 95%Confidence Intervals
a score ≥11 on the 20-point Vesikari scale was defined as severe

**Table 27 - Percentage of subjects reporting RV GE episodes requiring medical attention and vaccine efficacy during the first efficacy period - cohort for efficacy**

| Groups | N | RV GE requiring medical attention n | % | Vaccine efficacy % VE | 95% CI | *P-value* |
|---|---|---|---|---|---|---|
| $10^{6.5}$ $CCID_{50}$ | 2572 | 10 | 0.4 | 91.8 | 84.0-96.3 | *<0.001* |
| Placebo | 1302 | 62 | 4.8 | - | - | - |

N = number of subjects included in each group;
N/% = number/percentage of subjects reporting at least one RV GE episode requiring medical attention in each group;
P-value=two-sided Fisher's exact test (significant level of $\alpha$=0.05);
% VE = observed vaccine efficacy, 95%CI = 95% Confidence Intervals

The HRV vaccine was highly protective against any and severe RV GE caused by G1P[8], G3P[8], G4P[8] and G9P[8] strains (Table 28).

Protection against G2P[4] RV type that does not share any of the outer capsid antigens of the HRV vaccine was lower in this study however the results of a meta analysis taking into account phase II and III efficacy studies showed a significant protective efficacy against any and severe GE due to G2P[4] (see Example 13).

**Table 28 - Percentage of subjects reporting any or severe RV GE episodes** and **vaccine efficacy by serotype during the first efficacy period - cohort for efficacy**

| Groups | | Any RV GE | | | | | Severe RV GE (score ≥11 on Vesikari scale) | | | | |
|---|---|---|---|---|---|---|---|---|---|---|---|
| Viral Conc | N | n | % | Vaccine efficacy | | *P-value* | n | % | Vaccine efficacy | | *P-value* |
| ***RV Strain*** | | | | % VE | 95% CI | | | | % VE | 95% CI | |
| ***G1 P[8] Wild type*** | | | | | | | | | | | |
| $10^{6.5}$ $CCID_{50}$ | 2572 | 4 | 0.2 | **95.6** | 87.9-98.8 | <0.001 | 2 | 0.1 | **96.4** | 85.7-99.6 | <0.001 |
| Placebo | 1302 | 46t | 3.5 | | | | 28† | 2.2 | | | |
| ***G2 P[4]*** | | | | | | | | | | | |
| $10^{6.5}$ $CCID_{50}$ | 2572 | 3 | 0.1 | **62.0** | -124.4-94.4 | 0.234 | 1 | 0.0 | **74.7** | -386.2-99.6 | 0.263 |
| Placebo | 1302 | 4 | 0.3 | | | | 2 | 0.2 | | | |
| ***G3 P[8]*** | | | | | | | | | | | |
| $10^{6.5}$ $CCID_{50}$ | 2572 | 1 | 0.0 | **89.9** | 9.5-99.8 | 0.018 | 0 | 0.0 | **100** | 44.8-100 | 0.004 |
| Placebo | 1302 | 5 | 0.4 | | | | 5 | 0.4 | | | |
| ***G4 P[8]*** | | | | | | | | | | | |
| $10^{6.5}$ $CCID_{50}$ | 2572 | 3 | 0.1 | **88.3** | 57.5-97.9 | <0.001 | 0 | 0.0 | **100** | 64.9-100 | <0.001 |
| Placebo | 1302 | 13† | 1.0 | | | | 7† | 0.5 | | | |
| ***G9 P[8]*** | | | | | | | | | | | |
| $10^{6.5}$ $CCID_{50}$ | 2572 | 13 | 0.5 | **75.6** | 51.1-88.5 | <0.001 | 2 | 0.1 | **94.7** | 77.9-99.4 | <0.001 |
| Placebo | 1302 | 27 | 2.1 | | | | 19 | 1.5 | | | |
| ***Pooled non G1 P[8] (G2, G3, G4, G9)*** | | | | | | | | | | | |
| $10^{6.5}$ $CCID_{50}$ | 2572 | 20 | 0.8 | **79.3** | 64.6-88.4 | <0.001 | 3 | 0.1 | **95.4** | 85.3-99.1 | <0.001 |
| Placebo | 1302 | 49 | 3.8 | | | | 33 | 2.5 | | | |

N = number of subjects included in each group;
n/% = number/percentage of subjects reporting at least one RV GE episode in each group;
P-value = two-sided Fisher's exact test (significant level of $\alpha$=0.05);
% VE = observed vaccine efficacy,
95%CI = 95% Confidence Intervals
a score ≥11 on the 20-point Vesikari scale was defined as severe
t One subject from the Placebo group counted in G1 and G4 categories since both serotypes were isolated

### 15.3. Summary of results

Two oral doses of HRV Rotarix™ vaccine, co-administered with childhood vaccinations, were highly effective during the first efficacy period compared to the placebo in protecting infants against any RV GE caused by G1P[8] wild-type RV and by non-G1P[8] RV types, vaccine efficacy was 95.6% (95% CI: 87.9%; 98.8%) and 79.3% (95% CI: 64.6%; 88.4%) respectively. The efficacy against severe RV GE caused by G1 P[8] wild-type RVs and by non- G1P[8] RV types is 96.4% (95% CI: 85.7%; 99.6%) and 95.4% (95% CI: 85.3%; 99.1%) respectively.

These results are very supportive towards the conclusion that the HRV vaccine provides broad coverage against circulating RV strains (see Table 28: G1 P[8], G2 P[4], G3P[8], G4P[8], G9P[8]). A meta analysis on vaccine efficacy against G2 P[4] specifically was performed, please refer to Example 13.

### Overall Conclusions

The RIX4414 rotavirus vaccine proved to be highly protective against rotavirus gastroenteritis episodes measured by a clinical definition for case capture focusing on hospitalization and re-hydration, as well as by the validated Vesikari scale which includes quantifiable morbidity outcomes related with diarrhea, vomiting, fever, dehydration and hospitalization. Two oral doses of HRV vaccine were highly efficacious in protecting infants against any and severe RVGE and hospitalization due to multiple circulating rotavirus strains.

A high level of protection was demonstrated against homologous G1P[8] rotaviruses, which have two outer capsid proteins (VP4 and VP7) and one inner capsid protein (VP6) antigenically similar to the HRV vaccine. It also protected well against strains sharing only the genotype P[8] (VP4 antigen) and the VP6 antigen . Protection against rotavirus strains not sharing any of the outer capsid antigens of the HRV vaccine was also demonstrated in a meta analysis including the results of three phase II studies from Finland, Singapore and Latin America (all using identical methodology and efficacy criteria) and of 2 phase III studies from Latin America and Europe, and which are reported in Example 13, vaccine efficacy against G2P[4] type of any severity was 81 % (95 % C.I. 31.6-95.8) and vaccine efficacy against severe GE due to G2P[4] type was 71.4 % (95 percent C.I. 20.1-91.1) indicating that the vaccine can also protect against strains which do not share identical G or P proteins with the vaccine strain.

## Claims

**1.** Use of an attenuated rotavirus strain from a G1 P[8] type in the manufacture of a composition for conferring protection

against rotavirus infection caused by a rotavirus strain of a G2P[4] type.

2. Use according to claim 1, wherein the composition comprises a rotavirus having a VP4 gene comprising, in the nucleotide sequence, at least one of the following: an adenine base (A) at position 788, an adenine base (A) at position 802 and a thymine base (T) at position 501 from the start codon.

3. Use according to claim 2, in which the VP4 gene comprises a nucleotide sequence comprising an adenine base (A) at positions 788 and 802 and a thymine base (T) at position 501 from the start codon.

4. Use according to any of claims 1- 3, in which the composition comprises a rotavirus having a VP7 gene comprising, in the nucleotide sequence, at least one of the following: a thymine (T) at position 605, an adenine (A) at position 897 and a guanine (G) at position 897 from the start codon.

5. Use according to claim 4, in which the VP7 gene comprises a nucleotide sequence comprising a thymine (T) at position 605 and an adenine (A) or a guanine (G) at position 897 from the start codon.

6. Use according to any of claims 1 to 5, wherein the composition comprises a rotavirus having a VP4 gene comprising, in the nucleotide sequence, an adenine (A) at positions 788 and 802 and a thymine (T) at position 501 from the start codon; and wherein the VP7 gene comprises, in the nucleotide sequence, a thymine (T) at position 605 and an adenine (A) at position 897 from the start codon.

7. Use according to any of claims 1 to 6, wherein the composition further confers protection against at least one of the non-G1 serotypes selected from the group consisting of: G3, G4 and G9.

8. Use according to any preceding claim wherein the composition comprising an attenuated rotavirus strain of a G1P[8] type provides protection against severe rotavirus-induced gastroenteritis having a score equal or higher than 11 on the Vesikari scale caused by infection of a rotavirus strain of a G2P[4] type.

9. Use according to any of claims 1 to 8, wherein the composition comprising an attenuated rotavirus strain of a G1 P[8] type is at least 40% protective, in a population of vaccinated individuals, against diarrhoea caused by a rotavirus strain which is of a G2P[4] type.

10. Use according to claim 9, wherein the composition is at least 50% protective.

11. Use according to claim 9, wherein the composition is between 40% and 80% protective.

12. Use according to claim 11, wherein the composition is between 50% and 70% protective.

13. Use according to claim 8 wherein the composition is between 40% and 75% protective in a population of vaccinated individuals against severe gastro-enteritis having a score equal or higher than 11 on the Vesikari scale caused by infection of rotaviruses with a G2P[4] serotype.

14. Use according to any of claims 1 to 13, wherein the rotavirus strain in said composition is ECACC deposit 99081301, or is obtainable or derivable from ECACC deposit 99081301.

15. Use according to any of claims 1 to 14, wherein the composition is administered in a 2-dose regime.

16. Use according to any of claims 1 to 15, wherein the attenuated rotavirus strain is formulated with a suitable pharmaceutical carrier or with an antacid buffer or both.

17. Use according to any one of claims 1 to 16, wherein the composition is for conferring protection in a human subject.

**Patentansprüche**

1. Verwendung eines attenuierten Rotavirusstamms vom Typ G1P[8] bei der Herstellung einer Zusammensetzung zur Verleihung von Schutz gegen Rotavirusinfektion, welche von einem Rotavirusstamm vom Typ G2P[4] verursacht wird.

2. Verwendung gemäß Anspruch 1, wobei die Zusammensetzung ein Rotavirus mit einem VP4-Gen umfasst, das, in der Nukleotidsequenz, mindestens eines der folgenden umfasst: eine Adeninbase (A) an Position 788, eine Adeninbase (A) an Position 802 und eine Thyminbase (T) an Position 501 vom Startkodon.

3. Verwendung gemäß Anspruch 2, bei welcher das VP4-Gen eine Nukleotidsequenz umfasst, die eine Adeninbase (A) an Position 788 und 802 und eine Thyminbase (T) an Position 501 vom Startkodon umfasst.

4. Verwendung gemäß einem der Ansprüche 1-3, bei welcher die Zusammensetzung ein Rotavirus mit einem VP7-Gen umfasst, das, in der Nukleotidsequenz, mindestens eines der folgenden umfasst: ein Thymin (T) an Position 605, ein Adenin (A) an Position 897 und ein Guanin (G) an Position 897 vom Startkodon.

5. Verwendung gemäß Anspruch 4, bei welcher das VP7-Gen eine Nukleotidsequenz umfasst, die ein Thymin (T) an Position 605 und ein Adenin (A) oder Guanin (G) an Position 897 vom Startkodon umfasst.

6. Verwendung gemäß einem der Ansprüche 1-5, wobei die Zusammensetzung ein Rotavirus mit einem VP4-Gen umfasst, das, in der Nukleotidsequenz, ein Adenin (A) an den Positionen 788 und 802 und ein Thymin (T) an Position 501 vom Startkodon umfasst; und wobei das VP7-Gen, in der Nukleotidsequenz, ein Thymin (T) an Position 605 und ein Adenin (A) an Position 897 vom Startkodon umfasst.

7. Verwendung gemäß einem der Ansprüche 1-6, wobei die Zusammensetzung weiterhin Schutz gegen mindestens einen von den nicht-G1 Serotypen verleiht, die ausgewählt sind aus der Gruppe, bestehend aus: G3, G4 und G9.

8. Verwendung gemäß einem der vorangehenden Ansprüche, wobei die Zusammensetzung, die einen attenuierten Rotavirusstamm vom Typ G1P[8] umfasst, Schutz verleiht gegen schwere, Rotavirus-induzierte Gastroenteritis mit einer Punktzahl gleich oder höher als 11 auf der Vesikari Skala, verursacht durch eine Infektion mit einem Rotavirusstamm vom Typ G2P[4].

9. Verwendung gemäß einem der Ansprüche 1 bis 8, wobei die Zusammensetzung, die einen attenuierten Rotavirusstamm vom Typ G1P[8] umfasst, in einer Population von vakzinierten Individuen, mindestens 40% protektiv ist gegen Diarrhoe, die durch einen Rotavirusstamm vom Typ G2P[4] verursacht wird.

10. Verwendung gemäß Anspruch 9, wobei die Zusammensetzung mindestens 50% protektiv ist.

11. Verwendung gemäß Anspruch 9, wobei die Zusammensetzung zwischen 40% und 80% protektiv ist.

12. Verwendung gemäß Anspruch 11, wobei die Zusammensetzung zwischen 50% und 70% protektiv ist.

13. Verwendung gemäß Anspruch 8, wobei die Zusammensetzung in einer Population von vakzinierten Individuen zwischen 40% und 75% protektiv ist gegen schwere Gastroenteritis mit einer Punktzahl gleich oder höher als 11 auf der Vesikari Skala, verursacht durch eine Infektion mit Rotaviren mit einem G2P[4]-Serotyp.

14. Verwendung gemäß einem der Ansprüche 1 bis 13, wobei der Rotavirusstamm in der Zusammensetzung ECACC-Hinterlegung 99081301 ist oder erhältlich oder ableitbar von der ECACC-Hinterlegung 99081301 ist.

15. Verwendung gemäß einem der Ansprüche 1 bis 14, wobei die Zusammensetzung in einem 2-Gaben-Regime verabreicht wird.

16. Verwendung gemäß einem der Ansprüche 1 bis 15, wobei der attenuierte Rotavirusstamm mit einem geeigneten pharmazeutischen Träger oder mit einem Antazidumpuffer oder beiden formuliert ist.

17. Verwendung gemäß einem der Ansprüche 1 bis 16, wobei die Zusammensetzung zum Verleihen von Schutz in einem humanen Subjekt ist.

## Revendications

1. Utilisation d'une souche atténuée de rotavirus d'un type G1P[8] dans la fabrication d'une composition destinée à conférer une protection contre une infection à rotavirus provoquée par une souche de rotavirus d'un type G2P[4].

2. Utilisation selon la revendication 1, dans laquelle la composition comprend un rotavirus possédant un gène VP4 comprenant, dans la séquence nucléotidique, au moins l'une des suivantes : une base adénine (A) au niveau de la position 788, une base adénine (A) au niveau de la position 802 et une base thymine (T) au niveau de la position 501 à partir du codon de départ.

3. Utilisation selon la revendication 2, dans laquelle le gène VP4 comprend une séquence nucléotidique comprenant une base adénine (A) au niveau des positions 788 et 802 et une base thymine (T) au niveau de la position 501 à partir du codon de départ.

4. Utilisation selon l'une quelconque des revendications 1 à 3, dans laquelle la composition comprend un rotavirus possédant un gène VP7 comprenant, dans la séquence nucléotidique, au moins l'une des suivantes : une thymine (T) au niveau de la position 605, une adénine (A) au niveau de la position 897 et une guanine (G) au niveau de la position 897 à partir du codon de départ.

5. Utilisation selon la revendication 4, dans laquelle le gène VP7 comprend une séquence nucléotidique comprenant une thymine (T) au niveau de la position 605 et une adénine (A) ou une guanine (G) au niveau de la position 897 à partir du codon de départ.

6. Utilisation selon l'une quelconque des revendications 1 à 5, dans laquelle la composition comprend un rotavirus possédant un gène VP4 comprenant, dans la séquence nucléotidique, une adénine (A) au niveau des positions 788 et 802 et une thymine (T) au niveau de la position 501 à partir du codon de départ ; et où le gène VP7 comprend, dans la séquence nucléotidique, une thymine (T) au niveau de la position 605 et une adénine (A) au niveau de la position 897 à partir du codon de départ.

7. Utilisation selon l'une quelconque des revendications 1 à 6, dans laquelle la composition confère en outre une protection contre au moins l'un des sérotypes non G1 choisis dans le groupe constitué de : G3, G4 et G9.

8. Utilisation selon l'une quelconque des revendications précédentes, dans laquelle la composition comprenant une souche atténuée de rotavirus d'un type G1P[8] fournit une protection contre une gastroentérite sévère induite par un rotavirus présentant un score égal ou supérieur à 11 sur l'échelle de Vesikari provoquée par une infection d'une souche de rotavirus d'un type G2P[4].

9. Utilisation selon l'une quelconque des revendications 1 à 8, dans laquelle la composition comprenant une souche atténuée de rotavirus d'un type GlP[8] est au moins protectrice à 40 %, dans une population d'individus vaccinés, contre la diarrhée provoquée par une souche de rotavirus qui est d'un type G2P[4].

10. Utilisation selon la revendication 9, dans laquelle la composition est au moins protectrice à 50 %.

11. Utilisation selon la revendication 9, dans laquelle la composition est protectrice entre 40 % et 80 %.

12. Utilisation selon la revendication 11, dans laquelle la composition est protectrice entre 50 % et 70 %.

13. Utilisation selon la revendication 8, dans laquelle la composition est protectrice entre 40 % et 75 % dans une population d'individus vaccinés contre une gastroentérite sévère présentant un score égal ou supérieur à 11 sur l'échelle de Vesikari provoquée par une infection de rotavirus avec un sérotype G2P[4].

14. Utilisation selon l'une quelconque des revendications 1 à 13, dans laquelle la souche de rotavirus dans ladite composition est le dépôt 99081301 de l'ECACC, ou peut être obtenue ou dérivée du dépôt 99081301 de l'ECACC.

15. Utilisation selon l'une quelconque des revendications 1 à 14, dans laquelle la composition est administrée dans un régime de 2 doses.

16. Utilisation selon l'une quelconque des revendications 1 à 15, dans laquelle la souche atténuée de rotavirus est formulée avec un support pharmaceutique approprié ou avec un tampon antiacide ou les deux.

17. Utilisation selon l'une quelconque des revendications 1 à 16, dans laquelle la composition est destinée à conférer une protection chez un sujet humain.

FIG 1A VP4 sequence of P43

```
ATGGCTTCAC TCATTTATAG ACAACTTCTC ACTAATTCAT ATTCAGTAGA   50
TTTACATGAT GAAATAGAGC AAATTGGATC AGAAAAAACT CAGAATGTAA  100
CTATAAATCC GGGTCCATTT GCACAGACTA GATATGCTCC AGTCAATTGG  150
GATCATGGAG AGATAAATGA TTCGACTACA GTAGAACCAA TTTTAGATGG  200
TCCTTATCAG CCAACTACAT TTACTCCACC TAATGATTAT TGGATACTTA  250
TTAATTCAAA TACAAATGGA GTAGTATATG AAAGTACAAA TAATAGTGAC  300
TTTTGGACTG CAGTCGTTGC TATTGAACCG CACGTCAACC CAGTAGATAG  350
ACAATATATG ATATTTGGTG AAAGCAAGCA ATTTAATGTG AGTAACGATT  400
CAAATAAATG GAAGTTTTTA GAAATGTTTA GAAGCAGTAG TCAAAATGAA  450
TTTTATAATA GACGTACATT AACTTCTGAT ACCAGACTTG TAGGAATATT  500
TAAATATGGT GGAAGAGTAT GGACATTTCA TGGTGAAACA CCGAGAGCTA  550
CTACTGACAG TTCAAGTACT GCAAATTTAA ATAATATATC AATTACAATT  600
CATTCAGAAT TTTACATTAT TCCAAGGTCC CAGGAATCTA AATGTAATGA  650
ATATATTAAT AATGGTCTGC CACCAATTCA AAATACTAGA AATGTAGTTC  700
CATTGCCATT ATCATCTAGA TCGATACAGT ATAAGAGAGC ACAAGTTAAT  750
GAAGACATTA TAGTTTCAAA AACTTCATTA TGGAAAGAAA TGCAGTATAA  800
TAGGGATATT ATAATTAGAT TTAAATTTGG TAATAGTATT GTAAAGATGG  850
GAGGACTAGG TTATAAATGG TCTGAAATAT CATATAAGGC AGCAAATTAT  900
CAATATAATT ACTTACGTGA CGGTGAACAA GTAACCGCAC ACACCACTTG  950
TTCAGTAAAT GGAGTGAACA ATTTTAGCTA TAATGGAGGG TTTCTACCCA 1000
CTGATTTTGG TATTTCAAGG TATGAAGTTA TTAAAGAGAA TTCTTATGTA 1050
TATGTAGACT ATTGGGATGA TTCAAAAGCA TTTAGAAATA TGGTATATGT 1100
TAGATCATTA GCAGCTAATT TAAATTCAGT GAAATGTACA GGTGGAAGTT 1150
ATTATTTCAG TATACCAGTA GGTGCATGGC CAGTAATGAA TGGTGGCGCT 1200
GTTTCGTTGC ATTTTGCCGG AGTTACATTA TCCACGCAAT TTACTGATTT 1250
TGTATCATTA AATTCACTAC GATTTAGATT TAGTTTGACA GTTGATGAAC 1300
CACCTTTCTC AATACTGAGA ACACGTACAG TGAATTTGTA TGGATTACCA 1350
GCCGCTAATC CAAATAATGG AAATGAATAC TACGAAATAT CAGGAAGGTT 1400
TTCACTCATT TCTTTAGTTC CAACTAATGA TGATTATCAG ACTCCAATTA 1450
TGAATTCAGT GACGGTAAGA CAAGATTTAG AGCGCCAACT TACTGATTTA 1500
CGAGAAGAAT TTAACTCATT GTCACAAGAA ATAGCTATGG CACAATTGAT 1550
TGATTTAGCA CTGTTGCCTC TAGATATGTT TTCCATGTTT TCAGGAATTA 1600
AAAGTACAAT TGATTTAACT AAATCAATGG CGACTAGTGT AATGAAGAAA 1650
TTTAGAAAAT CAAAATTAGC TACATCAATT TCAGAAATGA CTAATTCATT 1700
GTCAGATGCT GCTTCATCAG CATCAAGAAA CGTTTCTATT AGATCGAATT 1750
TATCTGCGAT TTCAAATTGG ACTAATGTTT CAAATGATGT GTCAAACGTA 1800
ACTAATTCAT TGAACGATAT TTCAACACAA ACATCTACAA TTAGTAAGAA 1850
ACTTAGATTA AAAGAAATGA TTACTCAAAC TGAAGGAATG AGCTTTGACG 1900
ACATTTCAGC AGCTGTACTA AAAACAAAAA TAGATATGTC TACTCAAATT 1950
GGAAAAAATA CTTTACCTGA TATAGTTACA GAAGCATCTG AGAAATTTAT 2000
TCCAAAACGA TCATATCGAA TATTAAAGGA TGATGAAGTA ATGGAAATTA 2050
ATACTGAAGG AAAATTCTTT GCATACAAAA TTAATACATT TGATGAAGTG 2100
CCATTCGATG TAAATAAATT CGCTGAACTA GTAACAGATT CTCCAGTTAT 2150
ATCAGCGATA ATCGATTTTA AGACATTGAA AAATTTAAAT GATAATTATG 2200
GAATCACTCG TACAGAAGCG TTAAATTTAA TTAAATCGAA TCCAAATATG 2250
TTACGTAATT TCATTAATCA AAATAATCCA ATTATAAGGA ATAGAATTGA 2300
ACAGTTAATA CTACAATGTA AATTGTGAGA ACGCTATTGA GGATGTGACC 2350
```

FIG 1B VP4 sequence of P43 (RIX4414 VP4; 2359 bp)

```
1    ggctataaaA TGGCTTCGCT CATTTATAGA CAACTTCTCA CTAATTCATA TTCAGTAGAT
61   TTACATGATG AAATAGAGCA AATTGGATCA GAAAAAACTC AGAATGTAAC TATAAATCCG
121  GGTCCATTTG CACAGACTAG ATATGCTCCA GTCAATTGGG ATCATGGAGA GATAAATGAT
181  TCGACTACAG TAGAACCAAT TTTAGATGGT CCTTATCAGC CAACTACATT TACTCCACCT
241  AATGATTATT GGATACTTAT TAATTCAAAT ACAAATGGAG TAGTATATGA AAGTACAAAT
301  AATAGTGACT TTTGGACTGC AGTCGTTGCT ATTGAACCGC ACGTCAACCC AGTAGATAGA
361  CAATATATGA TATTTGGTGA AAGCAAGCAA TTTAATGTGA GTAACGATTC AAATAAATGG
421  AAGTTTTTAG AAATGTTTAG AAGCAGTAGT CAAAATGAAT TTTATAATAG ACGTACATTA
481  ACTTCTGATA CCAGACTTGT AGGAATATTT AAATATGGTG GAAGAGTATG GACATTTCAT
541  GGTGAAACAC CGAGAGCTAC TACTGACAGT TCAAGTACTG CAAATTTAAA TAATATATCA
601  ATTACAATTC ATTCAGAATT TTACATTATT CCAAGGTCCC AGGAATCTAA ATGTAATGAA
661  TATATTAATA ATGGTCTGCC ACCAATTCAA AATACTAGAA ATGTAGTTCC ATTGCCATTA
721  TCATCTAGAT CGATACAGTA TAAGAGAGCA CAAGTTAATG AAGACATTAT AGTTTCAAAA
781  ACTTCATTAT GGAAAGAAAT GCAGTATAAT AGGGATATTA TAATTAGATT TAAATTTGGT
841  AATAGTATTG TAAAGATGGG AGGACTAGGT TATAAATGGT CTGAAATATC ATATAAGGCA
901  GCAAATTATC AATATAATTA CTTACGTGAC GGTGAACAAG TAACCGCACA CACCACTTGT
961  TCAGTAAATG GAGTGAACAA TTTTAGCTAT AATGGAGGGT TTCTACCCAC TGATTTTGGT
1021 ATTTCAAGGT ATGAAGTTAT TAAAGAGAAT TCTTATGTAT ATGTAGACTA TTGGGATGAT
1081 TCAAAAGCAT TTAGAAATAT GGTATATGTT AGATCATTAG CAGCTAATTT AAATTCAGTG
1141 AAATGTACAG GTGGAAGTTA TTATTTCAGT ATACCAGTAG GTGCATGGCC AGTAATGAAT
1201 GGTGGCGCTG TTTCGTTGCA TTTTGCCGGA GTTACATTAT CCACGCAATT TACTGATTTT
1261 GTATCATTAA ATTCACTACG ATTTAGATTT AGTTTGACAG TTGATGAACC ACCTTTCTCA
1321 ATACTGAGAA CACGTACAGT GAATTTGTAT GGATTACCAG CCGCTAATCC AAATAATGGA
1381 AATGAATACT ACGAAATATC AGGAAGGTTT TCACTCATTT CTTTAGTTCC AACTAATGAT
1441 GATTATCAGA CTCCAATTAT GAATTCAGTG ACGGTAAGAC AAGATTTAGA GCGCCAACTT
1501 ACTGATTTAC GAGAAGAATT TAACTCATTG TCACAAGAAA TAGCTATGGC ACAATTGATT
1561 GATTTAGCAC TGTTGCCTCT AGATATGTTT TCCATGTTTT CAGGAATTAA AAGTACAATT
1621 GATTTAACTA AATCAATGGC GACTAGTGTA ATGAAGAAAT TTAGAAAATC AAAATTAGCT
1681 ACATCAATTT CAGAAATGAC TAATTCATTG TCAGATGCTG CTTCATCAGC ATCAAGAAAC
1741 GTTTCTATTA GATCGAATTT ATCTGCGATT TCAAATTGGA CTAATGTTTC AAATGATGTG
1801 TCAAACGTAA CTAATTCATT GAACGATATT TCAACACAAA CATCTACAAT TAGTAAGAAA
1861 CTTAGATTAA AAGAAATGAT TACTCAAACT GAAGGAATGA GCTTTGACGA CATTTCAGCA
1921 GCTGTACTAA AAACAAAAAT AGATATGTCT ACTCAAATTG GAAAAAATAC TTTACCTGAT
1981 ATAGTTACAG AAGCATCTGA GAAATTTATT CCAAAACGAT CATATCGAAT ATTAAAGGAT
2041 GATGAAGTAA TGGAAATTAA TACTGAAGGA AAATTCTTTG CATACAAAAT TAATACATTT
2101 GATGAAGTGC CATTCGATGT AAATAAATTC GCTGAACTAG TAACAGATTC TCCAGTTATA
2161 TCAGCGATAA TCGATTTTAA GACATTGAAA AATTTAAATG ATAATTATGG AATCACTCGT
2221 ACAGAAGCGT TAAATTTAAT TAAATCGAAT CCAAATATGT TACGTAATTT CATTAATCAA
2281 AATAATCCAA TTATAAGGAA TAGAATTGAA CAGTTAATAC TACAATGTAA ATTGTGAgaa
2341 cgctattgag gatgtgacc
```

Fig 2A VP7 sequence of P43

```
ATGTATGGTC TTGAATATAC CACAATTCTA ATCTTTCTGA TATCAATTAT  50
TCTACTCAAC TATATATTAA AATCAGTAAC TCGAATAATG GACTACATTA  100
TATATAGATC TTTGTTGATT TATGTAGCAT TATTTGCCTT GACAAGAGCT  150
CAGAATTATG GGCTTAACTT ACCAATAACA GGATCAATGG ACACTGTATA  200
CGCTAACTCT ACTCAAGAAG GAATATTTCT AACATCCACA TTATGTTTGT  250
ATTATCCAAC TGAAGCAAGT ACTCAAATTA ATGATGGTGA ATGGAAAGAC  300
TCATTGTCAC AAATGTTTCT CACAAAAGGT TGGCCAACAG GATCAGTCTA  350
TTTTAAAGAG TATTCAAGTA TTGTTGATTT TTCTGTCGAT CCACAATTAT  400
ATTGTGATTA TAACTTAGTA CTAATGAAAT ATGATCAAAA TCTTGAATTA  450
GATATGTCAG AGTTAGCTGA TTTAATATTG AATGAATGGT TATGTAATCC  500
AATGGATATA ACATTATATT ATTATCAACA ATCGGGAGAA TCAAATAAGT  550
GGATATCAAT GGGATCATCA TGTACTGTGA AAGTGTGTCC ACTGAATACG  600
CAAATGTTAG GAATAGGTTG TCAAACAACA AATGTAGACT CGTTTGAAAT  650
GGTTGCTGAG AATGAGAAAT TAGCTATAGT GGATGTCGTT GATGGGATAA  700
ATCATAAAAT AAATTTGACA ACTACGACAT GTACTATTCG AAATTGTAAG  750
AAGTTAGGTC CAAGAGAGAA TGTAGCTGTA ATACAAGTTG GTGGCTCTAA  800
TGTATTAGAC ATAACAGCAG ATCCAACGAC TAATCCACAA ACTGAGAGAA  850
TGATGAGAGT GAATTGGAAA AAATGGTGGC AAGTATTTTA TACTATAGTA  900
GATTATATTA ACCAAATCGT GCAGGTAATG TCCAAAAGAT CAAGATCATT  950
AAATTCTGCA GCTTTTTATT ATAGAGTATA GATATATCTT AGATTAGATC  1000
GATGTGACC
```

Fig 2B VP7 sequence of P43 - (RIX4414 VP7; 1046 bp)

```
1    ggctttaaaa gagagaattt ccgtctggct aacggttagc tccttttaAT GTATGGTATT
61   GAATATACCA CAATTCTAAT CTTTCTGATA TCAATTATTC TACTCAACTA TATATTAAAA
121  TCAGTAACTC GAATAATGGA CTACATTATA TATAGATCTT TGTTGATTTA TGTAGCATTA
181  TTTGCCTTGA CAAGAGCTCA GAATTATGGG CTTAACTTAC CAATAACAGG ATCAATGGAC
241  ACTGTATACG CTAACTCTAC TCAAGAAGGA ATATTTCTAA CATCCACATT ATGTTTGTAT
301  TATCCAACTG AAGCAAGTAC TCAAATTAAT GATGGTGAAT GGAAAGACTC ATTGTCACAA
361  ATGTTTCTCA CAAAAGGTTG GCCAACAGGA TCAGTCTATT TTAAAGAGTA TTCAAGTATT
421  GTTGATTTTT CTGTCGATCC ACAATTATAT TGTGATTATA ACTTAGTACT AATGAAATAT
481  GATCAAAATC TTGAATTAGA TATGTCAGAG TTAGCTGATT TAATATTGAA TGAATGGTTA
541  TGTAATCCAA TGGATATAAC ATTATATTAT TATCAACAAT CGGGAGAATC AAATAAGTGG
601  ATATCAATGG GATCATCATG TACTGTGAAA GTGTGTCCAC TGAATACGCA AATGTTAGGA
661  ATAGGTTGTC AAACAACAAA TGTAGACTCG TTTGAAATGG TTGCTGAGAA TGAGAAATTA
721  GCTATAGTGG ATGTCGTTGA TGGGATAAAT CATAAAATAA ATTTGACAAC TACGACATGT
781  ACTATTCGAA ATTGTAAGAA GTTAGGTCCA AGAGAGAATG TAGCTGTAAT ACAAGTTGGT
841  GGCTCTAATG TATTAGACAT AACAGCAGAT CCAACGACTA ATCCACAAAC TGAGAGAATG
901  ATGAGAGTGA ATTGGAAAAA ATGGTGGCAA GTATTTTATA CTATAGTAGA TTATATTAAC
961  CAAATCGTGC AGGTAATGTC CAAAAGATCA AGATCATTAA ATTCTGCAGC TTTTTATTAT
1021 AGAGTATAGa tatatcttag attaga
```

Figure 3 - Polypeptide sequence of P43 VP4. (RIX4414 VP4.pro; 775 aa)

```
1   MASLIYRQLL TNSYSVDLHD EIEQIGSEKT QNVTINPGPF AQTRYAPVNW DHGEINDSTT
61  VEPILDGPYQ PTTFTPPNDY WILINSNTNG VVYESTNNSD FWTAVVAIEP HVNPVDRQYM
121 IFGESKQFNV SNDSNKWKFL EMFRSSSQNE FYNRRTLTSD TRLVGIFKYG GRVWTFHGET
181 PRATTDSSST ANLNNISITI HSEFYIIPRS QESKCNEYIN NGLPPIQNTR NVVPLPLSSR
241 SIQYKRAQVN EDIIVSKTSL WKEMQYNRDI IIRFKFGNSI VKMGGLGYKW SEISYKAANY
301 QYNYLRDGEQ VTAHTTCSVN GVNNFSYNGG FLPTDFGISR YEVIKENSYV YVDYWDDSKA
361 FRNMVYVRSL AANLNSVKCT GGSYYFSIPV GAWPVMNGGA VSLHFAGVTL STQFTDFVSL
421 NSLRFRFSLT VDEPPFSILR TRTVNLYGLP AANPNNGNEY YEISGRFSLI SLVPTNDDYQ
481 TPIMNSVTVR QDLERQLTDL REEFNSLSQE IAMAQLIDLA LLPLDMFSMF SGIKSTIDLT
541 KSMATSVMKK FRKSKLATSI SEMTNSLSDA ASSASRNVSI RSNLSAISNW TNVSNDVSNV
601 TNSLNDISTQ TSTISKKLRL KEMITQTEGM SFDDISAAVL KTKIDMSTQI GKNTLPDIVT
661 EASEKFIPKR SYRILKDDEV MEINTEGKFF AYKINTFDEV PFDVNKFAEL VTDSPVISAI
721 IDFKTLKNLN DNYGITRTEA LNLIKSNPNM LRNFINQNNP IIRNRIEQLI LQCKL
```

Figure 4 - Polypeptide sequence of P43 VP7 (RIX4414 VP7.pro; 326 aa).

```
1   MYGIEYTTIL IFLISIILLN YILKSVTRIM DYIIYRSLLI YVALFALTRA QNYGLNLPIT
61  GSMDTVYANS TQEGIFLTST LCLYYPTEAS TQINDGEWKD SLSQMFLTKG WPTGSVYFKE
121 YSSIVDFSVD PQLYCDYNLV LMKYDQNLEL DMSELADLIL NEWLCNPMDI TLYYYQQSGE
181 SNKWISMGSS CTVKVCPLNT QMLGIGCQTT NVDSFEMVAE NEKLAIVDVV DGINHKINLT
241 TTTCTIRNCK KLGPRENVAV IQVGGSNVLD ITADPTTNPQ TERMMRVNWK KWWQVFYTIV
301 DYINQIVQVM SKRSRSLNSA AFYYRV
```

Figure 5 - Polypeptide sequence of NSP4 protein of RIX4414 (175 aa).

```
1   MDKLADLNYT LSVITLMNDT LHSIIQDPGM AYFSYIASVL TVLFILHKAS IPTMKIALKT
61  SKCSYKVIKY CIVTIINTLL KLAGYKEQVT TKDEIEQQMD RIVKEMRRQL DMIDKLTTRE
121 IEQVELLKRI HDNLITRPVD VIDMSKEFNQ KNIKTLDEWE SGKNPYEPSE VTASM
```

Figure 6 - Nucleotide sequence encoding NSP4 protein of RIX4414 (750 bp).

```
1   ggcttttaaa agttctgttc cgagagagcg cgtgcggaaa gATGGATAAG CTTGCCGACC
61  TCAACTACAC ATTGAGTGTA ATCACTTTAA TGAATGACAC ATTGCATTCT ATAATTCAAG
121 ATCCTGGAAT GGCGTATTTT TCATATATTG CATCTGTTCT AACAGTTTTA TTCATATTAC
181 ATAAAGCTTC AATTCCAACC ATGAAAATAG CATTGAAAAC ATCAAAATGT TCATATAAAG
241 TGATTAAATA TTGTATAGTC ACGATCATTA ATACTCTTTT AAAATTGGCT GGATATAAAG
301 AGCAGGTTAC TACAAAAGAC GAAATTGAGC AACAGATGGA CAGAATTGTT AAAGAGATGA
361 GACGTCAGCT GGATATGATT GATAAATTAA CTACTCGTGA AATTGAACAG GTTGAATTGC
421 TTAAACGTAT ACATGACAAC CTGATAACTA GACCAGTTGA CGTCATAGAT ATGTCGAAGG
481 AATTCAATCA GAAAAACATC AAAACGCTAG ATGAATGGGA GAGTGGAAAA AATCCATATG
541 AACCGTCAGA AGTGACTGCA TCCATGTGAg aggttgagtt accgtcgtct gtcttcggaa
601 gcggcggaac tcttcaccgc aagccccatt agacctgatg attgactgag aagccacagt
661 caatcatatc gcgtgtggct cagccttaat cccgtttaac caatccagcg agtgttggac
721 gttaatggaa ggaatggtct tagtgtgacc
```

Figure 7 - Polypeptide sequence of VP6 protein of RIX4414 (397 aa).

```
1   MEVLYSLSKT LKDARDKIVE GTLYSNVSDL IQQFNQMIVT MNGNDFQTGG IGNLPVRNWT
61  FDFGLLGTTL LNLDANYVEN ARTTIEYFID FIDNVCMDEM ARESQRNGVS PQSEALRKLA
121 GIKFKRINFD NSSEYIENWN LQNRRQRTGF VFHKPNIFPY SASFTLNRSQ PMHDNLMGTM
181 WLNAGSEIQV AGFDYSCAIN APANIQQFEH IVQLRRALTT ATITLLPDAE RFSFPRVINS
241 ADGATTWFFN PVILRPNNVE VEFLLNGQII NTYQARFGTI IARNFDTIRL LFQLMRPPNM
301 TPAVNALFPQ AQPFQHHATV GLTLRIESAV CESVLADANE TLLANVTAVR QEYAIPVGPV
361 FPPGMNWTEL ITNYSPSRED NLQRVFTVAS IRSMLIK
```

Figure 8 - Nucleotide sequence encoding VP6 protein of RIX4414 (1356 bp).

```
1    ggctttaaaa cgaagtcttc gacATGGAGG TTCTGTACTC ACTGTCAAAA ACTCTTAAAG
61   ATGCTAGGGA CAAAATTGTT GAAGGTACAT TATATTCTAA CGTTAGCGAT CTTATTCAGC
121  AATTCAATCA AATGATAGTA ACTATGAATG GAAATGACTT TCAGACTGGA GGAATTGGTA
181  ATTTACCTGT TAGAAATTGG ACTTTCGATT TTGGTCTATT AGGTACAACA CTTTTGAACT
241  TGGATGCTAA TTATGTTGAG AATGCAAGAA CTACAATTGA ATATTTTATT GACTTTATTG
301  ATAATGTATG TATGGATGAA ATGGCAAGAG AATCTCAAAG AAATGGAGTA TCGCCACAAT
361  CTGAAGCGTT AAGAAAGCTA GCGGGAATTA AATTTAAGAG AATAAATTTC GATAATTCAT
421  CAGAATACAT AGAAAATTGG AACTTACAAA ATAGAAGACA GCGCACCGGA TTTGTTTTTC
481  ATAAACCTAA CATTTTTCCA TACTCAGCTT CATTTACTCT AAATAGATCT CAACCAATGC
541  ATGATAATTT AATGGGAACC ATGTGGCTTA ATGCTGGATC AGAAATTCAA GTGGCTGGAT
601  TTGATTACTC ATGCGCCATA AATGCACCAG CGAACATACA GCAATTTGAG CATATCGTTC
661  AGCTTAGGCG CGCACTGACT ACAGCTACTA TAACTTTATT ACCTGATGCA GAGAGATTTA
721  GTTTTCCAAG AGTAATTAAT TCAGCTGATG GCGCGACTAC ATGGTTCTTT AATCCAGTTA
781  TTCTAAGACC AAACAATGTA GAGGTAGAAT TTTTATTGAA TGGACAAATT ATTAATACAT
841  ATCAGGCTAG ATTTGGTACT ATCATCGCAA GAAATTTTGA TACAATTCGT TTATTATTTC
901  AGTTGATGCG TCCACCTAAT ATGACACCAG CTGTTAATGC ACTATTTCCA CAAGCACAAC
961  CTTTTCAGCA CCATGCAACA GTTGGACTTA CATTACGTAT TGAATCTGCG GTTTGTGAAT
1021 CAGTGCTTGC GGACGCGAAT GAAACTCTGT TAGCAAATGT GACCGCGGTG CGTCAAGAAT
1081 ATGCCATACC AGTTGGACCG GTATTTCCAC CAGGCATGAA TTGGACTGAA TTGATTACTA
1141 ACTATTCGCC ATCTAGAGAA GATAACTTGC AACGCGTTTT CACGGTAGCT TCCATTAGAA
1201 GCATGTTGAT TAAGTGAgga ccagactaaa catctggtat ccaatcttag ttagcatgta
1261 gctacatcaa gtcattcaga ctcttcaagt aaggacatga tttcatgttc gctacgtaga
1321 gtaactgtct gaatgatgta gtgagaggat gtgacc
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5474773 A
- WO 0112797 A
- WO 05021033 A
- WO 9606627 A
- WO 9313202 A
- US 5182109 A
- WO 9856415 A
- WO 9400153 A
- GB 2122204 A


### Non-patent literature cited in the description


- **WALSH, J.A. et al.** *N. Engl. J. Med.,* 1979, vol. 301, 967-974
- **ESTES, M.K. et al.** Rotaviruses and Their Replication in Fields Virology. Raven Publishers, 1996
- **OFFIT, P.A. et al.** *Comp. Ther.,* 1982, vol. 8 (8), 21-26
- **SANTOS N. ; HOSHINO Y.** *Reviews in Medical Virology,* 2005, vol. 15, 29-56
- **BERNSTEIN, D.L. et al.** *Vaccine,* 1998, vol. 16 (4), 381-387
- **WARD.** *J. Clin. Microbiol.,* 1984, vol. 19, 748-753
- **PEREZ et al.** *42nd Interscience Conference on Antimicrobial Agents and Chemotherapy,* 27 September 2002
- **WARD et al.** *J. Infect. Disease,* 1990, vol. 161, 440-445
- **RUUSKA T et al.** *Scand. J. Infect. Dis.,* 1990, vol. 22, 259-267
- **CLARK HF ; BORIAN EF ; BELL LM.** Protective effect of WC3 vaccine against rotavirus diarrhea in infants during a predominantly serotype 1 rotavirus season. *J Infect Dis.,* 1988, 570-86
- **FOSTER, R. H. ; WAGSTAFF, A. J.** Tetravalent Rotavirus Vaccine, a review. ADIS drug evaluation. *BioDrugs, Gev,* 1998, vol. 9 (2), 155-178
- Vaccine Design, The Subunit and Adjuvant Approach. Plenum Press, 1995
- *Vaccine,* 1998
- **J.J. POWELL ; R.JUGDAOHSINGH ; R.P.H. THOMPSON.** The regulation of mineral adsorption in the gastrointestinal track. *Proceedings of the Nutrition Society,* 1999, vol. 58, 147-153
- Fields. Raven press, 1990, 1361

### SEQUENCE LISTING

<110> GlaxoSmithKline Biologicals s.a.

<120> Vaccine

<130> VB61582

<160> 42

<170> FastSEQ for Windows Version 4.0

<210> 1
<211> 2350
<212> DNA
<213> Rotavirus

<400> 1

```
atggcttcac tcatttatag acaacttctc actaattcat attcagtaga tttacatgat 60
gaaatagagc aaattggatc agaaaaaact cagaatgtaa ctataaatcc gggtccattt 120
gcacagacta gatatgctcc agtcaattgg gatcatggag agataaatga ttcgactaca 180
gtagaaccaa ttttagatgg tccttatcag ccaactacat ttactccacc taatgattat 240
tggatactta ttaattcaaa tacaaatgga gtagtatatg aaagtacaaa taatagtgac 300
ttttggactg cagtcgttgc tattgaaccg cacgtcaacc cagtagatag acaatatatg 360
atatttggtg aaagcaagca atttaatgtg agtaacgatt caaataaatg gaagttttta 420
gaaatgttta gaagcagtag tcaaaatgaa ttttataata gacgtacatt aacttctgat 480
accagacttg taggaatatt taaatatggt ggaagagtat ggacatttca tggtgaaaca 540
ccgagagcta ctactgacag ttcaagtact gcaaatttaa ataatatatc aattacaatt 600
cattcagaat tttacattat tccaaggtcc caggaatcta aatgtaatga atatattaat 660
aatggtctgc caccaattca aaatactaga aatgtagttc cattgccatt atcatctaga 720
tcgatacagt ataagagagc acaagttaat gaagacatta tagtttcaaa aacttcatta 780
tggaaagaaa tgcagtataa tagggatatt ataattagat ttaaatttgg taatagtatt 840
gtaaagatgg gaggactagg ttataaatgg tctgaaatat catataaggc agcaaattat 900
caatataatt acttacgtga cggtgaacaa gtaaccgcac acaccacttg ttcagtaaat 960
ggagtgaaca attttagcta taatggaggg tttctaccca ctgattttgg tatttcaagg 1020
tatgaagtta ttaaagagaa ttcttatgta tatgtagact attgggatga ttcaaaagca 1080
tttagaaata tggtatatgt tagatcatta gcagctaatt taaattcagt gaaatgtaca 1140
ggtggaagtt attatttcag tataccagta ggtgcatggc cagtaatgaa tggtggcgct 1200
gtttcgttgc atttgccgg agttacatta tccacgcaat ttactgattt tgtatcatta 1260
aattcactac gatttagatt tagtttgaca gttgatgaac cacctttctc aatactgaga 1320
acacgtacag tgaatttgta tggattacca gccgctaatc caaataatgg aaatgaatac 1380
tacgaaatat caggaaggtt ttcactcatt tctttagttc caactaatga tgattatcag 1440
actccaatta tgaattcagt gacggtaaga caagatttag agcgccaact tactgattta 1500
cgagaagaat ttaactcatt gtcacaagaa atagctatgg cacaattgat tgatttagca 1560
ctgttgcctc tagatatgtt ttccatgttt tcaggaatta aaagtacaat tgatttaact 1620
aaatcaatgg cgactagtgt aatgaagaaa tttagaaaat caaaattagc tacatcaatt 1680
tcagaaatga ctaattcatt gtcagatgct gcttcatcag catcaagaaa cgtttctatt 1740
agatcgaatt tatctgcgat ttcaaattgg actaatgttt caaatgatgt gtcaaacgta 1800
actaattcat tgaacgatat ttcaacacaa acatctacaa ttagtaagaa acttagatta 1860
aaagaaatga ttactcaaac tgaaggaatg agctttgacg acatttcagc agctgtacta 1920
aaaacaaaaa tagatatgtc tactcaaatt ggaaaaaata ctttacctga tatagttaca 1980
gaagcatctg agaaatttat tccaaaacga tcatatcgaa tattaaagga tgatgaagta 2040
atggaaatta atactgaagg aaaattcttt gcatacaaaa ttaatacatt tgatgaagtg 2100
ccattcgatg taaataaatt cgctgaacta gtaacagatt ctccagttat atcagcgata 2160
atcgatttta agacattgaa aaatttaaat gataattatg gaatcactcg tacagaagcg 2220

ttaaatttaa ttaaatcgaa tccaaatatg ttacgtaatt tcattaatca aaataatcca 2280
attataagga atagaattga acagttaata ctacaatgta aattgtgaga acgctattga 2340
ggatgtgacc 2350
```

<210> 2
<211> 2359
<212> DNA
<213> Rotavirus

<400> 2

```
ggctataaaa tggcttcgct catttataga caacttctca ctaattcata ttcagtagat 60
ttacatgatg aaatagagca aattggatca gaaaaaactc agaatgtaac tataaatccg 120
ggtccatttg cacagactag atatgctcca gtcaattggg atcatggaga gataaatgat 180
tcgactacag tagaaccaat tttagatggt ccttatcagc caactacatt tactccacct 240
aatgattatt ggatacttat taattcaaat acaaatggag tagtatatga aagtacaaat 300
aatagtgact tttggactgc agtcgttgct attgaaccgc acgtcaaccc agtagataga 360
caatatatga tatttggtga aagcaagcaa tttaatgtga gtaacgattc aaataaatgg 420
aagtttttag aaatgtttag aagcagtagt caaaatgaat tttataatag acgtacatta 480
acttctgata ccagacttgt aggaatattt aaatatggtg gaagagtatg gacatttcat 540
ggtgaaacac cgagagctac tactgacagt tcaagtactg caaatttaaa taatatatca 600
attacaattc attcagaatt ttacattatt ccaaggtccc aggaatctaa atgtaatgaa 660
tatattaata atggtctgcc accaattcaa aatactagaa atgtagttcc attgccatta 720
tcatctagat cgatacagta taagagagca caagttaatg aagacattat agtttcaaaa 780
acttcattat ggaaagaaat gcagtataat agggatatta taattagatt taaatttggt 840
aatagtattg taaagatggg aggactaggt tataaatggt ctgaaatatc atataaggca 900
gcaaattatc aatataatta cttacgtgac ggtgaacaag taaccgcaca caccacttgt 960
tcagtaaatg gagtgaacaa ttttagctat aatggagggt ttctacccac tgatttttggt 1020
atttcaaggt atgaagttat taaagagaat tcttatgtat atgtagacta ttgggatgat 1080
tcaaaagcat ttagaaatat ggtatatgtt agatcattag cagctaattt aaattcagtg 1140
aaatgtacag gtggaagtta ttatttcagt ataccagtag gtgcatggcc agtaatgaat 1200
ggtggcgctg tttcgttgca ttttgccgga gttacattat ccacgcaatt tactgatttt 1260
gtatcattaa attcactacg atttagattt agtttgacag ttgatgaacc acctttctca 1320
atactgagaa cacgtacagt gaatttgtat ggattaccag ccgctaatcc aaataatgga 1380
aatgaatact acgaaatatc aggaaggttt tcactcattt ctttagttcc aactaatgat 1440
gattatcaga ctccaattat gaattcagtg acggtaagac aagatttaga gcgccaactt 1500
actgatttac gagaagaatt taactcattg tcacaagaaa tagctatggc acaattgatt 1560
gatttagcac tgttgcctct agatatgttt tccatgtttt caggaattaa aagtacaatt 1620
gatttaacta aatcaatggc gactagtgta atgaagaaat ttagaaaatc aaaattagct 1680
acatcaattt cagaaatgac taattcattg tcagatgctg cttcatcagc atcaagaaac 1740
gtttctatta gatcgaattt atctgcgatt tcaaattgga ctaatgtttc aaatgatgtg 1800
tcaaacgtaa ctaattcatt gaacgatatt tcaacacaaa catctacaat tagtaagaaa 1860
cttagattaa aagaaatgat tactcaaact gaaggaatga gctttgacga catttcagca 1920
gctgtactaa aaacaaaaat agatatgtct actcaaattg gaaaaaatac tttacctgat 1980
atagttacag aagcatctga gaaatttatt ccaaaacgat catatcgaat attaaaggat 2040
gatgaagtaa tggaaattaa tactgaagga aaattctttg catacaaaat taatacattt 2100
gatgaagtgc cattcgatgt aaataaattc gctgaactag taacagattc tccagttata 2160
tcagcgataa tcgattttaa gacattgaaa aatttaaatg ataattatgg aatcactcgt 2220
acagaagcgt taaatttaat taaatcgaat ccaaatatgt tacgtaattt cattaatcaa 2280
aataatccaa ttataaggaa tagaattgaa cagttaatac tacaatgtaa attgtgagaa 2340
cgctattgag gatgtgacc                                              2359
```

<210> 3
<211> 1009
<212> DNA
<213> Rotavirus

<400> 3

```
atgtatggtc ttgaatatac cacaattcta atctttctga tatcaattat tctactcaac 60
tatatattaa aatcagtaac tcgaataatg gactacatta tatatagatc tttgttgatt 120
tatgtagcat tatttgcctt gacaagagct cagaattatg ggcttaactt accaataaca 180
ggatcaatgg acactgtata cgctaactct actcaagaag gaatatttct aacatccaca 240
ttatgtttgt attatccaac tgaagcaagt actcaaatta atgatggtga atggaaagac 300
tcattgtcac aaatgtttct cacaaaaggt tggccaacag gatcagtcta ttttaaagag 360
tattcaagta ttgttgattt ttctgtcgat ccacaattat attgtgatta taacttagta 420
ctaatgaaat atgatcaaaa tcttgaatta gatatgtcag agttagctga tttaatattg 480
aatgaatggt tatgtaatcc aatggatata acattatatt attatcaaca atcgggagaa 540
tcaaataagt ggatatcaat gggatcatca tgtactgtga aagtgtgtcc actgaatacg 600
caaatgttag gaataggttg tcaaacaaca aatgtagact cgtttgaaat ggttgctgag 660
aatgagaaat tagctatagt ggatgtcgtt gatgggataa atcataaaat aaatttgaca 720
actacgacat gtactattcg aaattgtaag aagttaggtc caagagagaa tgtagctgta 780
atacaagttg gtggctctaa tgtattagac ataacagcag atccaacgac taatccacaa 840
actgagagaa tgatgagagt gaattggaaa aaatggtggc aagtatttta tactatagta 900
gattatatta accaaatcgt gcaggtaatg tccaaaagat caagatcatt aaattctgca 960
gctttttatt atagagtata gatatatctt agattagatc gatgtgacc            1009
```

<210> 4
<211> 1046
<212> DNA
<213> Rotavirus

<400> 4

```
ggctttaaaa gagagaattt ccgtctggct aacggttagc tccttttaat gtatggtatt 60
gaatatacca caattctaat ctttctgata tcaattattc tactcaacta tatattaaaa 120
tcagtaactc gaataatgga ctacattata tatagatctt tgttgattta tgtagcatta 180
tttgccttga caagagctca gaattatggg cttaacttac caataacagg atcaatggac 240
actgtatacg ctaactctac tcaagaagga atatttctaa catccacatt atgtttgtat 300
tatccaactg aagcaagtac tcaaattaat gatggtgaat ggaaagactc attgtcacaa 360
atgtttctca caaaaggttg gccaacagga tcagtctatt ttaaagagta ttcaagtatt 420
gttgattttt ctgtcgatcc acaattatat tgtgattata acttagtact aatgaaatat 480
gatcaaaatc ttgaattaga tatgtcagag ttagctgatt taatattgaa tgaatggtta 540
tgtaatccaa tggatataac attatattat tatcaacaat cgggagaatc aaataagtgg 600
atatcaatgg gatcatcatg tactgtgaaa gtgtgtccac tgaatacgca aatgttagga 660
ataggttgtc aaacaacaaa tgtagactcg tttgaaatgg ttgctgagaa tgagaaatta 720
gctatagtgg atgtcgttga tgggataaat cataaaataa atttgacaac tacgacatgt 780
actattcgaa attgtaagaa gttaggtcca agagagaatg tagctgtaat acaagttggt 840
ggctctaatg tattagacat aacagcagat ccaacgacta atccacaaac tgagagaatg 900
atgagagtga attggaaaaa atggtggcaa gtattttata ctatagtaga ttatattaac 960
caaatcgtgc aggtaatgtc caaaagatca agatcattaa attctgcagc tttttattat 1020
agagtataga tatatcttag attaga                                      1046
```

<210> 5
<211> 775
<212> PRT
<213> Rotavirus

<400> 5

```
Met Ala Ser Leu Ile Tyr Arg Gln Leu Leu Thr Asn Ser Tyr Ser Val
1               5                   10                  15
Asp Leu His Asp Glu Ile Glu Gln Ile Gly Ser Glu Lys Thr Gln Asn
            20                  25                  30
Val Thr Ile Asn Pro Gly Pro Phe Ala Gln Thr Arg Tyr Ala Pro Val
        35                  40                  45
Asn Trp Asp His Gly Glu Ile Asn Asp Ser Thr Thr Val Glu Pro Ile
    50                  55                  60
```

```
Leu Asp Gly Pro Tyr Gln Pro Thr Thr Phe Thr Pro Pro Asn Asp Tyr
65                  70                  75                  80
Trp Ile Leu Ile Asn Ser Asn Thr Asn Gly Val Val Tyr Glu Ser Thr
                85                  90                  95
Asn Asn Ser Asp Phe Trp Thr Ala Val Val Ala Ile Glu Pro His Val
            100                 105                 110
Asn Pro Val Asp Arg Gln Tyr Met Ile Phe Gly Glu Ser Lys Gln Phe
        115                 120                 125
Asn Val Ser Asn Asp Ser Asn Lys Trp Lys Phe Leu Glu Met Phe Arg
    130                 135                 140
Ser Ser Ser Gln Asn Glu Phe Tyr Asn Arg Arg Thr Leu Thr Ser Asp
145                 150                 155                 160
Thr Arg Leu Val Gly Ile Phe Lys Tyr Gly Gly Arg Val Trp Thr Phe
                165                 170                 175
His Gly Glu Thr Pro Arg Ala Thr Thr Asp Ser Ser Ser Thr Ala Asn
            180                 185                 190
Leu Asn Asn Ile Ser Ile Thr Ile His Ser Glu Phe Tyr Ile Ile Pro
        195                 200                 205
Arg Ser Gln Glu Ser Lys Cys Asn Glu Tyr Ile Asn Asn Gly Leu Pro
    210                 215                 220
Pro Ile Gln Asn Thr Arg Asn Val Val Pro Leu Pro Leu Ser Ser Arg
225                 230                 235                 240
Ser Ile Gln Tyr Lys Arg Ala Gln Val Asn Glu Asp Ile Ile Val Ser
                245                 250                 255
Lys Thr Ser Leu Trp Lys Glu Met Gln Tyr Asn Arg Asp Ile Ile Ile
            260                 265                 270
Arg Phe Lys Phe Gly Asn Ser Ile Val Lys Met Gly Gly Leu Gly Tyr
        275                 280                 285
Lys Trp Ser Glu Ile Ser Tyr Lys Ala Ala Asn Tyr Gln Tyr Asn Tyr
    290                 295                 300
Leu Arg Asp Gly Glu Gln Val Thr Ala His Thr Thr Cys Ser Val Asn
305                 310                 315                 320
Gly Val Asn Asn Phe Ser Tyr Asn Gly Gly Phe Leu Pro Thr Asp Phe
                325                 330                 335
Gly Ile Ser Arg Tyr Glu Val Ile Lys Glu Asn Ser Tyr Val Tyr Val
            340                 345                 350
Asp Tyr Trp Asp Asp Ser Lys Ala Phe Arg Asn Met Val Tyr Val Arg
        355                 360                 365
Ser Leu Ala Ala Asn Leu Asn Ser Val Lys Cys Thr Gly Gly Ser Tyr
    370                 375                 380
Tyr Phe Ser Ile Pro Val Gly Ala Trp Pro Val Met Asn Gly Gly Ala
385                 390                 395                 400
Val Ser Leu His Phe Ala Gly Val Thr Leu Ser Thr Gln Phe Thr Asp
                405                 410                 415
Phe Val Ser Leu Asn Ser Leu Arg Phe Arg Phe Ser Leu Thr Val Asp
            420                 425                 430
Glu Pro Pro Phe Ser Ile Leu Arg Thr Arg Thr Val Asn Leu Tyr Gly
        435                 440                 445
Leu Pro Ala Ala Asn Pro Asn Asn Gly Asn Glu Tyr Tyr Glu Ile Ser
    450                 455                 460
Gly Arg Phe Ser Leu Ile Ser Leu Val Pro Thr Asn Asp Asp Tyr Gln
465                 470                 475                 480
Thr Pro Ile Met Asn Ser Val Thr Val Arg Gln Asp Leu Glu Arg Gln
                485                 490                 495
Leu Thr Asp Leu Arg Glu Glu Phe Asn Ser Leu Ser Gln Glu Ile Ala
            500                 505                 510
Met Ala Gln Leu Ile Asp Leu Ala Leu Leu Pro Leu Asp Met Phe Ser
```

```
            515                 520                 525
Met Phe Ser Gly Ile Lys Ser Thr Ile Asp Leu Thr Lys Ser Met Ala
    530                 535                 540
Thr Ser Val Met Lys Lys Phe Arg Lys Ser Lys Leu Ala Thr Ser Ile
545                 550                 555                 560
Ser Glu Met Thr Asn Ser Leu Ser Asp Ala Ala Ser Ser Ala Ser Arg
                565                 570                 575
Asn Val Ser Ile Arg Ser Asn Leu Ser Ala Ile Ser Asn Trp Thr Asn
            580                 585                 590
Val Ser Asn Asp Val Ser Asn Val Thr Asn Ser Leu Asn Asp Ile Ser
        595                 600                 605
Thr Gln Thr Ser Thr Ile Ser Lys Lys Leu Arg Leu Lys Glu Met Ile
    610                 615                 620
Thr Gln Thr Glu Gly Met Ser Phe Asp Asp Ile Ser Ala Ala Val Leu
625                 630                 635                 640
Lys Thr Lys Ile Asp Met Ser Thr Gln Ile Gly Lys Asn Thr Leu Pro
                645                 650                 655
Asp Ile Val Thr Glu Ala Ser Glu Lys Phe Ile Pro Lys Arg Ser Tyr
            660                 665                 670
Arg Ile Leu Lys Asp Asp Glu Val Met Glu Ile Asn Thr Glu Gly Lys
        675                 680                 685
Phe Phe Ala Tyr Lys Ile Asn Thr Phe Asp Glu Val Pro Phe Asp Val
    690                 695                 700
Asn Lys Phe Ala Glu Leu Val Thr Asp Ser Pro Val Ile Ser Ala Ile
705                 710                 715                 720
Ile Asp Phe Lys Thr Leu Lys Asn Leu Asn Asp Asn Tyr Gly Ile Thr
                725                 730                 735
Arg Thr Glu Ala Leu Asn Leu Ile Lys Ser Asn Pro Asn Met Leu Arg
            740                 745                 750
Asn Phe Ile Asn Gln Asn Asn Pro Ile Ile Arg Asn Arg Ile Glu Gln
        755                 760                 765
Leu Ile Leu Gln Cys Lys Leu
    770                 775
```

<210> 6
<211> 326
<212> PRT
<213> Rotavirus

<400> 6

```
Met Tyr Gly Ile Glu Tyr Thr Thr Ile Leu Ile Phe Leu Ile Ser Ile
1               5                   10                  15
Ile Leu Leu Asn Tyr Ile Leu Lys Ser Val Thr Arg Ile Met Asp Tyr
            20                  25                  30
Ile Ile Tyr Arg Ser Leu Leu Ile Tyr Val Ala Leu Phe Ala Leu Thr
        35                  40                  45
Arg Ala Gln Asn Tyr Gly Leu Asn Leu Pro Ile Thr Gly Ser Met Asp
    50                  55                  60
Thr Val Tyr Ala Asn Ser Thr Gln Glu Gly Ile Phe Leu Thr Ser Thr
65                  70                  75                  80
Leu Cys Leu Tyr Tyr Pro Thr Glu Ala Ser Thr Gln Ile Asn Asp Gly
                85                  90                  95
Glu Trp Lys Asp Ser Leu Ser Gln Met Phe Leu Thr Lys Gly Trp Pro
            100                 105                 110
Thr Gly Ser Val Tyr Phe Lys Glu Tyr Ser Ser Ile Val Asp Phe Ser
```

```
            115                 120                 125
Val Asp Pro Gln Leu Tyr Cys Asp Tyr Asn Leu Val Leu Met Lys Tyr
        130                 135                 140
Asp Gln Asn Leu Glu Leu Asp Met Ser Glu Leu Ala Asp Leu Ile Leu
145                 150                 155                 160
Asn Glu Trp Leu Cys Asn Pro Met Asp Ile Thr Leu Tyr Tyr Tyr Gln
                165                 170                 175
Gln Ser Gly Glu Ser Asn Lys Trp Ile Ser Met Gly Ser Ser Cys Thr
            180                 185                 190
Val Lys Val Cys Pro Leu Asn Thr Gln Met Leu Gly Ile Gly Cys Gln
        195                 200                 205
Thr Thr Asn Val Asp Ser Phe Glu Met Val Ala Glu Asn Glu Lys Leu
    210                 215                 220
Ala Ile Val Asp Val Val Asp Gly Ile Asn His Lys Ile Asn Leu Thr
225                 230                 235                 240
Thr Thr Thr Cys Thr Ile Arg Asn Cys Lys Lys Leu Gly Pro Arg Glu
                245                 250                 255
Asn Val Ala Val Ile Gln Val Gly Gly Ser Asn Val Leu Asp Ile Thr
            260                 265                 270
Ala Asp Pro Thr Thr Asn Pro Gln Thr Glu Arg Met Met Arg Val Asn
        275                 280                 285
Trp Lys Lys Trp Trp Gln Val Phe Tyr Thr Ile Val Asp Tyr Ile Asn
    290                 295                 300
Gln Ile Val Gln Val Met Ser Lys Arg Ser Arg Ser Leu Asn Ser Ala
305                 310                 315                 320
Ala Phe Tyr Tyr Arg Val
                325
```

<210> 7
<211> 175
<212> PRT
<213> Rotavirus

<400> 7

```
Met Asp Lys Leu Ala Asp Leu Asn Tyr Thr Leu Ser Val Ile Thr Leu
 1               5                  10                  15
Met Asn Asp Thr Leu His Ser Ile Ile Gln Asp Pro Gly Met Ala Tyr
            20                  25                  30
Phe Ser Tyr Ile Ala Ser Val Leu Thr Val Leu Phe Ile Leu His Lys

        35                  40                  45
Ala Ser Ile Pro Thr Met Lys Ile Ala Leu Lys Thr Ser Lys Cys Ser
    50                  55                  60
Tyr Lys Val Ile Lys Tyr Cys Ile Val Thr Ile Ile Asn Thr Leu Leu
65                  70                  75                  80
Lys Leu Ala Gly Tyr Lys Glu Gln Val Thr Thr Lys Asp Glu Ile Glu
                85                  90                  95
Gln Gln Met Asp Arg Ile Val Lys Glu Met Arg Arg Gln Leu Asp Met
            100                 105                 110
Ile Asp Lys Leu Thr Thr Arg Glu Ile Glu Gln Val Glu Leu Leu Lys
        115                 120                 125
Arg Ile His Asp Asn Leu Ile Thr Arg Pro Val Asp Val Ile Asp Met
    130                 135                 140
Ser Lys Glu Phe Asn Gln Lys Asn Ile Lys Thr Leu Asp Glu Trp Glu
145                 150                 155                 160
Ser Gly Lys Asn Pro Tyr Glu Pro Ser Glu Val Thr Ala Ser Met


            165                 170                 175
```

<210> 8
<211> 750
<212> DNA
<213> Rotavirus

<400> 8

```
ggcttttaaa agttctgttc cgagagagcg cgtgcggaaa gatggataag cttgccgacc 60
tcaactacac attgagtgta atcactttaa tgaatgacac attgcattct ataattcaag 120
atcctggaat ggcgtatttt tcatatattg catctgttct aacagtttta ttcatattac 180
ataaagcttc aattccaacc atgaaaatag cattgaaaac atcaaaatgt tcatataaag 240
tgattaaata ttgtatagtc acgatcatta atactctttt aaaattggct ggatataaag 300
agcaggttac tacaaaagac gaaattgagc aacagatgga cagaattgtt aaagagatga 360
gacgtcagct ggatatgatt gataaattaa ctactcgtga aattgaacag gttgaattgc 420
ttaaacgtat acatgacaac ctgataacta gaccagttga cgtcatagat atgtcgaagg 480
aattcaatca gaaaaacatc aaaacgctag atgaatggga gagtggaaaa aatccatatg 540
aaccgtcaga agtgactgca tccatgtgag aggttgagtt accgtcgtct gtcttcggaa 600
gcggcggaac tcttcaccgc aagccccatt agacctgatg attgactgag aagccacagt 660
caatcatatc gcgtgtggct cagccttaat cccgtttaac caatccagcg agtgttggac 720
gttaatggaa ggaatggtct tagtgtgacc                                  750
```

<210> 9
<211> 397
<212> PRT
<213> Rotavirus

<400> 9

```
Met Glu Val Leu Tyr Ser Leu Ser Lys Thr Leu Lys Asp Ala Arg Asp
1               5                   10                  15
Lys Ile Val Glu Gly Thr Leu Tyr Ser Asn Val Ser Asp Leu Ile Gln
            20                  25                  30
Gln Phe Asn Gln Met Ile Val Thr Met Asn Gly Asn Asp Phe Gln Thr
        35                  40                  45
Gly Gly Ile Gly Asn Leu Pro Val Arg Asn Trp Thr Phe Asp Phe Gly
    50                  55                  60
Leu Leu Gly Thr Thr Leu Leu Asn Leu Asp Ala Asn Tyr Val Glu Asn
65                  70                  75                  80
Ala Arg Thr Thr Ile Glu Tyr Phe Ile Asp Phe Ile Asp Asn Val Cys
                85                  90                  95
Met Asp Glu Met Ala Arg Glu Ser Gln Arg Asn Gly Val Ser Pro Gln
            100                 105                 110
Ser Glu Ala Leu Arg Lys Leu Ala Gly Ile Lys Phe Lys Arg Ile Asn
        115                 120                 125
Phe Asp Asn Ser Ser Glu Tyr Ile Glu Asn Trp Asn Leu Gln Asn Arg
    130                 135                 140
Arg Gln Arg Thr Gly Phe Val Phe His Lys Pro Asn Ile Phe Pro Tyr
145                 150                 155                 160
Ser Ala Ser Phe Thr Leu Asn Arg Ser Gln Pro Met His Asp Asn Leu
                165                 170                 175
Met Gly Thr Met Trp Leu Asn Ala Gly Ser Glu Ile Gln Val Ala Gly
            180                 185                 190
Phe Asp Tyr Ser Cys Ala Ile Asn Ala Pro Ala Asn Ile Gln Gln Phe
        195                 200                 205
Glu His Ile Val Gln Leu Arg Arg Ala Leu Thr Thr Ala Thr Ile Thr
    210                 215                 220
```

```
Leu Leu Pro Asp Ala Glu Arg Phe Ser Phe Pro Arg Val Ile Asn Ser
225                 230                 235                 240
Ala Asp Gly Ala Thr Thr Trp Phe Phe Asn Pro Val Ile Leu Arg Pro
                245                 250                 255
Asn Asn Val Glu Val Glu Phe Leu Leu Asn Gly Gln Ile Ile Asn Thr
            260                 265                 270
Tyr Gln Ala Arg Phe Gly Thr Ile Ile Ala Arg Asn Phe Asp Thr Ile
        275                 280                 285
Arg Leu Leu Phe Gln Leu Met Arg Pro Pro Asn Met Thr Pro Ala Val
    290                 295                 300
Asn Ala Leu Phe Pro Gln Ala Gln Pro Phe Gln His His Ala Thr Val
305                 310                 315                 320
Gly Leu Thr Leu Arg Ile Glu Ser Ala Val Cys Glu Ser Val Leu Ala
                325                 330                 335
Asp Ala Asn Glu Thr Leu Leu Ala Asn Val Thr Ala Val Arg Gln Glu
            340                 345                 350
Tyr Ala Ile Pro Val Gly Pro Val Phe Pro Pro Gly Met Asn Trp Thr
        355                 360                 365
Glu Leu Ile Thr Asn Tyr Ser Pro Ser Arg Glu Asp Asn Leu Gln Arg
    370                 375                 380
Val Phe Thr Val Ala Ser Ile Arg Ser Met Leu Ile Lys
385                 390                 395
```

<210> 10
<211> 1356
<212> DNA
<213> Rotavirus

<400> 10

```
ggctttaaaa cgaagtcttc gacatggagg ttctgtactc actgtcaaaa actcttaaag 60
atgctaggga caaaattgtt gaaggtacat tatattctaa cgttagcgat cttattcagc 120
aattcaatca aatgatagta actatgaatg gaaatgactt tcagactgga ggaattggta 180
atttacctgt tagaaattgg actttcgatt ttggtctatt aggtacaaca cttttgaact 240
tggatgctaa ttatgttgag aatgcaagaa ctacaattga atatttttatt gactttattg 300
ataatgtatg tatggatgaa atggcaagag aatctcaaag aaatggagta tcgccacaat 360
ctgaagcgtt aagaaagcta gcgggaatta aatttaagag aataaatttc gataattcat 420
cagaatacat agaaaattgg aacttacaaa atagaagaca gcgcaccgga tttgtttttc 480
ataaacctaa catttttcca tactcagctt catttactct aaatagatct caaccaatgc 540
atgataattt aatgggaacc atgtggctta atgctggatc agaaattcaa gtggctggat 600
ttgattactc atgcgccata aatgcaccag cgaacataca gcaatttgag catatcgttc 660
agcttaggcg cgcactgact acagctacta taactttatt acctgatgca gagagattta 720
gttttccaag agtaattaat tcagctgatg gcgcgactac atggttcttt aatccagtta 780
ttctaagacc aaacaatgta gaggtagaat tttattgaa tggacaaatt attaatacat 840
atcaggctag atttggtact atcatcgcaa gaaattttga tacaattcgt ttattatttc 900
agttgatgcg tccacctaat atgacaccag ctgttaatgc actatttcca caagcacaac 960
cttttcagca ccatgcaaca gttggactta cattacgtat tgaatctgcg gtttgtgaat 1020
cagtgcttgc ggacgcgaat gaaactctgt tagcaaatgt gaccgcggtg cgtcaagaat 1080
atgccatacc agttggaccg gtatttccac caggcatgaa ttggactgaa ttgattacta 1140
actattcgcc atctagagaa gataacttgc aacgcgtttt cacggtagct tccattagaa 1200
gcatgttgat taagtgagga ccagactaaa catctggtat ccaatcttag ttagcatgta 1260
gctacatcaa gtcattcaga ctcttcaagt aaggacatga tttcatgttc gctacgtaga 1320
gtaactgtct gaatgatgta gtgagaggat gtgacc                          1356
```

<210> 11
<211> 28
<212> DNA
<213> Artificial Sequence

<220>
<223> Oligonucleotide sharing homoly with rotavirus nucleotide sequences

<400> 11
ggctttaaaa gagagaattt ccgtctgg 28

<210> 12
<211> 25
<212> DNA
<213> Artificial Sequence

<220>
<223> Oligonucleotide sharing homoly with rotavirus nucleotide sequences

<400> 12
ggttagctcc ttttaatgta tggta 25

<210> 13
<211> 27
<212> DNA
<213> Artificial Sequence

<220>
<223> Oligonucleotide sharing homoly with rotavirus nucleotide sequences

<400> 13
ggtcacatcg aacaattcta atctaag 27

<210> 14
<211> 22
<212> DNA
<213> Artificial Sequence

<220>
<223> Oligonucleotide sharing homoly with rotavirus nucleotide sequences

<400> 14
caagtactca aatcaatgat gg 22

<210> 15
<211> 23
<212> DNA
<213> Artificial Sequence

<220>
<223> Oligonucleotide sharing homoly with rotavirus nucleotide sequences

<400> 15
tgttgatttt tctgtcgatc cac 23

<210> 16
<211> 32
<212> DNA
<213> Artificial Sequence

<220>
<223> Oligonucleotide sharing homoly with rotavirus nucleotide sequences

<400> 16
ggttgctgag aatgagaaat tagctatagt gg 32

<210> 17
<211> 32
<212> DNA
<213> Artificial Sequence

<220>
<223> Oligonucleotide sharing homoly with rotavirus nucleotide sequences

<400> 17
ccactatagc taatttctca ttctcagcaa cc 32

<210> 18
<211> 22
<212> DNA
<213> Artificial Sequence

<220>
<223> Oligonucleotide sharing homoly with rotavirus nucleotide sequences

<400> 18
tggcttcgcc attttataga ca 22

<210> 19
<211> 20
<212> DNA
<213> Artificial Sequence

<220>
<223> Oligonucleotide sharing homoly with rotavirus nucleotide sequences

<400> 19
atttcggacc atttataacc 20

<210> 20
<211> 22
<212> DNA
<213> Artificial Sequence

<220>
<223> Oligonucleotide sharing homoly with rotavirus nucleotide sequences

<400> 20
tggcttcact catttataga ca 22

<210> 21
<211> 23
<212> DNA
<213> Artificial Sequence

<220>
<223> Oligonucleotide sharing homoly with rotavirus nucleotide sequences

<400> 21
atttcagacc atttataacc tag 23

<210> 22
<211> 29
<212> DNA

<213> Artificial Sequence

<220>
<223> Oligonucleotide sharing homoly with rotavirus nucleotide sequences

<400> 22
ggagtagtat atgaaagtac aaataatag 29

<210> 23
<211> 29
<212> DNA
<213> Artificial Sequence

<220>
<223> Oligonucleotide sharing homoly with rotavirus nucleotide sequences

<400> 23
ctattatttg tactttcata tactactcc 29

<210> 24
<211> 25
<212> DNA
<213> Artificial Sequence

<220>
<223> Oligonucleotide sharing homoly with rotavirus nucleotide sequences

<400> 24
tcgatacagt ataagagagc acaag 25

<210> 25
<211> 27
<212> DNA
<213> Artificial Sequence

<220>
<223> Oligonucleotide sharing homoly with rotavirus nucleotide sequences

<400> 25
ttcattaact tgtgctctct tatactg 27

<210> 26
<211> 22
<212> DNA
<213> Artificial Sequence

<220>
<223> Oligonucleotide sharing homoly with rotavirus nucleotide sequences

<400> 26
gtatatgtag actattggga tg 22

<210> 27
<211> 22
<212> DNA
<213> Artificial Sequence

<220>

<223> Oligonucleotide sharing homoly with rotavirus nucleotide sequences

<400> 27
catcccaata gtctacatat ac 22

<210> 28
<211> 23
<212> DNA
<213> Artificial Sequence

<220>
<223> Oligonucleotide sharing homoly with rotavirus nucleotide sequences

<400> 28
tgtaactccg gcaaaatgca acg 23

<210> 29
<211> 23
<212> DNA
<213> Artificial Sequence

<220>
<223> Oligonucleotide sharing homoly with rotavirus nucleotide sequences

<400> 29
cgttgcattt tgccggagtt aca 23

<210> 30
<211> 23
<212> DNA
<213> Artificial Sequence

<220>
<223> Oligonucleotide sharing homoly with rotavirus nucleotide sequences

<400> 30
gtaagacaag atttagagcg cca 23

<210> 31
<211> 23
<212> DNA
<213> Artificial Sequence

<220>
<223> Oligonucleotide sharing homoly with rotavirus nucleotide sequences

<400> 31
tggcgctcta aatcttgtct tac 23

<210> 32
<211> 25
<212> DNA
<213> Artificial Sequence

<220>
<223> Oligonucleotide sharing homoly with rotavirus nucleotide sequences

<400> 32

cttgatgctg atgaagcagc atctg 25

<210> 33
<211> 25
<212> DNA
<213> Artificial Sequence

<220>
<223> Oligonucleotide sharing homoly with rotavirus nucleotide sequences

<400> 33
cagatgctgc ttcatcagca tcaag 25

<210> 34
<211> 25
<212> DNA
<213> Artificial Sequence

<220>
<223> Oligonucleotide sharing homoly with rotavirus nucleotide sequences

<400> 34
cgatcatatc gaatattaaa ggatg 25

<210> 35
<211> 25
<212> DNA
<213> Artificial Sequence

<220>
<223> Oligonucleotide sharing homoly with rotavirus nucleotide sequences

<400> 35
catcctttaa tattcgatat gatcg 25

<210> 36
<211> 25
<212> DNA
<213> Artificial Sequence

<220>
<223> Oligonucleotide sharing homoly with rotavirus nucleotide sequences

<400> 36
agcgttcaca caatttacat tgtag 25

<210> 37
<211> 32
<212> DNA
<213> Artificial Sequence

<220>
<223> Oligonucleotide sharing homoly with rotavirus nucleotide sequences

<400> 37
agtattttat actatagtag attatattaa tc 32

<210> 38

<211> 32
<212> DNA
<213> Artificial Sequence

<220>
<223> Oligonucleotide sharing homoly with rotavirus nucleotide sequences

<400> 38
agtattttat actatggtag attatattaa tc 32

<210> 39
<211> 25
<212> DNA
<213> Artificial Sequence

<220>
<223> Oligonucleotide sharing homoly with rotavirus nucleotide sequences

<400> 39
atccccatta tactgcattc ctttc 25

<210> 40
<211> 25
<212> DNA
<213> Artificial Sequence

<220>
<223> Oligonucleotide sharing homoly with rotavirus nucleotide sequences

<400> 40
atccctatta tactgcattt ctttc 25

<210> 41
<211> 25
<212> DNA
<213> Artificial Sequence

<220>
<223> Oligonucleotide sharing homoly with rotavirus nucleotide sequences

<400> 41
atccccatta tactgcattt ctttc 25

<210> 42
<211> 25
<212> DNA
<213> Artificial Sequence

<220>
<223> Oligonucleotide sharing homoly with rotavirus nucleotide sequences

<400> 42
atccctatta tactgcattc ctttc 25